# EUROPEAN PATENT SPECIFICATION

(11) **EP 4 197 528 B1**
(45) Date of publication and mention of the grant of the patent: **03.04.2024**
(21) Application number: 23155432.0
(22) Date of filing: 08.07.2022
(51) Int. Cl.: A61K 9/00, A61K 31/167, A61K 31/40, A61K 31/58, A61P 1/00

(54) **COMPOSITIONS, METHODS AND SYSTEMS FOR AEROSOL DRUG DELIVERY**
ZUSAMMENSETZUNGEN, VERFAHREN UND SYSTEME ZUR AEROSOLWIRKSTOFFFREISETZUNG
COMPOSITIONS, PROCÉDÉS ET SYSTÈMES POUR L'ADMINISTRATION DE MÉDICAMENT EN AÉROSOL

(30) Priority: 09.07.2021 US 202163220362 P; 23.11.2021 US 202163282356 P
(43) Date of publication of application: 21.06.2023
(62) Divisional of application: 22748656.0
(73) Proprietor: AstraZeneca Pharmaceuticals LP, Wilmington, DE 19850 (US)
(72) Inventor: JOSHI, Vidya, South San Francisco, 94080 (US); ARCHBELL, James, South San Franciso, 94080 (US); LACHACZ, Kellisa, South San Francisco, 94080 (US); LAMPA, Charina, South San Francisco, 94080 (US); MELLO, Lauren, Durham, 27703 (US); GUTIERREZ, Gertrude, South San Francisco, 94080 (US); LECHUGA-BALLESTEROS, David, South San Franciso, 94080 (US); TAN, Penny, South San Francisco, 94080 (US); RIEBE, Michael, South San Francisco, 94080 (US)
(74) Representative: Mewburn Ellis LLP

(56) References cited:
- WO-A1-2014/190204
- US-A1- 2012 039 817
- Anonymous: "AstraZeneca progresses Ambition Zero Carbon programme with Honeywell partnership to develop next-generation respiratory inhalers", , 22 February 2022 (2022-02-22), XP055961867, Retrieved from the Internet: URL:https://www.astrazeneca.com/content/as traz/media-centre/press-releases/2022/astr azeneca-progresses-ambition-zero-carbon-pr ogramme-with-honeywell-partnership-to-deve lop-next-generation-respiratory-inhalers.h tml#! [retrieved on 2022-09-16]
- MYRDAL PAUL B. ET AL: "Advances in Metered Dose Inhaler Technology: Formulation Development", AAPS PHARMSCITECH , vol. 15, no. 2 23 January 2014 (2014-01-23), pages 434-455, XP055795960, DOI: 10.1208/s12249-013-0063-x Retrieved from the Internet: URL:http://link.springer.com/article/10.12 08/s12249-013-0063-x/fulltext.html [retrieved on 2022-09-16]

## Description

### BACKGROUND

Methods of targeted drug delivery that deliver an active agent at the site of action are often desirable. For example, targeted delivery of active agents can reduce undesirable side effects, lower dosing requirements, and decrease therapeutic costs. In the context of respiratory delivery, inhalers are well known devices for administering an active agent to a subject's respiratory tract, and several different inhaler systems are currently commercially available. Three common inhaler systems include dry powder inhalers, nebulizers, and metered dose inhalers (MDIs), also known as pressurized metered dose inhalers (pMDIs).

MDIs may be used to deliver medicaments in a solubilized form or as a suspension. Typically, MDIs use a relatively high vapor pressure propellant to expel aerosolized droplets containing an active agent into the respiratory tract when the MDI is activated. Dry powder inhalers generally rely on the patient's inspiratory efforts to introduce a medicament in a dry powder form to the respiratory tract. Nebulizers form a medicament aerosol to be inhaled by imparting energy to a liquid solution or suspension. MDI have provided a reliable, instantly available, and easy to use medical aerosol delivery system for more than sixty years. While dry powder inhalers and nebulizers both have an important role to play in the management of airway and parenchymal disease, there is not yet any all-purpose aerosol generation and delivery system to replace the MDI.

MDIs are active delivery devices that utilize the pressure generated by a propellant. The propellant must be safe for patients' use and be pharmaceutically acceptable. The active agent to be delivered by an MDI is typically provided as a suspension of fine particulates dispersed within a propellant or a combination of two or more propellants (i.e., a propellant "system"). However, fine particles of active agent suspended in a propellant or propellant system tend to aggregate or flocculate rapidly. In turn, aggregation or flocculation of these fine particles may complicate the delivery of the active agent. Another problem associated with such suspension MDI formulations relates to crystal growth of the drug during storage, resulting in a decrease over time of aerosol properties and delivered dose uniformity of such MDIs. Thus, it is critical to properly formulate the active agents with the excipients and propellants to form a stable suspension suitable for MDI. The properties of the propellant play an important role in the performance of a suspension formulation for MDIs. For example, the liquid density, vapor pressure and water solubility of a propellant affect the suspension stability, dose uniformity, aerosol performance and moisture ingress. Other properties of a propellant, such as dipole moment, surface tension, boiling point, liquid viscosity, latent heat, etc., are also factors to be considered when formulating the suspension formulation. Historically, the phase out of chlorofluorocarbon (CFC) propellants, which are ozone-depleting agents, necessitated the reformulation of MDIs with hydrofluoroalkane (HFA) propellants. Although not ozone-depleting, HFA propellants are greenhouse gases having high global warming potential (GWP) and so there remains a need for alternative MDI propellants with reduced environmental impact. However, reformulation of MDI propellants is not a simple task - substantial new technology had to be developed to enable the switch from CFCs to HFAs in MDIs due to considerations of the physicochemical properties of various excipients and how the addition of these excipients may impact overall MDI performance. For example, one of the main challenges was that conventional surfactants used for CFC-based MDIs were not suitable for HFAs.

As there is a desire to develop new environmentally friendly MDIs, there remains a need to research and develop innovative suspension MDI formulations.

US 2012/0039817 A1 describes compositions, methods and systems for pulmonary or nasal delivery of two or more active agents via a metered dose inhaler, wherein at least one of the active agents is selected from long-acting muscarinic antagonist ("LAMA"), long-acting 8, adrenergic agonist (""LABA"), and corticosteroid active agents. WO 2014/190204 A1 describes pharmaceutical compositions, systems and methods suitable for respiratory delivery of a fixed combination of LAMA, LABA, and ICS active agents are described. Myrdal PB, Sheth P, Stein SW. "Advances in metered dose inhaler technology: formulation development" AAPS PharmSciTech. 2014;15(2):434-455 is a review article that presents a survey of challenges associated with formulating MDIs as solution or suspension products with one or more drugs, while considering the physicochemical properties of various excipients and how the addition of these excipients may impact overall product performance of the MDI. Propellants, volatile and nonvolatile cosolvents, surfactants, polymers, suspension stabilizers, and bulking agents are among the variety of excipients discussed in this review article.

### BRIEF SUMMARY

The present invention is defined by the claims. The present disclosure provides compositions, methods, and systems for respiratory delivery of one or more active agents.

In some embodiments, the compositions described herein are formulated for pulmonary delivery of one or more active agents via an MDI. In other embodiments, the compositions described herein may be formulated for nasal delivery via an MDI. Compositions of the invention comprise a propellant of pharmaceutical grade (1*E*)-1,3,3,3-tetrafluoropropene (HFO-1234ze(E)) having a purity of at least 99.90%; (i) a plurality of budesonide particles and a plurality of albuterol particles, (ii) a plurality of glycopyrrolate particles and a plurality of formoterol particles or (iii) a plurality of budesonide particles and a plurality of formoterol particles; and a plurality of phospholipid particles comprising perforated microstructures.

In some embodiments, the compositions described herein further comprise a plurality of a third species of active agent particle, wherein the third species of active agent particle comprises a third active agent. In some embodiments, the compositions described herein further comprise a plurality of a fourth species of active agent particle, wherein the fourth species of active agent particle comprises a fourth active agent. In further embodiments, the third active agent is selected from a long-acting muscarinic antagonist (LAMA), a long-acting β2-agonist (LABA), a short-acting beta-agonist (SABA), an inhaled corticosteroid (ICS), and a non-corticosteroid anti-inflammatory agent. In yet further embodiments, the fourth active agent is selected from a long-acting muscarinic antagonist (LAMA), a long-acting β2-agonist (LABA), a short-acting beta-agonist (SABA), an inhaled corticosteroid (ICS), and a non-corticosteroid anti-inflammatory agent.

The methods described herein include methods of treating a pulmonary disease or disorder in a patient by actuating a metered dose inhaler containing a composition as described herein.

Also described herein are systems for pulmonary delivery of one or more active agents. In some embodiments, such systems include an MDI comprising a canister with an outlet valve including an actuator (e.g., a depressible valve stem) for dispensing a metered amount of a composition according to the claims. In some embodiments, the outlet valve is at least partially composed of bromobutyl material. For example, an internal neck gasket of the outlet valve may comprise or consist of bromobutyl material. In addition, one or more internal seat gaskets of the outlet valve may comprise or consist of bromobutyl material.

The invention provides a pharmaceutical composition deliverable from a metered dose inhaler, the pharmaceutical composition comprising: a propellant of pharmaceutical grade (1*E*)-1,3,3,3-Tetrafluoro-1-propene (HFO-1234ze(E)) having a purity of at least 99.90%; (i) a plurality of budesonide particles and a plurality of albuterol particles, (ii) a plurality of glycopyrrolate particles and a plurality of formoterol particles or (iii) a plurality of budesonide particles and a plurality of formoterol particles;and a plurality of phospholipid particles comprising perforated microstructures.

In one embodiment, the pharmaceutical composition comprises a plurality of a first species of active agent particle; wherein the active agent is budesonide; or a pharmaceutically acceptable salt or solvate thereof; and a plurality of a second species of active agent particle; wherein the active agent is formoterol; or a pharmaceutically acceptable salt or solvate thereof.

In one embodiment of the pharmaceutical composition, the ICS is budesonide or a pharmaceutically acceptable salt or solvate thereof; and the LABA is formoterol or a pharmaceutically acceptable salt or solvate thereof.

In one embodiment of the pharmaceutical composition, the formoterol is present at a concentration in the range of 0.04 mg/mL to 2.25 mg/mL.

In one embodiment of the pharmaceutical composition, the formoterol is present at a concentration in the range of 0.01 mg/mL to 1 mg/mL.

In one embodiment of the pharmaceutical composition, the budesonide is present at a concentration in the range of 0.1 mg/mL to 20 mg/mL.

In one embodiment of the pharmaceutical composition, the perforated microstructures comprise 1,2-Distearoyl-sn-glycero-3-phosphocholine (DSPC). In another embodiment, the perforated microstructures further comprise calcium chloride.

In one embodiment of the pharmaceutical composition, the phospholipid particles are present at a concentration in the range of 0.1 mg/mL to 10 mg/mL.

In one embodiment, the present pharmaceutical composition comprises: a propellant of pharmaceutical grade HFO-1234ze(E) having a purity of at least 99.90%; a plurality of budesonide particles; a plurality of albuterol particles; and a plurality of phospholipid particles comprising perforated microstructures.

In one embodiment, the pharmaceutical composition comprises: a propellant of pharmaceutical grade HFO-1234ze(E) having a purity of at least about 99.90%; a plurality of budesonide particles; a plurality of formoterol fumarate particles; and a plurality of phospholipid particles comprising perforated microstructures.

In one embodiment of the pharmaceutical composition, the albuterol particles are in the propellant at a concentration sufficient to provide a delivered dose of albuterol per actuation of the metered dose inhaler selected from between 5 µg and 50 µg per actuation, between 2 µg and 25 µg per actuation, and between 6 µg and 15 µg per actuation.

In one embodiment of the pharmaceutical composition, the albuterol particles comprise micronized and crystalline albuterol sulfate.

In one embodiment of the pharmaceutical composition, the formoterol particles are included in the composition at a concentration sufficient to provide a delivered dose of formoterol selected from between 1 µg and 30 µg, between 0.5 µg and 10 µg, between 2 µg and 5 µg, between 3 µg and 10 µg, between 5 µg and 10 µg, and between 3 µg and 30 µg per actuation of the metered dose inhaler.

In one embodiment of the pharmaceutical composition, the formoterol particles comprise micronized and crystalline formoterol fumarate.

In one embodiment of the pharmaceutical composition, the budesonide particles are included in the composition at a concentration sufficient to provide a delivered dose of budesonide selected from between 50 µg and 400 µg, between 20 µg and 600 µg, between 30 µg and 100 µg, between 50 µg and 200 µg, and between 150 µg and 350 µg per actuation of the metered dose inhaler.

In one embodiment of the pharmaceutical composition, the budesonide particles comprise micronized budesonide.

In one embodiment of the pharmaceutical composition, the phospholipid particles are included in the composition at a concentration sufficient to provide a delivered dose of the phospholipid particles selected from between 50 µg and 400 µg.

The pharmaceutical composition may exhibit Cmax, AUCinf or AUClast of any one or more of the active agents, which is about 80% to about 125% of Cmax, AUCinf or AUClast of the one or more of the active agents of a reference pharmaceutical composition which comprises a propellant of pharmaceutical grade HFA-134a.

The invention provides a metered dose inhaler comprising a canister with an outlet valve including an actuator for dispensing a metered amount of a pharmaceutical composition according to the claims, wherein the canister contains the pharmaceutical composition.

In one embodiment of the metered dose inhaler, the outlet valve comprises a neck gasket and at least one seat gasket; and the neck gasket or the at least one seat gasket is composed of bromobutyl material.

In one embodiment, the metered dose inhaler exhibits less than 10%, 9%, 8%, 7%, 6%, or 5% reduced shot weight per actuation throughout emptying of the canister.

In one embodiment, the metered dose inhaler exhibits less than 1.0%, 0.5%, 0.4%, 0.3%, 0.2%, or 0.1% weight loss at 25°C/60% RH per year.

In one embodiment, the metered dose inhaler exhibits a delivered dose uniformity (DDU) for the pharmaceutical formulation selected from a DDU of ± 20%, or better, a DDU of ± 15%, or better, and a DDU of ± 10%, or better, throughout emptying of the canister.

Disclosed herein is a method of treating a pulmonary disease or disorder in a patient, comprising administering a pharmaceutical composition according to any one of the aforementioned embodiments to the patient by actuating a metered dose inhaler according to any one of the aforementioned embodiments; wherein the metered dose inhaler contains the pharmaceutical composition.

In one example of the method, the pulmonary disease or disorder is asthma or COPD.

Disclosed herein is a pharmaceutical composition according to any one of the aforementioned embodiments for use in the manufacture of a medicament for the treatment of a pulmonary disease or disorder.

The invention provides a pharmaceutical composition according to any one of claims 1 to 11 for use in the treatment of a pulmonary disease or disorder.

### BRIEF DESCRIPTION OF THE SEVERAL VIEWS OF THE DRAWINGS

**FIG. 1** is an isometric view of an aerosol delivery unit in the form of an MDI, according to one example embodiment.
**FIG. 2** is an exploded isometric view of the aerosol delivery unit of **FIG. 1****.**
**FIG. 3A** is a side view of the aerosol delivery unit of **FIG. 1** with a portion thereof illustrated in cross-section, showing the unit in a standby or storage configuration in which the discharge passageway is exposed to a desiccant material.
**FIG. 3B** is a side view of the aerosol delivery unit of **FIG. 1** with a portion thereof illustrated in cross-section, showing the unit in a discharge configuration in which the discharge passageway is temporarily isolated from the desiccant material as aerosolized matter is discharged from the canister into an inhalation passageway for delivery to a user.
**FIG. 4** is a perspective view of an outlet valve of a canister suitable for use in connection with the aerosol delivery unit of **FIGs 1** through **3B****.**
**FIG. 5** is a CT scan of a discharge passageway of an MDI according to certain aspects of the present disclosure, showing a discharge orifice of the MDI substantially free of deposited or accumulated matter despite repeated use of the MDI to dispense formulations described herein.
**FIG. 6** is a chart illustrating formulation weight loss over time for a variety of MDI canisters including an outlet valve with internal gaskets of different materials, when filled with a formulation comprising an HFO propellant.
**FIG. 7** shows individual deposition distributions for active agent particles dispensed from an MDI containing a triple co-suspension of glycopyrrolate, budesonide, and formoterol active agent particles suspended in HFO-1234ze(E) propellant with phospholipid suspending particles.
**FIG. 8** shows the deposition distribution of formoterol active agent particles dispensed from an MDI containing a triple co-suspension of glycopyrrolate, budesonide, and formoterol active agent particles suspended in HFO-1234ze(E) propellant with phospholipid suspending particles, at several different relative humidity levels.
**FIG. 9** shows the deposition distribution of budesonide active agent particles dispensed from an MDI containing a triple co-suspension of glycopyrrolate, budesonide, and formoterol active agent particles suspended in HFO-1234ze(E) propellant with phospholipid suspending particles, at several different relative humidity levels.
**FIG. 10A** shows the fine particle fraction (FPF) present in the delivered dose upon actuation of an MDI containing budesonide, formoterol, or glycopyrrolate active agent particles and phospholipid particles, as measured following storage of the MDI under at 25°C and 60% relative humidity for the indicated periods of time.
**FIG. 10B** shows the fine particle fraction (FPF) present in the delivered dose upon actuation of an MDI containing budesonide, formoterol, or glycopyrrolate active agent particles and phospholipid particles, as measured following storage of the MDI under at 40°C and 75% relative humidity for the indicated periods of time.
**FIG. 10C** shows the fine particle fraction (FPF) present in the delivered dose upon actuation of an MDI containing budesonide, formoterol, or glycopyrrolate active agent particles and phospholipid particles, as measured following storage of the MDI under at 30°C and 65% relative humidity for the indicated periods of time.
**FIG. 11A** shows the fine particle mass (FPM) present in the delivered dose upon actuation of an MDI containing budesonide and phospholipid particles, as measured following storage of the MDI at 25°C and 60% relative humidity for the indicated periods of time.
**FIG. 11B** shows the fine particle mass (FPM) present in the delivered dose upon actuation of an MDI containing budesonide and phospholipid particles, as measured following storage of the MDI at 40°C and 75% relative humidity for the indicated periods of time.
**FIG. 11C** shows the fine particle mass (FPM) present in the delivered dose upon actuation of an MDI containing budesonide and phospholipid particles, as measured following storage of the MDI at 30°C and 65% relative humidity for the indicated periods of time.
**FIG. 12A** shows measurements of degradation of budesonide active agent particles in an MDI canister containing active agent particles and phospholipid particles following storage of the MDI at 25°C and 60% relative humidity for the indicated periods of time.
**FIG. 12B** shows measurements of degradation of budesonide active agent particles in an MDI canister containing active agent particles and phospholipid particles following storage of the MDI at 40°C and 75% relative humidity for the indicated periods of time.
**FIG. 12C** shows measurements of degradation of budesonide active agent particles in an MDI canister containing active agent particles and phospholipid particles following storage of the MDI at 30°C and 65% relative humidity for the indicated periods of time.
**FIG. 13A** shows measurements of degradation of glycopyrrolate active agent particles in an MDI canister containing active agent particles and phospholipid particles following storage of the MDI at 25°C and 60% relative humidity for the indicated periods of time.
**FIG. 13B** shows measurements of degradation of glycopyrrolate active agent particles in an MDI canister containing active agent particles and phospholipid particles following storage of the MDI at 40°C and 75% relative humidity for the indicated periods of time.
**FIG. 13C** shows measurements of degradation of glycopyrrolate active agent particles in an MDI canister containing active agent particles and phospholipid particles following storage of the MDI at 30°C and 65% relative humidity for the indicated periods of time.
**FIG. 14A** shows the delivered dose uniformity (DDU) upon actuation of an MDI containing budesonide active agent particles and phospholipid particles following storage of the MDI at 25°C and 60% relative humidity for the indicated periods of time.
**FIG. 14B** shows the delivered dose uniformity (DDU) upon actuation of an MDI containing budesonide active agent particles and phospholipid particles following storage of the MDI at 40°C and 75% relative humidity for the indicated periods of time.
**FIG. 14C** shows the delivered dose uniformity (DDU) upon actuation of an MDI containing budesonide active agent particles and phospholipid particles following storage of the MDI at 30°C and 65% relative humidity for the indicated periods of time.
**FIG. 15** shows BD, FF, and DSPC Aerodynamic Particle Size Distribution by NGI of BFF-1234ze.
**FIG. 16** shows aerodynamic particle size distribution by NGI of BD comparing BFF-1234ze and BFF-134a formulations.
**FIG. 17** shows aerodynamic particle size distribution by NGI ofFF comparing BFF-1234ze and BFF-134a formulations.
**FIG. 18** shows BD aerodynamic Particle Size Distribution by NGI Stability Data for BFF-1234ze, 25°C/60% RH - Valve Down, Protected at initial, 6 months, and 12 months.
**FIG. 19** shows FF Aerodynamic Particle Size Distribution by NGI Stability Data for BFF-1234ze, 25°C/60% RH - Valve Down, Protected at initial, 6 months, and 12 months.
**FIG. 20** shows DSPC Aerodynamic Particle Size Distribution by NGI Stability Data for BFF-1234ze, 25°C/60% RH - Valve Down, Protected at initial, 6 months, and 12 months.
**FIG. 21** shows BD and FF Delivered Dose Uniformity Stability Data for BFF-1234ze, 25°C/60% RH - Valve Down, Protected.
**FIG. 22** shows BD, AB, and DSPC Aerodynamic Particle Size Distribution by NGI of BDA-1234ze.
**FIG. 23** shows Aerodynamic particle size distribution by NGI of BD comparing BDA-1234ze and BDA-134a formulations.
**FIG. 24** shows Aerodynamic particle size distribution by NGI of AB comparing BDA-1234ze and BDA-134a formulations.
**FIG. 25** shows BD aerodynamic Particle Size Distribution by NGI Stability Data for BDA-1234ze, 25°C/60% RH - Valve Down, Protected at initial, 6 months, and 12 months.
**FIG. 26** shows AB Aerodynamic Particle Size Distribution by NGI Stability Data for BDA-1234ze, 25°C/60% RH - Valve Down, Protected at initial, 6 months, and 12 months.
**FIG. 27** shows BD and AB Delivered Dose Uniformity Stability Data for BDA-1234ze, 25°C/60% RH - Valve Down, Protected.
**FIG. 28** shows GP and FF Aerodynamic Particle Size Distribution by NGI of GFF-1234ze.

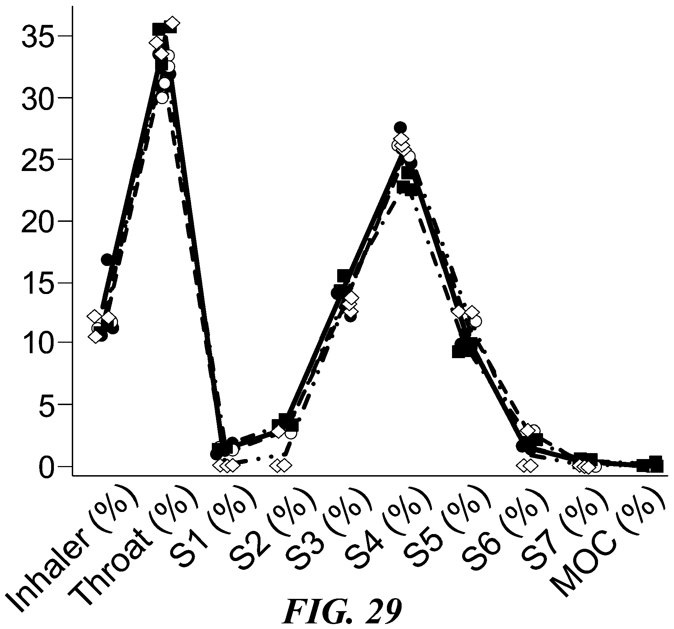

**FIG. 29** shows BD, GP, FF, and RF Aerodynamic Particle Size Distribution by NGI of BGFR-1234ze.

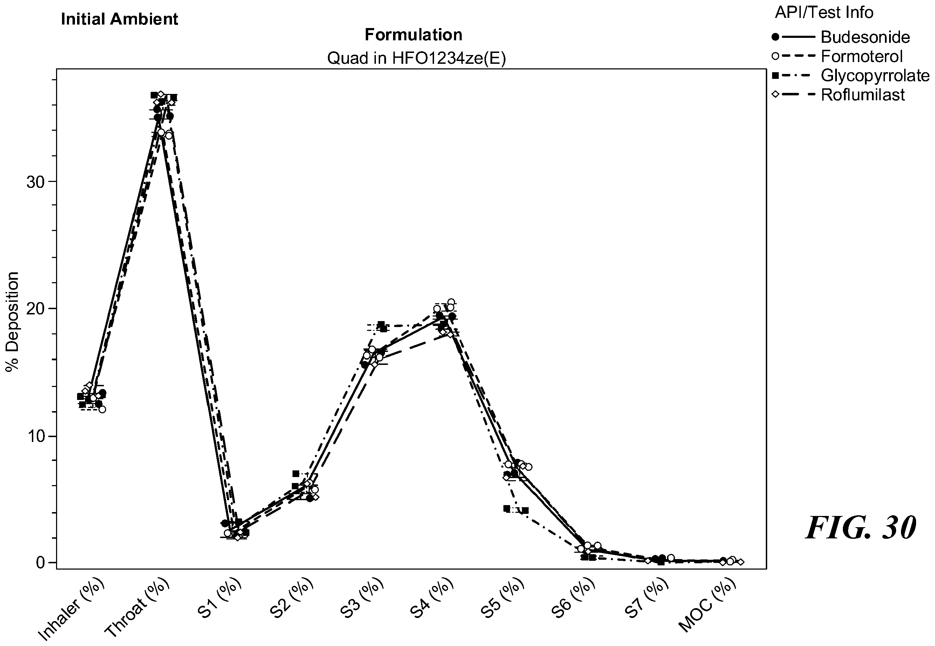

**FIG. 30** shows the deposition distribution of budesonide, glycopyrrolate, formoterol, and roflumilast active agent particles dispensed from an MDI containing a quadruple co-suspension of glycopyrrolate, budesonide, formoterol and roflumilast active agent particles suspended in HFO-1234ze(E) propellant with phospholipid suspending particles

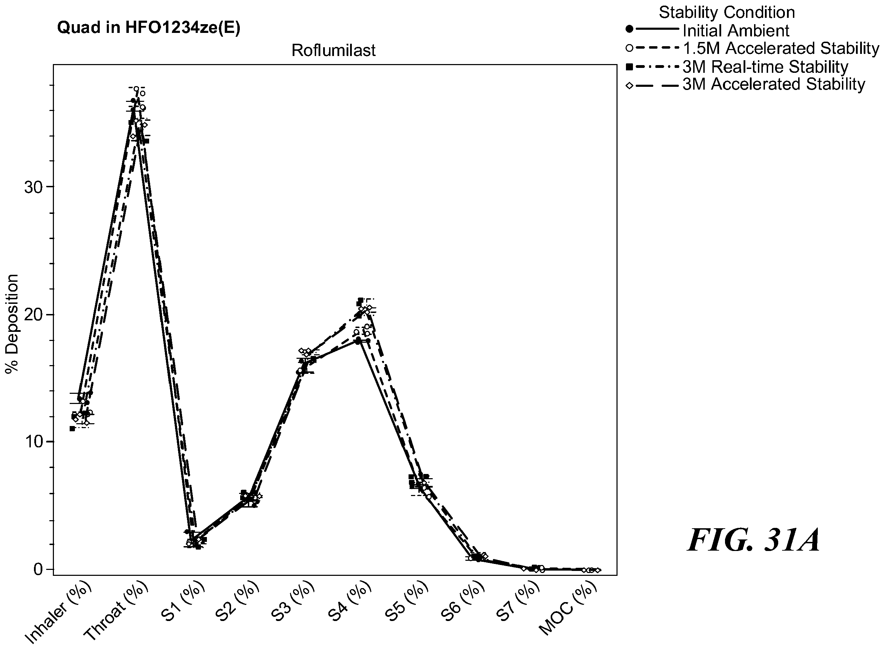

**FIG. 31A** shows the deposition distribution of roflumilast active agent particles dispensed from an MDI containing a quadruple co-suspension of glycopyrrolate, budesonide, and formoterol and roflumilast active agent particles suspended in HFO-1234ze(E) propellant with phospholipid suspending particles upon actuation after 3 months in stability storage conditions representative of accelerated stability (40°C/75% RH -Valve Down, Protected) and 3 months in stability storage conditions representative of real-time stability (25°C/60% RH -Valve Down, Protected.)

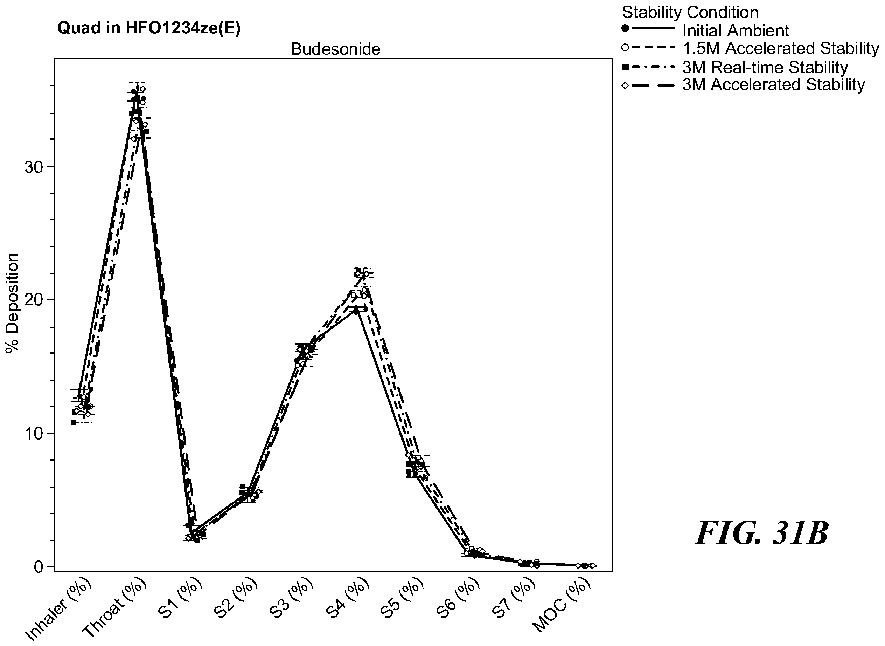

**FIG. 31B** shows the deposition distribution of budesonide active agent particles dispensed from an MDI containing a quadruple co-suspension of glycopyrrolate, budesonide, and formoterol and roflumilast active agent particles suspended in HFO-1234ze(E) propellant with phospholipid suspending particles upon actuation after 3 months in stability storage conditions representative of accelerated stability (40°C/75% RH -Valve Down, Protected) and 3 months in stability storage conditions representative of real-time stability (25°C/60% RH -Valve Down, Protected.)

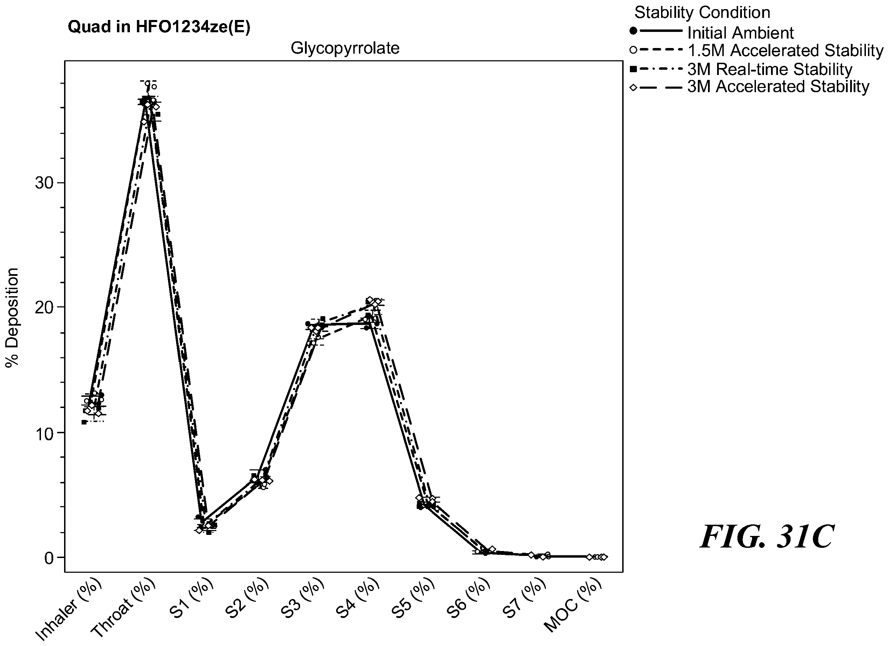

**FIG. 31C** shows the deposition distribution of glycopyrrolate active agent particles dispensed from an MDI containing a quadruple co-suspension of glycopyrrolate, budesonide, and formoterol and roflumilast active agent particles suspended in HFO-1234ze(E) propellant with phospholipid suspending particles upon actuation after 3 months in stability storage conditions representative of accelerated stability (40°C/75% RH -Valve Down, Protected) and 3 months in stability storage conditions representative of real-time stability (25°C/60% RH -Valve Down, Protected.)

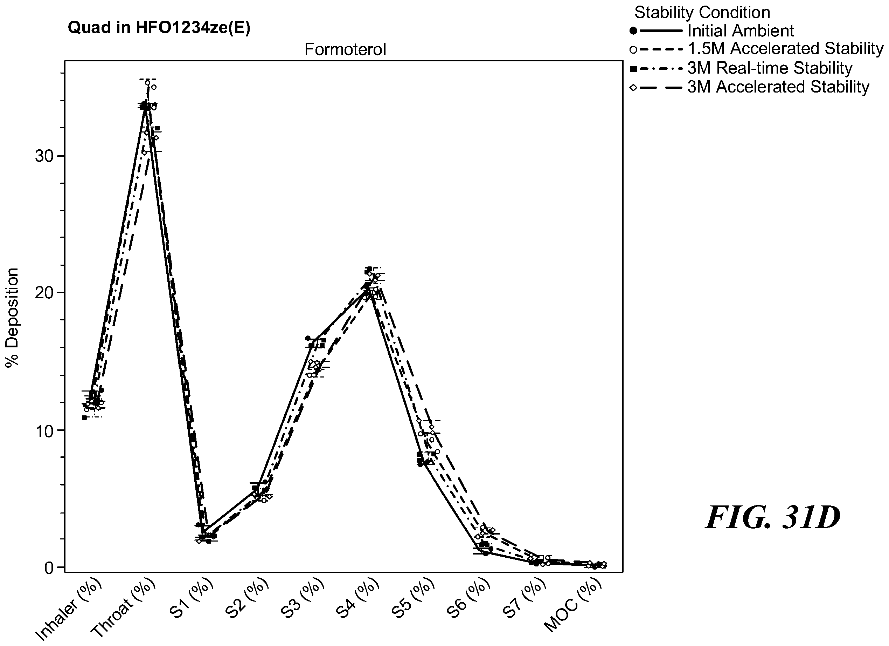

**FIG. 31D** shows the deposition distribution of formoterol active agent particles dispensed from an MDI containing a quadruple co-suspension of glycopyrrolate, budesonide, and formoterol and roflumilast active agent particles suspended in HFO-1234ze(E) propellant with phospholipid suspending particles upon actuation after 3 months in stability storage conditions representative of accelerated stability (40°C/75% RH -Valve Down, Protected) and 3 months in stability storage conditions representative of real-time stability (25°C/60% RH -Valve Down, Protected.)

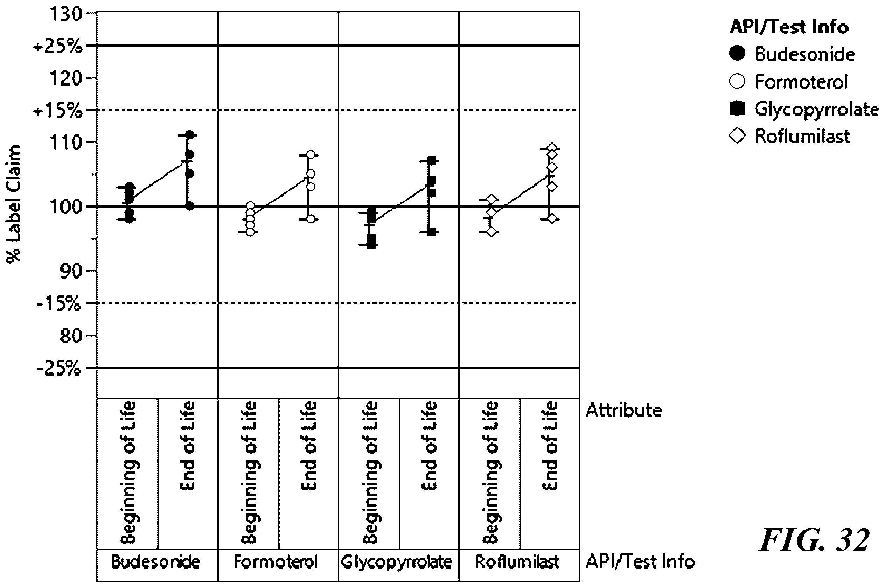

**FIG. 32** shows the delivered dose uniformity (DDU) of roflumilast, formoterol, budesonide and glycopyrrolate active agent particles dispensed at the beginning and life and at the end of life from an MDI containing a quadruple co-suspension of glycopyrrolate, budesonide, and formoterol and roflumilast active agent particles suspended in HFO-1234ze(E) propellant with phospholipid suspending particles.

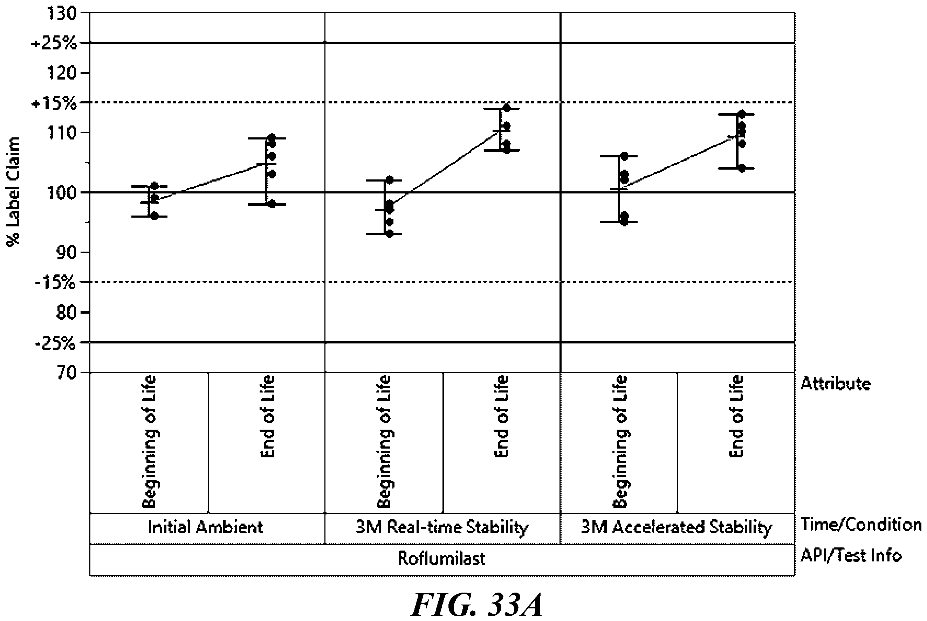

**FIG. 33A** shows the delivered dose uniformity (DDU) of roflumilast active agent particles dispensed at the beginning and life and at the end of life from an MDI containing a quadruple co-suspension of glycopyrrolate, budesonide, and formoterol and roflumilast active agent particles suspended in HFO-1234ze(E) propellant with phospholipid suspending particles after 3 months in stability storage conditions representative of accelerated stability (40°C/75% RH -Valve Down, Protected) and 3 months in stability storage conditions representative of real-time stability (25°C/60% RH -Valve Down, Protected.)

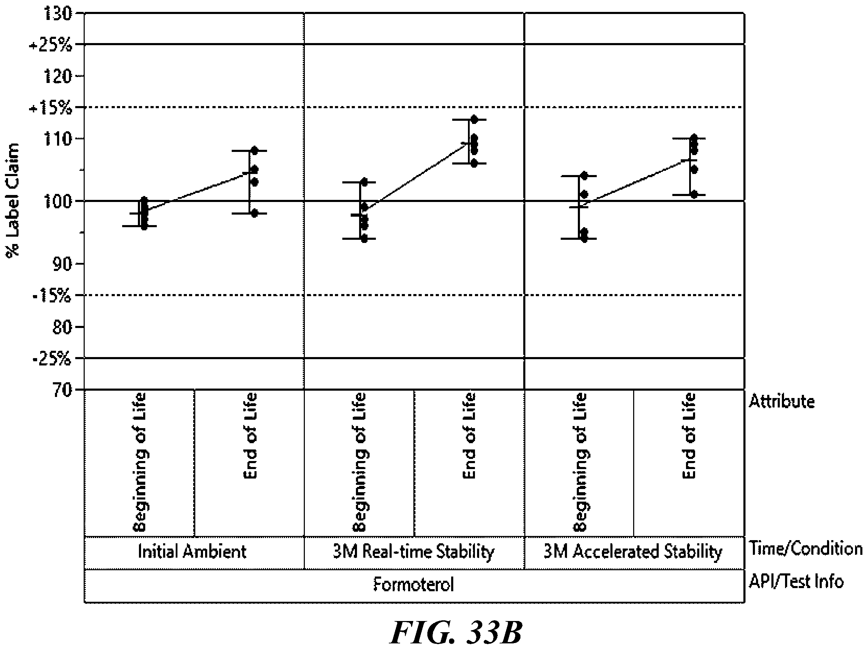

**FIG. 33B** shows the delivered dose uniformity (DDU) of formoterol active agent particles dispensed at the beginning and life and at the end of life from an MDI containing a quadruple co-suspension of glycopyrrolate, budesonide, and formoterol and roflumilast active agent particles suspended in HFO-1234ze(E) propellant with phospholipid suspending particles after 3 months in stability storage conditions representative of accelerated stability (40°C/75% RH -Valve Down, Protected) and 3 months in stability storage conditions representative of real-time stability (25°C/60% RH -Valve Down, Protected.)

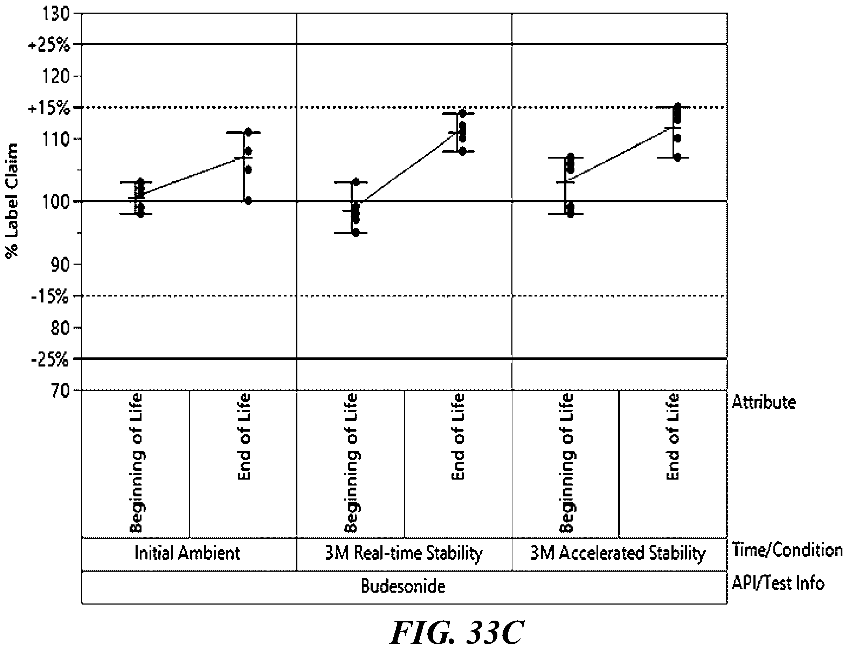

**FIG. 33C** shows the delivered dose uniformity (DDU) of budesonide active agent particles dispensed at the beginning and life and at the end of life from an MDI containing a quadruple co-suspension of glycopyrrolate, budesonide, and formoterol and roflumilast active agent particles suspended in HFO-1234ze(E) propellant with phospholipid suspending particles after 3 months in stability storage conditions representative of accelerated stability (40°C/75% RH -Valve Down, Protected) and 3 months in stability storage conditions representative of real-time stability (25°C/60% RH -Valve Down, Protected.)

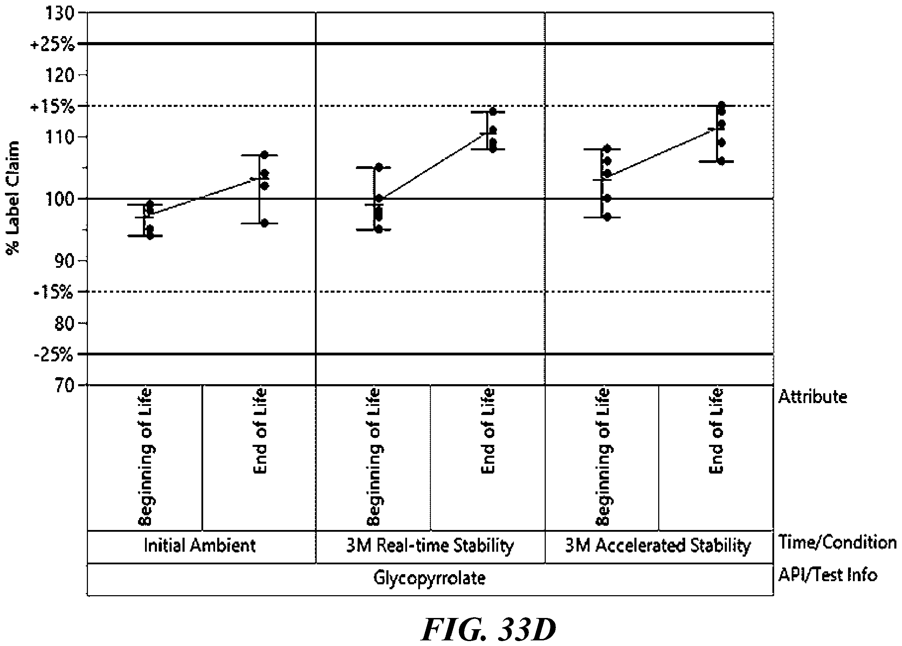

**FIG. 33D** shows the delivered dose uniformity (DDU) of glycopyrrolate active agent particles dispensed at the beginning and life and at the end of life from an MDI containing a quadruple co-suspension of glycopyrrolate, budesonide, and formoterol and roflumilast active agent particles suspended in HFO-1234ze(E) propellant with phospholipid suspending particles after 3 months in stability storage conditions representative of accelerated stability (40°C/75% RH -Valve Down, Protected) and 3 months in stability storage conditions representative of real-time stability (25°C/60% RH -Valve Down, Protected.)
**FIG. 34** shows budesonide and formoterol fumarate aerodynamic particle size distribution by Next Generation Impactor (NGI) of HFA-134a (BFF-134a) and HFO-1234ze (BFF-1234ze).
**FIG. 35** shows budesonide and formoterol fumarate aerodynamic particle size distribution by Next Generation Impactor (NGI) of HFA-134a (BFF crystal-134a) and HFO-1234ze (BFF crystal-1234ze).
**FIG. 36** shows budesonide, glycopyrronium, and formoterol fumarate aerodynamic particle size distribution by Next Generation Impactor (NGI) of HFA-134a (BGF-134a) and HFO-1234ze (BGF-1234ze).
**FIG. 37** shows budesonide, glycopyrronium, and formoterol fumarate aerodynamic particle size distribution by Next Generation Impactor (NGI) of HFA-134a (BGF crystal-134a) and HFO-1234ze (BGF-1234ze).

### DETAILED DESCRIPTION

### Definitions

Unless specifically defined otherwise, the technical terms, as used herein, have their normal meaning as understood in the art. The following terms are specifically defined for the sake of clarity.

The term "active agent" is used herein to include any agent, drug, compound, composition, or other substance that may be used on, or administered to, a human or animal for any purpose, including therapeutic, pharmaceutical, pharmacological, diagnostic, cosmetic, and prophylactic agents and immunomodulators. Active agent may be used interchangeably with the terms drug, pharmaceutical, medicament, drug substance, or therapeutic. As used herein, active agent may also encompass natural or homeopathic products that are not generally considered therapeutic.

The terms "associate," "associate with," or "association" refer to an interaction or relationship between a chemical entity, composition, or structure in a condition of proximity of a surface, such as the surface of another chemical entity, composition, or structure. Association includes, for example, adsorption, adhesion, covalent bonding, hydrogen boding, ionic bonding and electrostatic attraction, Lifshitzvan der Waals interactions, and polar interactions. The terms "adhere" or "adhesion" refer to a form of association, and are used a generic terms for all forces tending to cause a particle or mass to be attracted to a surface. Adhere also refers to bringing and keeping particles in contact with each other, such that there is substantially no visible separation between particles due to their different buoyancies in a propellant under normal conditions. In one embodiment, a particle that attaches to or binds to a surface is encompassed by the term adhere. Normal conditions may include storage at room temperature or under an accelerative force due to gravity. As described herein, active particles may associate with suspending particles to form a co-suspension, where there is substantially no visible separation between the suspending particles and the active agent particles or flocculates thereof due to differences in buoyance within a propellant.

"Suspending particles" refer to a material or combination of materials that is acceptable for respiratory delivery and acts as a vehicle for active agent particles. Suspending particles interact with the active agent particle to facilitate repeatable dosing, delivery, or transport of active agent to the target site of delivery, i.e., the respiratory tract. The suspending particles described herein are dispersed within a suspension medium including a propellant or propellant system, and can be configured according to any shape, size, or surface characteristic suited to achieving a desired suspension stability or active agent delivery performance. Exemplary suspending particles include particles that exhibit a particle size that facilitates respiratory delivery of active agent and have physical configurations suited to formulation and delivery of the stabilized suspensions as described herein.

The term "co-suspension" refers to a suspension of two or more types of particles having different compositions within a suspension medium, wherein one type of particle associates at least partially with one or more of the other particle types. The association leads to an observable change in one or more characteristics of at least one of the individual particle types suspended in the suspension medium. Characteristics modified by the association may include, for example, one or more of the rate of aggregation or flocculation, the rate and nature of separation, i.e., sedimentation or creaming, density of a cream or sediment layer, adhesion to container walls, adhesion to valve components, and the rate and level of dispersion upon agitation. The term co-suspension includes partial co-suspensions, where a majority of the at least two particle types associate with each other, however, some separation (i.e., less than a majority) of the at least two particle types may be observed.

The term "metered dose" or "actuated dose" refers to the amount of active agent contained in the volume of formulation that exits the canister upon actuation of an MDI. The term "delivered dose" refers to the amount of active agent contained in the volume of formulation that exits the actuator nozzle and is available to be drawn into a patient's lungs. In some embodiments, the delivered dose is about 85% to about 95% of the metered dose.

In the context of a composition containing or providing respirable aggregates, particles, drops, etc., such as compositions described herein, the term "fine particle dose" or "FPD" refers to the dose, either in total mass or fraction of the nominal dose or metered dose, that is within a respirable range. The dose that is within the respirable range in measured *in vitro* to be the sum of the dose delivered at stages 3 through Micro Orifice Collector in a Next Generation Impactor operated at a flow rate of 30 1/min.

In the context of a composition containing or providing respirable aggregates, particles, drops, etc., such as compositions described herein, the term "fine particle fraction" or "FPF" refers to the proportion of the delivered material relative to the delivered dose (i.e., the amount that exits the actuator of a delivery device, such as an MDI) that is within a respirable range. The amount of delivered material within the respirable range is measured *in vitro* as the sum of the material delivered at stages 3 through Micro Orifice Collector in a Next Generation Impactor operated at a flow rate of 30 1/min.

As used herein, the term "inhibit" refers to a measurable lessening of the tendency of a phenomenon, symptom, or condition to occur or the degree to which that phenomenon, symptom, or condition occurs. The term "inhibit", or any form thereof, is used in its broadest sense and includes minimize, prevent, reduce, repress, suppress, curb, constrain, restrict, slow progress of, and the like.

"Mass median aerodynamic diameter" or "MMAD" as used herein refers to the aerodynamic diameter of an aerosol below which 50% of the mass of the aerosol consists of particles with an aerodynamic diameter smaller than the MMAD, with the MMAD being calculated according to monograph 601 of the United States Pharmacopeia ("USP).

When referred to herein, the term "optical diameter" indicates the size of a particle as measured by the Fraunhofer diffraction mode using a laser diffraction particle size analyzer equipped with a dry powder dispenser (e.g., Sympatec GmbH, Clasthal-Zellerfeld, Germany).

The term "solution mediated transformation" refers to the phenomenon in which a more soluble form of a solid material (i.e., particles with small radius of curvature (a driving force for Ostwald ripening), or amorphous material) dissolves and recrystallizes into the more stable crystal form that can coexist in equilibrium with its saturated propellant solution.

A "patient" refers to an animal in which the one or more active agents as described herein will have a therapeutic effect. In some embodiments, the patient is a human being.

"Perforated microstructures" refers to suspending particles that include a structural matrix that exhibits, defines, or comprises voids, pores, defects, hollows, spaces, interstitial spaces, apertures, perforations, or holes that allow the surrounding suspension medium to permeate, fill, or pervade the microstructure, such as those materials and preparations described in U.S. Patent No. 6, 309,623 to Weers, et al., and in U.S. Patent No. 8,815,258, U.S. Patent No. 9,463,161, and U.S. Patent Application Publication 2011/0135737. The primary form of the perforated microstructure is, generally, not essential, and any overall configuration that provides the desired formulation characteristics is contemplated herein. Accordingly, in some embodiments, the perforated microstructures may comprise approximately spherical shapes, such as hollow, porous, spray-dried microspheres. However, collapsed, corrugated, deformed, or fractured particulates of any primary form or aspect ratio may also be compatible.

As is true of the suspending particles described herein, perforated microstructures may be formed of any biocompatible material that does not substantially degrade or dissolve in the selected suspension medium. While a wide variety of materials may be used to form the particles, in some embodiments, the structural matrix is associated with, or includes, a surfactant such as a phospholipid or fluorinated surfactant.

The term "suspension medium" as used herein refers to a substance providing a continuous phase within which active agent particles and suspending particles can be dispersed to provide a co-suspension formulation. The suspension medium used in formulations described herein includes propellant. As used herein, the term "propellant" refers to one or more pharmacologically inert substances which exert a sufficiently high vapor pressure at normal room temperature to propel a medicament from the canister of an MDI to a patient on actuation of the MDI's metering valve. Therefore, the term "propellant" refers to both a single propellant and to a combination of two or more different propellants forming a "propellant system."

The term "respirable" generally refers to particles, aggregates, drops, etc. sized such that they can be inhaled and reach the airways of the lung.

When used to refer to compositions described herein, the terms "physical stability" and "physically stable" refer to a composition that is resistant to one or more of aggregation, flocculation, and particle size changes due to solution mediated transformations and is capable of substantially maintaining the MMAD of suspending particles and the fine particle dose. In some embodiments, physical stability may be evaluated through subjecting compositions to accelerated degradation conditions, such as by temperature cycling.

When referring to active agents, the term "potent" indicates active agents that are therapeutically effective at or below doses ranging from about 0.01 mg/kg to about 1 mg/kg. Typical doses of potent active agents generally range from about 100 µg to about 100 mg.

When referring to active agents, the term "highly potent" indicates active agents that are therapeutically effective at or below doses of about 10 µg/kg. Typical doses of highly potent active agents generally range up to about 100 µg.

The terms "suspension stability" and "stable suspension" refer to suspension formulations capable of maintaining the properties of a co-suspension of active agent particles and suspending particles over a period of time. In some embodiments, suspension stability may be measured through delivered dose uniformity achieved by compositions described herein.

The term "substantially insoluble" means that a composition is either totally insoluble in a particular solvent or it is poorly soluble in that particular solvent. Substantially insoluble means that a particular solute has a solubility of less than one part per 100 parts solvent. The term substantially insoluble includes the definitions of "slightly soluble" (from 100 to 1000 parts solvent per one part solute), "very slightly soluble" (from 1000 to 10,000 parts solvent per one part solute), and "practically insoluble" (more than 10,000 parts solvent per one part solute) as given in Table 16-1 of Remington: The Science and Practice of Pharmacy, 21st ed. Lippincott, Williams & Wilkins, 2006, p. 212.

The term "surfactant" as used herein refers to any agent with preferentially adsorbs to an interface between two immiscible phases, such as the interface between water and an organic polymer solution, a water/air interface, or an organic solvent/air interface. Surfactants generally possess a hydrophilic moiety and a lipophilic moiety, such that upon adsorbing to microparticles they tend to present moieties to the continuous phase that do not attract similarly-coated particles, thus reducing particle agglomeration.

A "therapeutically effective amount" is the amount of compound which achieves a therapeutic effect by inhibiting a disease or disorder in a patient or by prophylactically inhibiting or preventing the onset of a disease or disorder. A therapeutically effective amount may be an amount which relieves to some extent one or more symptoms of a disease or disorder in a patient; returns to normal either partially or completely one or more physiological or biochemical parameters associated with or causative of the disease or disorder; and/or reduces the likelihood of the onset of the disease or disorder.

The terms "chemically stable" and "chemical stability" refer to formulations wherein the individual degradation products of active agent remain below the limits specified by regulatory requirements during the shelf life of the product for human use (e.g., 1% of total chromatographic peak area per ICH guidance Q3B(R2)) and there is acceptable mass balance (e.g., as defined in ICH guidance Q1E) between active agent assay and total degradation products.

### Compositions

The compositions described herein comprise a suspension medium including a propellant, active agent particles, and suspending particles. If desired, the compositions described herein may include one or more additional constituents. Moreover, variations and combinations of components of the compositions described herein may be used. For example, the active agent particles included in the compositions may include two or more active agents; or two or more different species of active agent particles may be used, with each different species of active agent particle comprising a different active agent. In some embodiments, two or more species of suspending particles may be used in compositions for the delivery of two or more active agents or active agent particles. In some embodiments, when two or more active agent particles are present, the present composition is in form of a fixed dose combination. By "fixed dose combination", it is meant two or more active agents in a single dose form, such as a formulation in a single metered dose inhaler.

Generally, due to density differences between distinct species of particles and the medium within which they are suspended (e.g., a propellant or propellant system), buoyancy forces cause creaming of particles with lower density than the propellant and sedimentation of particles with higher density than the propellant. Therefore, in suspensions that consist of a mixture of different types of particles with different density or different tendencies to flocculate, sedimentation or creaming behavior is expected to be specific to each of the different particle types and to the specific suspension medium used, and is expected to lead to separation of the different particle types within the suspension medium.

However, the combinations of propellant, active agent particles, and suspending particles described herein provide co-suspensions wherein active agent particles and suspending particles co-locate within the propellant (i.e., the active agent particles associate with the suspending particles such that suspending particles and active agent particles do not exhibit substantial separation relative to each other, such as by differential sedimentation or creaming, even after a time sufficient for the formation of a cream or sediment layer). In particular, the active agent particles associate with the suspending particles such that there is no substantial separation of active agent particles and suspending particles within the continuous phase formed by the suspension medium under typical patient use conditions.

Compositions of propellant, active agent particles, and suspending particles according to the present description provide desirable chemical stability, suspension stability, and active agent delivery characteristics. For example, in certain embodiments, when present within an MDI canister, compositions as described herein can inhibit or reduce one or more of the following: flocculation of active agent material; differential sedimentation or creaming of active agent particles and suspending particles; solution mediated transformation of active agent material; and loss of active agent to the surfaces of the container closure system, in particular the metering valve components. Such qualities work to achieve and preserve aerosol performance as the formulation is delivered from an MDI such that desirable fine particle fraction, fine particle dose, and delivered dose uniformity characteristics are achieved and substantially maintained throughout emptying of an MDI canister within which the formulation is contained. Additionally, compositions according to the present description can provide a stable formulation that provides consistent dosing characteristics, even for potent and highly potent active agents, while using a relatively simple HFO suspension medium that does not require modification by the addition of, for example, cosolvents, antisolvents, solubilizing agents, or adjuvants. Moreover, in specific embodiments, the pharmaceutical compositions described herein can be formulated with an HFO propellant or propellant system substantially free of anti solvents, solubilizing agents, cosolvents, or adjuvants.

In some embodiments, compositions formulated according to the present teachings inhibit physical and/or chemical degradation of the active agents included therein. For example, in specific embodiments, the compositions described herein may inhibit one or more of chemical degradation, flocculation, aggregation, and solution mediated transformation of the active agents included in the compositions. The chemical and suspension stability provided by the compositions described herein provides for enhanced robustness in simulated use testing (SUT) as compared to conventional preparations. Simulated use testing includes storage of an MDI canister for five weeks at 25 °C and 75% relative humidity (RH), with no weekly cleaning of the device, and dispensing of the composition from the MDI at 25 °C and 50% RH. Enhanced robustness can take the form of consistency of shot weight (i.e., the weight of the composition dispensed upon activation of the MDI), low levels of propellant leakage, and desirable delivered dose uniformity throughout emptying of an MDI canister ("DDU"), even where the active agents to be delivered are highly potent and delivered at very low doses. For example, in some embodiments the compositions described herein exhibit less than about 10%, less than about 9%, less than about 8%, less than about 7%, less than about 6%, or less than about 5% reduced shot weight when delivered by MDI in SUT. In further embodiments, the compositions described herein exhibit less than about 1.0%, less than about 0.5%, less than about 0.4%, less than about 0.3%, less than about 0.2%, or less than about 0.1% weight loss in the MDI per year at 20 °C and 60% RH. In still further embodiments, the compositions described herein exhibit a DDU of ±20% or better, ±15% or better, or ±10% or better, throughout emptying of the MDI canister. Moreover, compositions according to the present description exhibit enhance robustness by substantially preserving FPF and FPD performance throughout emptying of an MDI canister, even after being subjected to accelerated degradation conditions. For example, in some embodiments, the compositions described herein are dispensed from an MDI at a FPF that is maintained within about 85% or within about 95% of the initial FPF. Compositions described herein provide the added benefit of achieving such performance while being formulated using HFO propellants. In specific embodiments, the compositions described herein achieve one or more of a targeted DDU, FPF, or FPD, while being formulated with suspension medium including only one or more HFO propellants and without the need to modify the characteristics of the propellant, such as by the addition of, for example, one or more cosolvent, antisolvent, solubilizing agent, adjuvant, or other propellant modifying material.

### Suspension Medium

The suspension medium included in a composition described herein includes one or more propellants. In general, suitable propellants for use as suspension mediums are those propellant gases that can be liquefied under pressure at room temperature, and upon inhalation or topical use, are safe and toxicologically innocuous. Additionally, it is desirable that the selected propellant be relatively non-reactive with the suspending particles or active agent particles. In the past, compositions for delivery by MDIs were typically formulated using chlorofluorocarbon (CFC) propellants, hydrofluoroalkanes (HFAs), or perfluorinated compounds (PFCs). Propellants comprising hydrofluoroolefins (HFOs) are considered more environmentally friendly, but several barriers existed to the use of HFOs in MDI formulations given the marked difference between HFOs and other propellants. For example, 1,1,1,2-tetrafluoroethane (also known as norflurane, HFA-134a or HFC-134a) is widely used in industrial, consumer, and pharmaceutical products as refrigerant or propellant, however, it has been demonstrated that HFOs cannot be used as a drop-in replacement of HFA-134a due to the thermodynamic differences between HFOs and HFA-134a. Also, HFOs for industrial or consumer use are not in compliance with the good manufacturing practice (GMP) regulations and are not considered to be safe for patient use. In addition, the performance of a pMDI is highly dependent on the propellant because the propellant properties impact on the suspension stability, suspension atomization, aerosol droplet diameter, etc. For example, a recent study has shown that the same suspending particles have lower suspension stability in HFO-1234ze compared to HFA-134a and HFA-227ea (Wang, H., et al., Respiratory Drug Delivery, Lisbon, Portugal, 2019). Thus, extensive experimentation would be needed to identify a formulation that would deliver the desired doses of active agent particles with desirable DDU and consistent FPF values. As shown in Table A below, the physiochemical properties vary widely among different propellants.

**Table A. Propellant Properties:**

| **Propellant** | **HFA-134a** | **HFA-227ea** | **HFC-152a** | **HFO-1234ze** | **HFO-1234yf** |
|---|---|---|---|---|---|
| Chemical Formula | C₂H₃F₄ | C₃HF₇ | C₂H₄F₂ | C₃H₂F₄ | C₃H₂F₄ |
| Molecular Weight (g/mol) | 102 | 170 | 66 | 114 | 114 |
| Liquid Density @ 20^{°}C (g/mL) | 1.23 | 1.41 | 0.91 | 1.18 | 1.11 |
| Dipole Moment (Debye) | 2.06 | 1.46 | 2.30 | 1.44 | 2.54 |
| Surface Tension @ 20^{°}C (mN/m) | 8.09 | 7.50 | 10.4 | 8.9 | 6.8 |
| Boiling Point (°C) | -26.1 | -16.5 | -24.7 | -19.0 | -29.5 |
| Liquid Viscosity @ 20^{°}C (mPa·S) | 0.211 | 0.267 | 0.171 | 0.206³ | 0.164 |
| Vapor Pressure @ 20^{°}C (kPa) | 570 | 390 | 510 | 427 | 592 |
| Water Solubility in Propellant (ppm) | 1100@25 | 610@25 | 2100@25 | 225@20 | 200@24 |
| Latent Heat @ 25^{°}C (kJ/kg) | 177.7 | 110.97 | 279.1 | 166.8 | 145.4 |
| Latent Heat @ Boiling Point (kJ/kg) | 216.7 | 131.4 | 318.4 | 195.43 | 180.5 |

Surprisingly, it was found that for compositions comprising active agent particles and suspending particles as described herein, MDI formulations comprising HFO propellants are suitable for use as inhalant medicine, despite the fact that HFOs and other propellants, e.g., HFAs, have significantly different structures and properties.

In compositions of the invention, the HFO propellant is 1,3,3,3-tetrafluoropropene, also referred to as HFO-1234ze. HFO-1234ze has a trans form ((1*E*)-1,3,3,3-tetrafluoropropene, also referred to as HFO-1234ze(E)) and a cis form ((1*Z*)-1,3,3,3-tetrafluoropropene, also referred to as HFO-1234ze(Z)). In the composition of the invention, the HFO propellant is HFO-1234ze(E), also known as *trans*-1,3,3,3-Tetrafluoroprop-1-ene. In the composition of the invention, the propellant is pharmaceutical grade HFO-1234ze(E). The term "pharmaceutical grade propellant," as used herein, indicates a propellant that is in compliance with the GMP regulations for use in humans. For example, the pharmaceutical grade propellant is consistent with the major health authorities' guidelines, such as FDA's or EMA's Guideline on The Pharmaceutical Quality of Inhalation and Nasal Products, and its specification as an excipient has been established to ensure the quality and safety of the propellant, e.g., HFO-1234ze(E), for pharmaceutical product use. The specification tests include propellant identity, appearance, assay, acidity, total residue, moisture content, related impurities, and unrelated impurities. Stability studies are also in progress to demonstrate long-term physicochemical stability. In the compositions of the invention, the pharmaceutical grade HFO-1234ze(E) has a purity of at least 99.90%. In some embodiments, the propellant is pharmaceutical grade HFO-1234ze(E) having a purity of at least about 99.91%, at least about 99.92%, at least about 99.93%, at least about 99.94%, at least about 99.95%. Pharmaceutical grade HFO-1234ze(E) is suitable for use as a propellant due to both its overall purity and the absence or low concentration of specific impurities. In some embodiments, the pharmaceutical grade HFO-1234ze(E) contains about 10 ppm or less, about 9 ppm or less, about 8 ppm or less, about 7 ppm or less, about 6 ppm or less, or about 5 ppm or less of any one of the following impurities: HFO-1234yf, HFO-1234ze(Z), HFC-125, CFC-11, HFC-245cb, HFO-1225ye(Z) or HFO-1225ye(E), CFC-113, and CFC-114. In some embodiments, the pharmaceutical grade HFO-1234ze(E) contains about 10 ppm or less, about 9 ppm or less, about 8 ppm or less, about 7 ppm or less, about 6 ppm or less, or about 5 ppm or less of HFO-1234yf. In some embodiments, the pharmaceutical grade HFO-1234ze(E) contains about 10 ppm or less, about 9 ppm or less, about 8 ppm or less, about 7 ppm or less, about 6 ppm or less, or about 5 ppm or less of HFO-1234ze(Z). In some embodiments, the pharmaceutical grade HFO-1234ze(E) contains about 10 ppm or less, about 9 ppm or less, about 8 ppm or less, about 7 ppm or less, about 6 ppm or less, or about 5 ppm or less of HFC-125. In some embodiments, the pharmaceutical grade HFO-1234ze(E) contains about 10 ppm or less, about 9 ppm or less, about 8 ppm or less, about 7 ppm or less, about 6 ppm or less, about 5 ppm or less of CFC-11. In some embodiments, the pharmaceutical grade HFO-1234ze(E) contains about 10 ppm or less, about 9 ppm or less, about 8 ppm or less, about 7 ppm or less, about 6 ppm or less, or about 5 ppm or less of HFC-245cb. In some embodiments, the pharmaceutical grade HFO-1234ze(E) contains about 10 ppm or less, about 9 ppm or less, about 8 ppm or less, about 7 ppm or less, about 6 ppm or less, or about 5 ppm or less of HFO-1225ye(Z). In some embodiments, the pharmaceutical grade HFO-1234ze(E) contains about 10 ppm or less, about 9 ppm or less, about 8 ppm or less, about 7 ppm or less, about 6 ppm or less, or about 5 ppm or less of HFO-1225ye(E). In some embodiments, the pharmaceutical grade HFO-1234ze(E) contains about 10 ppm or less, about 9 ppm or less, about 8 ppm or less, about 7 ppm or less, about 6 ppm or less, or about 5 ppm or less of CFC-113. In some embodiments, the pharmaceutical grade HFO-1234ze(E) contains about 10 ppm or less, about 9 ppm or less, about 8 ppm or less, about 7 ppm or less, about 6 ppm or less, or about 5 ppm or less of CFC-114. In some embodiments, the pharmaceutical grade HFO-1243ze(E) contains about 150 ppm or less, about 140 ppm or less, about 130 ppm or less, about 120 ppm or less, about 110 ppm or less, or about 100 ppm or less of HCFC-124. In some embodiments, the pharmaceutical grade HFO-1234ze(E) contains about 400 ppm or less, about 375 ppm or less, about 350 ppm or less, about 325 ppm or less, or about 300 ppm or less of HFC-152a.

In some embodiments, the suspension medium may be formed of a single propellant, e.g., the pharmaceutical grade HFO-1234ze(E). In certain embodiments, certain vapor pressure compounds are present in a relatively low level. Such compounds may be associated with the suspending particles.

In some embodiments, the suspension medium may be formed of a propellant or propellant system that is substantially free of additional materials, including, for example, antisolvents, solubilizing agents, stabilizing agents, cosolvents, or adjuvants.

In some embodiments, the present pharmaceutical composition, which comprises a propellant of pharmaceutical grade HFO-1234ze(E); a plurality of active agent particles; and a plurality of phospholipid particles, exhibits similar or comparable bioavailability of the active agent(s) compared to a reference pharmaceutical composition, which comprises a propellant of pharmaceutical grade HFA-134a; a plurality of active agent particles; and a plurality of phospholipid particles. As used herein, a "reference pharmaceutical composition" means an alternative pharmaceutical composition which contains the same active agent particles and the same suspending particles as the present pharmaceutical composition except the propellant. For example, the present pharmaceutical composition and the reference pharmaceutical composition comprise the same active agent particles and the same phospholipids particles, but the reference pharmaceutical composition comprises a propellant of pharmaceutical grade HFA-134a, while the present pharmaceutical composition comprises a propellant of pharmaceutical grade HFO-1234ze(E). HFA-134a is a hydrofluorocarbon with the chemical name: 1,1,1,2-tetrafluoroethane. HFA-134a has been used as a propellant in metered dose inhalers. As used herein, "bioavailability" means the proportion of an active agent which enters the circulation when introduced into the body through the lungs. In one embodiment, similar or comparable bioavailability can be shown, wherein a ratio of the geometric mean of logarithmic transformed Cmax, AUCinf or AUClast for the two products (e.g., the present pharmaceutical composition and the reference pharmaceutical composition) is about 0.80 to about 1.25 with or without the 90% confidence interval (CI) limits.

In some embodiments, the present pharmaceutical composition exhibits Cmax, AUCinf or AUClast of any one or more of the active agents, which is about 80% to about 125% of Cmax, AUCinf or AUClast of the one or more of the active agents of a reference pharmaceutical composition with geometric means. In some embodiments, the present pharmaceutical composition comprises a propellant of pharmaceutical grade HFO-1234ze(E); a plurality of active agent particles; and a plurality of phospholipid particles comprising perforated microstructures, while the reference pharmaceutical composition comprises a propellant of pharmaceutical grade HFA-134a; a plurality of active agent particles; and a plurality of phospholipid particles comprising perforated microstructures. In some embodiments, the present pharmaceutical composition and the reference pharmaceutical composition are both administered by actuating metered dose inhalers, wherein each actuation of the present pharmaceutical composition provides the same delivered dose of the active agent(s) as each actuation of the reference pharmaceutical composition does.

As used herein, Cmax, AUCinf and AUClast are pharmacokinetic measures used to determine active agent dosing. As used herein, Cmax means the highest concentration of an active agent in the blood after a dose is administered, e.g., via inhalation. As used herein, the area under the curve (AUC) is the definite integral of a curve that describes the variation of an active agent concentration in blood plasma as a function of time. As used herein, AUCinf means area under the curve from the time of dosing to the last measurable concentration and extrapolated to infinity. As used herein, AUClast means area under the curve from the time of dosing to the last measurable concentration.

The present pharmaceutical composition may exhibit Cmax of budesonide, which is about 80% to about 125% of Cmax of budesonide of a reference pharmaceutical composition. The present pharmaceutical composition may exhibit Cmax of glycopyrrolate, which is about 80% to about 125% of Cmax of glycopyrrolate of a reference pharmaceutical composition. The present pharmaceutical composition may exhibit Cmax of formoterol, which is about 80% to about 125% of Cmax of formoterol of a reference pharmaceutical composition. In some embodiment, Cmax of budesonide is the geometric mean of logarithmic transformed value. In some embodiments, the present pharmaceutical composition comprises a combination of budesonide and formoterol active agent particles. In some embodiments, the present pharmaceutical composition comprises a combination of budesonide and albuterol active agent particles. In some embodiments, the present pharmaceutical composition comprises a combination of glycopyrrolate and formoterol active agent particles.

The present pharmaceutical composition may exhibit AUCinf of budesonide, which is about 80% to about 125% of AUCinf of budesonide of a reference pharmaceutical composition. The present pharmaceutical composition may exhibit AUCinf of formoterol, which is about 80% to about 125% of AUCinf of formoterol of a reference pharmaceutical composition. In some embodiment, AUCinf of budesonide is the geometric mean of logarithmic transformed value. In some embodiments, the present pharmaceutical composition comprises a combination of budesonide and formoterol active agent particles. In some embodiments, the present pharmaceutical composition comprises a combination of budesonide and albuterol active agent particles. In some embodiments, the present pharmaceutical composition comprises a combination of glycopyrrolate and formoterol active agent particles.

The present pharmaceutical composition may exhibit AUClast of budesonide, which is about 80% to about 125% of AUClast of budesonide of a reference pharmaceutical composition. The present pharmaceutical composition may exhibit AUClast of glycopyrrolate, which is about 80% to about 125% of AUClast of glycopyrrolate of a reference pharmaceutical composition. The present pharmaceutical composition may exhibit AUClast of formoterol, which is about 80% to about 125% of AUClast of formoterol of a reference pharmaceutical composition. The present pharmaceutical composition may exhibit AUClast of budesonide and formoterol, which is about 80% to about 125% of AUClast of budesonide and formoterol of a reference pharmaceutical composition. In some embodiment, AUClast of budesonide is the geometric mean of logarithmic transformed value. In some embodiments, the present pharmaceutical composition comprises a combination of budesonide and formoterol active agent particles. In some embodiments, the present pharmaceutical composition comprises a combination of budesonide and albuterol active agent particles. In some embodiments, the present pharmaceutical composition comprises a combination of glycopyrrolate and formoterol active agent particles.

### Active Agent Particles

The active agent particles included in the compositions described herein are formed of a material capable of being dispersed and suspended within the suspension medium and are sized to facilitate delivery of respirable particles from the composition. In one embodiment, therefore, the active agent particles are provided as micronized particles wherein at least 90% of the active agent particles by volume exhibit an optical diameter of about 7 µm or less. In some embodiments, at least 90% of the active agent particles by volume exhibit an optical diameter of about 5 µm or less. In other embodiments, at least 90% of the active agent particles by volume exhibit an optical diameter selected from a range of about 7 µm to about 1 µm, about 5 µm to about 2 µm, and about 3 µm to about 2µm. In further embodiments, at least 90% of the active agent particles by volume exhibit an optical diameter selected from 6 µm or less, 5 µm or less, 4 µm or less, or 3 µm or less. In another embodiment, the active agent particles are provided as micronized particles wherein at least 50% of the active agent particles by volume exhibit an optical diameter of about 4 µm or less. In further embodiments, the active agent particles are provided as micronized particles wherein at least 50% of the active agent particles by volume exhibit an optical diameter selected from about 3 µm or less, about 2 µm or less, about 1.5 µm or less, and about 1 µm or less. In still further embodiments, the active agent particles are provided as micronized particles wherein at least 50% of the active agent particles by volume exhibit an optical diameter selected from a range of about 4 µm to about 1 µm, about 3 µm to about 1 µm, about 2 µm to about 1 µm, about 1.3 µm, and about 1.9 µm.

In certain embodiments, the active agent particles comprise glycopyrrolate and at least 90% of the active agent particles by volume exhibit an optical diameter of about 7 µm or less. In certain embodiments, the active agent particles comprise budesonide and at least 90% of the active agent particles by volume exhibit an optical diameter of about 7 µm or less. In certain embodiments, the active agent particles comprise formoterol and at least 90% of the active agent particles by volume exhibit an optical diameter of about 5 µm or less. In certain embodiments, the active agent particles comprise albuterol and at least 90% of the active agent particles by volume exhibit an optical diameter of about 5 µm or less.

The active agent particles may be formed entirely of active agent or they may be formulated to include one or more active agents in combination with one or more excipients or adjuvants. In specific embodiments, an active agent present in the active agent particles may be entirely or substantially crystalline. In another embodiment, the active agent particles may include an active agent present in both crystal and amorphous states. In yet another embodiment, the active agent particles may include an active agent present in both crystal and amorphous states. In yet a further embodiment, where two or more active agents are present in active agent particles, at least one such active agent may be present in crystalline or substantially crystalline form and at least another active agent may be present in an amorphous state. In still another embodiment, where two or more active agents are present in active agent particles, each such active agent may be present in crystalline or substantially crystalline form. Where the active agent particles described herein include one or more active agents in combination with one or more excipients or adjuvants, the excipients and adjuvants can be selected based on the chemical and physical properties of the active agent used. Suitable excipients for formulation of active agent particles include, for example, lipid, phospholipids, carbohydrates, amino acids, organic salts, peptides, proteins, alditols, synthetic or natural polymers, or surfactant materials.

Any suitable process may be employed to achieve micronized active agent particles for inclusion in the compositions described herein. A variety of processes may be used to create active agent particles suitable for use in the formulations described herein, including, but not limited to, micronization by milling or grinding processes, crystallization or recrystallization processes, processes using precipitation from supercritical or near-supercritical solvents, spray drying, spray freeze drying, or lyophilization. Patent references teaching suitable methods for obtaining micronized active agent particles include, for example, U.S. Patent No. 6,063,138, U.S. Patent No. 5,858,410, U.S. Patent No. 5,851,453, U.S. Patent No. 5,833,891, U.S. Patent No. 5,707,634, and International Patent Publication No. WO 2007/009164. Where the active agent particles include active agent material formulated with one or more excipient or adjuvant, micronized active agent particles can be formed using one or more of the preceding processes and such processes can be used to achieve active agent particles having a desired size distribution and particle configuration.

The active agent particles may be provided in any suitable concentration within the suspension medium. The active agent included in the active agent particles is substantially insoluble in the suspension medium. In some embodiments, the active agent, despite being substantially insoluble, exhibits measurable solubility in the suspension medium. However, even where the active agent exhibits measurable solubility in the suspension medium, the compositions described herein work to preserve the physical stability of such active agents. In particular, in specific embodiments, an active agent included in the compositions described herein may exhibit sufficient solubility in the suspension medium such that as much as 5% of the total active agent mass dissolves in the suspension medium. Alternatively, the solubility of an active agent may result in dissolution of as much as 1% of the total active agent mass in the suspension medium. In another embodiment, the solubility of an active agent may result in dissolution of as much as 0.5% of the total active agent mass in the suspension medium. In yet another embodiment, the solubility of an active agent may result in dissolution of as much as 0.05% of the total active agent mass in the suspension medium. In still another embodiment, the solubility of an active agent may result in dissolution of as much as 0.025% of the total active agent mass in the suspension medium.

A variety of therapeutic or prophylactic agents can be incorporated into the co-suspension compositions disclosed herein. Exemplary active agents include those that may be administered in the form of aerosolized medicaments, and active agents suitable for use in the compositions described herein include those that may be presented in a form or formulated in a manner which is dispersible within the selected suspension medium (e.g., is substantially insoluble or exhibits a solubility in the suspension medium that substantially maintains a co-suspension formulation), is capable of forming a co-suspension with the suspending particles, and is subject to respirable uptake in physiologically effective amounts. The active agents that may be utilized in forming the active agent particles described herein can have a variety of biological activities.

Where appropriate, the active agents provided in the composition, including but not limited to those specifically described herein, may be used in the form of salts (e.g., alkali metal or amine salts or as acid addition salts) or as esters, solvates (e.g., hydrates), derivatives, or a free base thereof. Additionally, the active agents may be in any crystalline form or isomeric form or mixture of isomeric forms, for example, as pure enantiomers, a mixture of enantiomers, as racemates or as mixtures thereof. In this regard, the form of the active agents may be selected to optimize the activity and/or stability of the active agent and/or to minimize the solubility of the active agent in the suspension medium.

Because the compositions disclosed enable the reproducible delivery of very low doses of active agents, in certain embodiments, the active agent included in the compositions described herein may be selected from one or more potent or highly potent active agents. For example, in certain embodiments, the compositions described herein may include one or more potent active agents that are to be delivered at a dose selected from between about 100 µg and about 100 mg, about 100 µg and about 10 mg, and about 100 µg and 1 mg per actuation of an MDI. In other embodiments, the compositions described herein may include one or more potent or highly potent active agents that are to be delivered at a dose selected from up to about 80 µg, up to about 40 µg, up to about 20 µg, between about 10 µg and about 100 µg, between about 5 µg and about 50 µg, and between about 1 µg and about 10 µg per actuation of an MDI. Additionally, in certain embodiments, the compositions described herein may include one or more highly potent active agents that are to be delivered at a dose selected from between about 0.1 and about 2 µg, about 0.1 and about 1 µg, and about 0.1 and about 0.5 µg per actuation of an MDI.

The composition of the invention contains a combination of two or more active agents. For example, a combination of two or more species of active agent particles may be co-suspended with a single species of suspending particles. Alternatively, a composition may include two or more species of active agent particles co-suspended with two or more different species of suspending particles. Even further, a composition as described herein may include two or more active agents combined within a single species of active agent particle. For example, where the active agent particles are formulated using one or more excipients or adjuvants in addition to the active agent material, such active agent particles may include individual particles that include two or more different active agents.

In certain embodiments, the active agent may be glycopyrrolate, including any pharmaceutically acceptable salts, esters, isomers or solvates thereof. In some embodiments, glycopyrrolate is present at a concentration in the range of about 0.04 mg/mL to about 2.25 mg/mL.

Glycopyrrolate can be used to treat inflammatory or obstructive pulmonary diseases and disorders such as, for example, those described herein. As an anticholinergic, glycopyrrolate acts as a bronchodilator and provides an antisecretory effect, which is a benefit for use in the therapy of pulmonary diseases and disorders characterized by increased mucus secretions. Glycopyrrolate is a quaternary ammonium salt. Where appropriate, glycopyrrolate may be used in the form of salts (e.g., alkali metal or amine salts, or as acid addition salts) or as esters or as solvates (hydrates). Additionally, the glycopyrrolate may be in any crystalline form or isomeric form or mixture of isomeric forms, for example a pure enantiomer, a mixture of enantiomers, a racemate or a mixture thereof. In this regard, the form of glycopyrrolate may be selected to optimize the activity and/or stability of glycopyrrolate and/or to minimize the solubility of glycopyrrolate in the suspension medium. Suitable counter ions are pharmaceutically acceptable counter ions including, for example, fluoride, chloride, bromide, iodide, nitrate, sulfate, phosphate, formate, acetate, trifluoroacetate, propionate, butyrate, lactate, citrate, tartrate, malate, maleate, succinate, benzoate, p-chlorobenzoate, diphenyl-acetate or triphenylacetate, o-hydroxybenzoate, p-hydroxybenzoate, 1-hydroxynaphthalene-2-carboxylate, 3-hydroxynaphthalene-2-carboxylate, methanesulfonate and benzenesulfonate. In particular embodiments of the compositions described herein, the bromide salt of glycopyrrolate, namely 3-[(cyclopentyl-hydroxyphenylacetyl)oxy]-1,1-dimethylpyrrolidinium bromide, also referred to as (RS)-[3-(SR)-Hydroxy-1,1-dimethylpyrrolidinium bromide]α-cyclopentylmandelate, is used and can be prepared according to the procedures set out in U.S. Pat. No. 2,956,062.

Where the compositions described herein include glycopyrrolate, in certain embodiments, the compositions may include sufficient glycopyrrolate to provide a target delivered dose selected from between about 1 µg and about 200 µg per actuation of an MDI, about 5 µg and about 150 µg per actuation of an MDI, about 10 µg and 100 µg per actuation of an MDI, about 5 µg and about 50 µg per actuation of an MDI, between about 2 µg and about 25 µg per actuation of an MDI, and between about 6 µg and about 15 µg per actuation of an MDI. In other such embodiments, the formulations include sufficient glycopyrrolate to provide a dose selected from up to about 200 µg, up to about 150 µg, up to about 75 µg, up to about 40 µg, up to about 20 µg, or up to about 10 µg per actuation. In yet further embodiments, the formulations include sufficient glycopyrrolate to provide a dose selected from about 2 µg per actuation, about 5 µg per actuation, about 7 µg per actuation, about 9 µg per actuation, about 18 µg per actuation, 36 µg per actuation or about 72 µg per actuation. In order to achieve targeted delivered doses as described herein, where compositions described herein include glycopyrrolate as the active agent, in specific embodiments, the amount of glycopyrrolate included in the compositions may be selected from, for example, between about 0.04 mg/mL and about 2.25 mg/mL.

In certain embodiments, the compositions described herein include formoterol, and any pharmaceutically acceptable salts, esters, isomers or solvates thereof. In some embodiments formoterol is present at a concentration in the range of about 0.01 mg/mL to about 1 mg/mL.

Formoterol can be used to treat inflammatory or obstructive pulmonary diseases and disorders such as, for example, those described herein. Formoterol has the chemical name (±)-2-hydroxy-5-[(1RS)-1-hydroxy-2-[[(1RS)-2-(4-methoxyphenyl)-1-methylethyl]-amino]ethyl]formanilide, and is commonly used in pharmaceutical compositions as the racemic fumarate dihydrate salt. Where appropriate, formoterol may be used in the form of salts (e.g. alkali metal or amine salts or as acid addition salts) or as esters or as solvates (hydrates). Additionally, the formoterol may be in any crystalline form or isomeric form or mixture of isomeric forms, for example a pure enantiomer, a mixture of enantiomers, a racemate or a mixture thereof. In this regard, the form of formoterol may be selected to optimize the activity and/or stability of formoterol and/or to minimize the solubility of formoterol in the suspension medium. Pharmaceutically acceptable salts of formoterol include, for example, salts of inorganic acids such as hydrochloric, hydrobromic, sulfuric and phosphoric acids, and organic acids such as fumaric, maleic, acetic, lactic, citric, tartaric, ascorbic, succinic, glutaric, gluconic, tricarballylic, oleic, benzoic, p-methoxybenzoic, salicylic, o- and p-hydroxybenzoic, p-chlorobenzoic, methanesulfonic, p-toluenesulfonic and 3-hydroxy-2-naphthalene carboxylic acids. Hydrates of formoterol are described, for example, in U.S. Pat. No. 3,994,974 and U.S. Pat. No. 5,684,199. Specific crystalline forms of formoterol and other β2 adrenergic receptor agonists are described, for example, in WO95/05805, and specific isomers of formoterol are described in U.S. Pat. No. 6,040,344.

In specific embodiments, the formoterol material utilized to form the formoterol particles is formoterol fumarate, and in one such embodiment, the formoterol fumarate is present in the dihydrate form. Formoterol fumarate may be referred to by the chemical name N-[2-Hydroxy-5-[(1RS)-1-hydroxy-2-[[(1RS)-2-(4-methoxyphenyl)-1-methylethyl]-amino]ethyl]phenyl]formamide (E)-2-butenedioate dehydrate. Where the compositions described herein include formoterol, in certain embodiments, the compositions described herein may include formoterol at a concentration that achieves a targeted delivered dose selected from between about 1 µg and about 30 µg, about 0.5 µg and about 10 µg, about 1 µg and about 10 µg, about 2 µg and 5 µg, about 2 µg and about 10 µg, about 3 µg and about 10 µg, about 5 µg and about 10 µg, and 3 µg and about 30 µg per actuation of an MDI. In other embodiments, the compositions described herein may include formoterol in an amount sufficient to provide a targeted delivered dose selected from up to about 30 µg, up to about 10 µg, up to about 5 µg, up to about 2.5 µg, up to about 2 µg, or up to about 1.5 µg per actuation. In yet further embodiments, the formulations include sufficient formoterol to provide a dose selected from about 2 µg per actuation, about 4.5 µg per actuation, about 4.8 µg per actuation, about 5 µg per actuation, about 10 µg per actuation, about 20 µg per actuation, or about 30 µg per actuation. In order to achieve targeted delivered doses as described herein, where compositions described herein include formoterol as the active agent, in specific embodiments, the amount of formoterol included in the compositions may be selected from, for example, between about 0.01 mg/mL and about 1 mg/mL, between about 0.01 mg/mL and about 0.5 mg/mL, and between about 0.03 mg/mL and about 0.4 mg/mL.

In certain embodiments, the composition may comprise albuterol (salbutamol), including any pharmaceutically acceptable salts, esters, isomers, or solvates thereof. Albuterol has the chemical name α¹-[(tert-butylamino)methyl]4-hydroxy-m-xylene-α,α'-diol and has an empirical formula of C₁₃H₂₁NO₃. Albuterol can be used to treat inflammatory or obstructive pulmonary diseases and disorders such as, for example, those described herein. Albuterol, pharmaceutically acceptable salts of albuterol (such as albuterol sulfate), and methods for producing the same are described, for example, in U.S. Pat. No. 3,705,233.

Where albuterol is included as a SABA active agent, in certain embodiments, the compositions described herein may include albuterol at a concentration that achieves a delivered dose selected from between about 10 µg and about 200 µg, about 20 µg and about 300 µg, about 30 µg and 150 µg, about 50 µg and about 200 µg, about 30 µg and about 100 µg, and about 1 µg and about 300 µg per actuation of an MDI. In other embodiments, the compositions described herein may include albuterol in an amount sufficient to provide a delivered dose selected from up to about 300 µg, up to about 200 µg, up to about 150 µg, up to about 100 µg, up to about 50 µg, up to about 30 µg, up to about 20 µg, or up to about 10 µg per actuation of an MDI. In yet further embodiments, the formulations include sufficient albuterol to provide a dose selected from about 20 µg, about 30 µg, about 40 µg, about 50 µg, about 60 µg, about 70 µg, about 80 µg, about 90 µg, about 100 µg, about 110 µg, about 120 µg, about 130 µg, about 140 µg, or about 150 µg per actuation. In order to achieve targeted delivered doses as described herein, where compositions described herein include albuterol as the active agent, in specific embodiments, the amount of albuterol included in the compositions may be selected from, for example, between about 0.1 mg/mL and about 10 mg/mL, between about 0.1 mg/mL and about 5 mg/mL, and between about 0.3 mg/mL and about 4 mg/mL.

In still other embodiments, the compositions described herein include budesonideIn some embodiments, budesonide is present at a concentration in the range of about 0.1 mg/mL to about 10 mg/mL.

In other embodiments, the compositions described herein include budesonide. Budesonide is suitable for use in treatment of conditions associated with pulmonary inflammation or obstruction, such as those described herein. Budesonide, which has the chemical name (RS)-11β, 16α, 17, 21-Tetrahydroxypregna-1,4-diene-3,20-dione cyclic 16,17-acetal with butyraldehyde, is also well known and described, for example, in U.S. Pat. No. 3,929,768. Where the compositions described herein include budesonide, in certain embodiments, the compositions described herein may include budesonide, including any pharmaceutically acceptable salts, esters, isomers or solvates thereof, at a concentration that achieves a targeted delivered dose selected from between about 30 µg and about 240 µg, about 30 µg and about 120 µg, between about 30 µg and about 100 µg, between about 50 µg and about 400 µg, between about 20 µg and about 600 µg, between about 50 µg and about 200 µg, between about 150 µg and about 350 µg, and between about 30 µg and about 50 µg per actuation of an MDI. In other embodiments, the compositions described herein may include budesonide, including any pharmaceutically acceptable salts, esters, isomers or solvates thereof, in an amount sufficient to provide a targeted delivered dose selected from up to about 240 µg, up to about 160 µg, up to about 120 µg, up to about 80 µg, or up to about 50 µg per actuation of an MDI. In yet further embodiments, the formulations include sufficient budesonide to provide a dose selected from about 20 µg per actuation, about 40 µg per actuation, about 80 µg per actuation, about 100 µg per actuation, about 160 µg per actuation, about 200 µg per actuation, or about 300 µg per actuation. In order to achieve targeted delivered doses as described herein, where compositions described herein include budesonide as the active agent, in specific embodiments, the amount of budesonide included in the compositions may be selected from, for example, between about 0.1 mg/mL and about 20 mg/mL, between about 0.1 mg/mL and about 5 mg/mL, and between about 0.3 mg/mL and about 6 mg/mL.

In yet further examples, the compositions described herein include a non-corticosteroid anti-inflammatory agent, such as a phosphodiesterase-4 (PDE-4) inhibitor and a Janus kinase (JAK) inhibitor. Such anti-inflammatory agents may be selected from, for example, roflumilast, apremilast, crisaborole, ruxolitinib, tofacitinib, oclacitinib, baricitinib, peficitinib, fedratinib, and upadacitinib; or any pharmaceutically acceptable salts, esters, isomers or solvates thereof. Roflumilast, pharmaceutically acceptable salts of roflumilast, and preparation of such materials are known and described, for example, in U.S. Pat. No. 8,604,064, U.S. Pat. No. 9,145,365, and U.S. Pat. No. 9,321,726. Roflumilast is suitable for use in treating diseases or disorders associated with pulmonary inflammation or obstruction, such as those described herein. Roflumilast is sometimes used for the treatment of COPD, particularly severe COPD, and is available as an oral medication. Gastrointestinal side effects are common with oral administration of roflumilast.

Where the compositions described herein include roflumilast, in certain examples, the compositions described herein may include roflumilast, including any pharmaceutically acceptable salts, esters, isomers or solvates thereof, at a concentration that achieves a targeted delivered dose selected from between about 1 µg and about 100 µg, about 5 µg and about 80 µg, about 5 µg and about 50 µg, about 5 µg and about 25 µg, about 10 µg and 25 µg, about 30 µg and about 240 µg, about 30 µg and about 120 µg, between about 30 µg and about 100 µg, between about 50 µg and about 400 µg, between about 20 µg and about 600 µg, between about 50 µg and about 200 µg, between about 150 µg and about 350 µg, and between about 30 µg and about 50 µg per actuation of an MDI. In other embodiments, the compositions described herein may include roflumilast, including any pharmaceutically acceptable salts, esters, isomers or solvates thereof, in an amount sufficient to provide a targeted delivered dose selected from up to about 240 µg, up to about 160 µg, up to about 120 µg, up to about 80 µg, or up to about 50 µg per actuation of an MDI. In yet further embodiments, the formulations include sufficient roflumilast to provide a dose selected from about 20 µg per actuation, about 40 µg per actuation, about 80 µg per actuation, about 100 µg per actuation, about 160 µg per actuation, about 200 µg per actuation, or about 300 µg per actuation. In order to achieve targeted delivered doses as described herein, where compositions described herein include roflumilast as the active agent, in specific embodiments, the amount of roflumilast included in the compositions may be selected from, for example, between about 0.1 mg/mL and about 20 mg/mL, between about 0.1 mg/mL and about 5 mg/mL, and between about 0.3 mg/mL and about 6 mg/mL.

The composition of the invention may include a combination of active agents selected from a combination of glycopyrrolate and formoterol, a combination of formoterol and budesonide, and a combination of budesonide and albuterol.

### Suspending Particles

The suspending particles included in the compositions described herein work to facilitate stabilization and delivery of the active agent included in the compositions. Though various forms of suspending particles may be used, the suspending particles are typically formed from pharmacologically inert material that is acceptable for inhalation and is substantially insoluble in the propellant selected. Generally, the majority of suspending particles are sized within a respirable range. In particular embodiments, therefore, the MMAD of the suspending particles will not exceed about 10 µm but is not lower than about 500 nm. In an alternative embodiment, the MMAD of the suspending particles is between about 5 µm and about 750 nm. In yet another embodiment, the MMAD of the suspending particles is between about 1 µm and about 3 µm. When used in an embodiment for nasal delivery from an MDI, the MMAD of the suspending particles is between 10 µm and 50 µm.

In order to achieve respirable suspending particles within the MMAD ranges described, the suspending particles will typically exhibit a volume median optical diameter between about 0.2 µm and about 50 µm. In one embodiment, the suspending particles exhibit a volume median optical diameter that does not exceed about 25 µm. In another embodiment, the suspending particles exhibit a volume median optical diameter selected from between about 0.5 µm and about 15 µm, between about 1.5 µm and about 10 µm, and between about 2 µm and about 5 µm.

The concentration of suspending particles included in a composition according to the present description can be adjusted, depending on, for example, the amount of active agent particles and suspension medium used. In one embodiment, the suspending particles are included in the suspension medium at a concentration selected from about 0.1 mg/mL to about 15 mg/mL, about 0.1 mg/mL to about 10 mg/mL, 1 mg/mL to about 15 mg/mL, about 3 mg/mL to about 10 mg/mL, 5 mg/mL to about 8 mg/mL, and about 6 mg/mL. In another embodiment, the suspending particles are included in the suspension medium at a concentration of up to about 30 mg/mL. In yet another embodiment, the suspending particles are included in the suspension medium at a concentration of up to about 25 mg/mL.

The relative amount of suspending particles to active agent particles is selected to achieve a co-suspension as contemplated herein. A co-suspension composition may be achieved where the amount of suspending particles, as measured by mass, exceeds that of the active agent particles. For example, in specific embodiments, the ratio of the total mass of the suspending particles to the total mass of active agent particles may be between about 3:1 and about 15:1, or alternatively from about 2:1 and 8:1. Alternatively, the ratio of the total mass of the suspending particles to the total mass of active agent particles may be above about 1, such as up to about 1.5, up to about 5, up to about 10, up to about 15, up to about 17, up to about 20, up to about 30, up to about 40, up to about 50, up to about 60, up to about 75, up to about 100, up to about 150, and up to about 200, depending on the nature of the suspending particles and active agent particles used. In further embodiments, the ratio of the total mass of the suspending particles to the total mass of the active agent particles may be selected from between about 10 and about 200, between about 60 and about 200, between about 15 and about 60, between about 15 and about 170, between about 15 and about 60, about 16, about 60, and about 170.

In other embodiments, the amount of suspending particles, as measured by mass, is less than that of the active agent particles. For example, in particular embodiments, the mass of the suspending particles may be as low as 20% of the total mass of the active agent particles. However, in some embodiments, the total mass of the suspending particles may also approximate or equal the total mass of the active agent particles.

Suspending particles suitable for use in the compositions described herein may be formed of one or more pharmaceutically acceptable materials or excipients that are suitable for inhaled delivery and do not substantially degrade or dissolve in the suspension medium. In one embodiment, perforated microstructures, as defined herein, may be used as the suspending particles. Suspending particles and perforated microstructures for use as suspending particles, and methods for preparation thereof, are described in U.S. Patent No. 8,815,258 and U.S. Patent No. 9,463,161, and in U.S. Patent Application Publication 2011/0135737.

Phospholipids from both natural and synthetic sources may be used in preparing suspending particles comprising perforated microstructures suitable for use in the compositions described herein. In particular embodiments, the phospholipid chosen will have a gel to liquid crystal phase transition of greater than about 400°C. Exemplary phospholipids are relatively long chain (i.e., C16-C22) saturated lipids and may comprise saturated phospholipids, such as saturated phosphatidylcholines having acyl chain lengths of 16 C or 18 C (palmitoyl and stearoyl). Exemplary phospholipids include phosphoglycerides such as dipalmitoylphosphatidylcholine, disteroylphosphatidylcholine, diarachidoylphosphatidylcholine, dibehenoylphosphatidylcholine, diphosphatidyl glycerol, short-chain phosphatidylcholines, long-chain saturated phosphatidylethanolamines, long-chain saturated phosphatidylserines, long-chain saturated phosphatidylglycerols, and long-chain saturated phosphatidylinositols. Additional excipients are disclosed in International Patent Publication No. WO 96/32149 and U.S. Patent Nos. 6,358,530, 6,372,258 and 6,518,239. In certain embodiments, the suspending particles are phospholipid particles comprising 1,2-Distearoyl-sn-glycero-3-phosphocholine (DSPC).

In another aspect, the suspending particles utilized in the compositions described herein may be selected to increase storage stability of the selected active agent, similar to that disclosed in International Patent Publication No. WO 2005/000267. For example, in one embodiment, the suspending particles my include pharmaceutically acceptable glass stabilization excipients having a Tg of at least 55 °C, at least 75 °C, or at least 100 °C. Glass formers suitable for use in compositions described herein include, but are not limited to, one or more of trileucine, sodium citrate, sodium phosphate, ascorbic acid, inulin, cyclodextrin, polyvinyl pyrrolidone, mannitol, sucrose, trehalose, lactose, and, proline. Examples of additional glass-forming excipients are disclosed in U. S. Patent Nos. RE 37,872, 5,928,469, 6,258,341, and 6,309,671. In particular embodiments, suspending particles may include a calcium salt, such as calcium chloride, as described, for example, in U.S. Patent No. 7,442,388.

In certain embodiments, the suspending particles are perforated microstructures comprising DSPC and calcium chloride. In some embodiments, the perforated microstructures comprise about 93% or more of DSPC and about 7% or less of calcium chloride. In some embodiments, the perforated microstructures comprise about 94% of DSPC and about 6% of calcium chloride. In some embodiments, the perforated microstructures comprise about 95% of DSPC and about 5% of calcium chloride.

The suspending particles may be designed, sized and shaped as desired to provide desirable stability and active agent delivery characteristics. In one exemplary embodiment, the suspending particles comprise perforated microstructures as described herein. Where perforated microstructures are used as suspending particles in the compositions described herein, they may include at least one of the following: lipids, phospholipids, nonionic detergents, nonionic block copolymers, ionic surfactants, biocompatible fluoronated surfactants and combinations thereof, particularly those approved for pulmonary use. Specific surfactants that may be used in the preparation of perforated microstructures include poloxamer 188, poloxamer 407 and poloxamer 338. Other specific surfactants include oleic acid or its alkali salts. In one embodiment, the perforated microstructures include greater than about 10% w/w surfactant.

Furthermore, suspending particles as described herein may include bulking agents, such as polymeric particles. Polymeric polymers may be formed from biocompatible and/or biodegradable polymers, copolymers or blends. In one embodiment, polymers capable of forming aerodynamically light particles may be used, such as functionalized polyester graft copolymers and biodegradable polyanhydrides. For example, bulk eroding polymers based on polyesters including poly(hydroxy acids) can be used. Polyglycolic acid (PGA), polylactic acid (PLA) or copolymers thereof may be used to form suspending particles. The polyester may include a charged or functionalizable group, such as an amino acid. For example, suspending particles may be formed of poly(D,t-lactic acid) and/or poly(D, 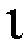 -lactic-co- glycolic acid) (PLGA), which incorporate a surfactant such as DPPC.

Other potential polymer candidates for use in suspending particles may include polyamides, polycarbonates, polyalkylenes such as polyethylene, polypropylene, poly(ethylene glycol), poly(ethylene oxide), polyethylene terephthalate), polyvinyl compounds such as polyvinyl alcohols, polyvinyl ethers, and polyvinyl esters, polymers of acrylic and methacrylic acids, celluloses and other polysaccharides, and peptides or proteins, or copolymers or blends thereof. Polymers may be selected with or modified to have the appropriate stability and degradation rates in vivo for different controlled drug delivery applications.

In an embodiment of a composition as described herein that includes one or more of glycopyrrolate, formoterol, budesonide, and albuterol as an active agent, the ratio of the total mass of the suspending particles to the total mass of the active agent particles may be selected from between about 1 and about 20, between about 1 and about 15, between about 1.5 and about 10, between about 2.5 and about 15, between about 2.5 and about 10, between about 2.5 and about 8, between about 10 and about 30, between about 15 and about 25, between about 10 and about 200, between about 50 and about 125, and between about 5 and about 50.

In some embodiments, suspending particles may be prepared by forming an oil-in-water emulsion, using a fluorocarbon oil (e.g., perfluorooctyl bromide, perfluorodecalin) which may be emulsified using a surfactant such as a long chain saturated phospholipid. The resulting perfluorocarbon in water emulsion may be then processed using a high pressure homogenizer to reduce the oil droplet size. The perfluorocarbon emulsion may be fed into a spray dryer. As is well known, spray drying is a one-step process that converts a liquid feed to a dried particulate form. Spray drying has been used to provide powdered pharmaceutical material for various administrative routes, including inhalation. In the context of spray drying, a fluorocarbon oil such as described above may function as a blowing agent. Operating conditions of the spray dryer (such as inlet and outlet temperature, feed rate, atomization pressure, flow rate of the drying air and nozzle configuration) can be adjusted to produce the desired particle size producing a yield of the resulting dry microstructures. Such methods of producing exemplary perforated microstructures are disclosed in U.S. Patent No. 8,815,258, U.S. Patent No. 9,463,161, and U.S. Patent Application Publication 2011/0135737,

The compositions described herein may include two or more species of suspending particles. For example, the compositions described herein may include a single species of active agent particle and two or more species of suspending particles. Alternatively, in other embodiments, the compositions described herein may include two or more species of active agent particles combined with two or more species of suspending particles.

### Embodiments of Compositions

In an embodiment of a composition as described herein, the ratio of the total mass of the suspending particles to the total mass of the active agent particles may be selected from between about 1 and about 20, between about 1 and about 15, between about 1.5 and about 10, between about 2.5 and about 15, between about 2.5 and about 10, between about 2.5 and about 8, between about 10 and about 30, between about 15 and about 25, between about 10 and about 200, between about 50 and about 125, and between about 5 and about 50. In all embodiments, the ratio of the active agents to the suspending particles is based on the free form (e.g., free acid or free base form) of the active agents. In an embodiment, the composition is administered by oral inhalation. In a certain embodiment, the composition described herein may be contained in a reservoir in a metered dose inhalation (MDI) device. In some embodiments, the composition described herein may include budenoside, including any pharmaceutically acceptable salts, esters, isomers or solvates thereof, at a concentration that achieves a targeted delivered dose selected from between about 70 µg to about 170 µg, between about 75 µg to about 165 µg, and between about 80 µg to about 160 µg, of budenoside per inhalation. In some embodiments, the composition described herein may include glycopyrrolate, including any pharmaceutically acceptable salts, esters, isomers or solvates thereof, at a concentration that achieves a targeted delivered dose selected from between about 5 µg to about 10 µg, and between about 5 µg to about 15 µg of glycopyrrolate per inhalation. In some embodiments, the composition described herein may include formoterol, including any pharmaceutically acceptable salts, esters, isomers or solvates thereof, at a concentration that achieves a targeted delivered dose selected from between about 1µg to about 5 µg and between about 2 µg to about 4 µg of formoterol per inhalation. In some embodiments, the composition may be administered as two inhalations per dose in two doses per day. Table 1 shows an exemplary composition comprising a combination of two or more active agents containing glycopyyrrolate, formoterol, and budesonide as active agents. The exemplary composition of Table 1 can provide a delivered dose of about 160 µg budesonide, about 9 µg glycopyrronium bromide and about 4.8 µg formoterol fumarate per actuation of the metered dose inhaler.

**Table 1. Budesonide, glycopyrronium bromide and formoterol fumarate pressurized inhalation suspension with HFO-1234ze(E) propellant**

| **Components** | **Function** | **Amount (per canister)** | **Weight %** |
|---|---|---|---|
| Budesonide, micronized | API | 31.05 mg | 0.2986 |
| Glycopyrronium bromide, micronized | API | 1.40 mg | 0.0134 |
| Formoterol fumarate, micronized | API | 0.93 mg | 0.0090 |
| Porous particles of DSPC | Suspending particles | 53.30 mg | 0.5125 |
| HFO-1234ze(E) | Propellant | 10.31 g | 99.1665 |

In one embodiment, a composition described herein comprising a combination of two or more active agents may contain glycopyrrolate and formoterol as active agents. In an embodiment of a composition as described herein, that includes, glycopyrrolate and formoterol as active agents, the ratio of the total mass of the suspending particles to the total mass of the active agent particles may be selected from between about 1 and about 25, between about 1 and about 20, between about 1.5 and about 10, between about 2.5 and about 15, between about 2.5 and about 10, between about 2.5 and about 8, between about 10 and about 30, between about 15 and about 25, between about 10 and about 200, between about 50 and about 125, and between about 5 and about 50. In all embodiments, the ratio of the active agents to the suspending particles is based on the free base form of the active agents. In an embodiment, the composition is administered by oral inhalation. In a certain embodiment, the composition as described herein that includes glycopyrrolate and formoterol, as active agents may be contained in a reservoir in a metered dose inhalation (MDI) device. In some embodiments, the composition as described herein may include glycopyrrolate, including any pharmaceutically acceptable salts, esters, isomers or solvates thereof, at a concentration that achieves a targeted delivered dose selected from between about 5 µg to about 10 µg, and between about 5 µg to about 15 µg of glycopyrrolate per inhalation. In some embodiments, the composition as described herein may include formoterol, including any pharmaceutically acceptable salts, esters, isomers or solvates thereof, at a concentration that achieves a targeted delivered dose selected from between about 1 µg to about 5 µg of formoterol per inhalation. In some embodiments, the composition may be administered as two inhalations per dose in two doses per day. In some embodiments, a composition described herein comprising about 0.011% to about 0.016% by weight (w/w) glycopyrronium bromide, about 0.007% to about 0.011% by weight (w/w) formoterol fumarate, about 0.411% to about 0.617% by weight (w/w) DSPC porous particles, and HFO-1234ze(E). In some embodiments, a composition described herein comprising about 0.012% to about 0.015% by weight (w/w) glycopyrronium bromide, about 0.008% to about 0.010% by weight (w/w) formoterol fumarate, about 0.411% to about 0.617% by weight (w/w) DSPC porous particles, and HFO-1234ze(E). In some embodiments, a composition described herein comprising about 0.013% to about 0.014% by weight (w/w) glycopyrronium bromide, about 0.008% to about 0.010% by weight (w/w) formoterol fumarate, about 0.488% to about 0.540% by weight (w/w) DSPC porous particles, and HFO-1234ze(E). Table 2 shows an exemplary embodiment of a composition containing glycopyyrrolate and formoterol as active agents. In one embodiment, the exemplary composition of Table 2 can provide a delivered dose of about 9 µg glycopyrronium bromide and about 4.8 µg formoterol fumarate per actuation of the metered dose inhaler.

**Table 2. Glycopyrronium bromide and formoterol fumarate pressurized inhalation suspension with HFO-1234ze(E) propellant**

| **Components** | **Function** | **Quantity (per canister)** | **Weight%** |
|---|---|---|---|
| Glycopyrronium bromide, micronized | API | 1.40 mg | 0.0135 |
| Formoterol Fumarate, micronized | API | 0.93 mg | 0.0090 |
| Porous particles of DSPC | Suspending particles | 53.30 mg | 0.5142 |
| HFO-1234ze(E) | Propellant | 10.31 g | 99.4633 |

In one embodiment, a composition described herein comprising a combination of two or more active agents may contain budesonide and albuterol sulfate as active agents. In an embodiment of a composition as described herein, that includes budesonide and albuterol sulfate as active agents, the ratio of the total mass of the suspending particles to the total mass of the active agent particles may be selected from between about 1 and about 25, between about 1 and about 20, between about 1.5 and about 10, between about 2.5 and about 15, between about 2.5 and about 10, between about 2.5 and about 8, between about 10 and about 30, between about 15 and about 25, between about 10 and about 200, between about 50 and about 125, and between about 5 and about 50. In all embodiments, the ratio of the active agents to the suspending particles is based on the free base form of the active agents. In an embodiment, the composition is administered by oral inhalation. In a certain embodiment, the composition as described herein that includes budesonide and albuterol sulfate, as active agents, may be contained in a reservoir in a metered dose inhalation (MDI) device. In some embodiments, the composition described herein may include budesonide, including any pharmaceutically acceptable salts, esters, isomers or solvates thereof, at a concentration that achieves a targeted delivered dose selected from between about 35µg to about 90 µg, and between about 40 µg to about 85 µg, and between about 45 µg to about 80 µg of budesonide per inhalation. In some embodiments, the composition described herein may include albuterol sulfate, including any pharmaceutically acceptable salts, esters, isomers or solvates thereof, at a concentration that achieves a targeted delivered dose selected from between about 75µg to about 95 µg and between 80 µg to about 90 µg of albuterol sulfate per inhalation. In some embodiments, the composition may be administered as two inhalations per dose in two doses per day. In some embodiments, a composition described herein comprising about 0.058% to about 0.088% by weight (w/w) budesonide, about 0.131% to about 0.197% by weight (w/w) albuterol sulfate, about 0.267% to about 0.401% by weight (w/w) DSPC porous particles, and HFO-1234ze(E). In some embodiments, a composition described herein comprising about 0.065% to about 0.080% by weight (w/w) budesonide, about 0.147% to about 0.180% by weight (w/w) albuterol sulfate, about 0.301% to about 0.367% by weight (w/w) DSPC porous particles, and HFO-1234ze(E). In some embodiments, a composition described herein comprising about 0.069% to about 0.077% by weight (w/w) budesonide, about 0.156% to about 0.172% by weight (w/w) albuterol sulfate, about 0.317% to about 0.351% by weight (w/w) DSPC porous particles, and HFO-1234ze(E). In some embodiments, a composition described herein comprising about 0.116% to about 0.175% by weight (w/w) budesonide, about 0.131% to about 0.197% by weight (w/w) albuterol sulfate, about 0.266% to about 0.400% by weight (w/w) DSPC porous particles, and HFO-1234ze(E). In some embodiments, a composition described herein comprising about 0.131% to about 0.161% by weight (w/w) budesonide, about 0.147% to about 0.180% by weight (w/w) albuterol sulfate, about 0.300% to about 0.366% by weight (w/w) DSPC porous particles, and HFO-1234ze(E). In some embodiments, a composition described herein comprising about 0.138% to about 0.153% by weight (w/w) budesonide, about 0.156% to about 0.172% by weight (w/w) albuterol sulfate, about 0.316% to about 0.350% by weight (w/w) DSPC porous particles, and HFO-1234ze(E). Tables 3A and 3B show two exemplary embodiments of a composition containing budesonide and albuterol sulfate as active agents. In one embodiment, the exemplary composition of Table 3A can provide a delivered dose of about 40 µg budesonide and about 90 µg albuterol sulfate per actuation of the metered dose inhaler. In one embodiment, the exemplary composition of Table 3B can provide a delivered dose of about 80 µg budesonide and about 90 µg albuterol sulfate per actuation of the metered dose inhaler.

**Table 3A. Budesonide and albuterol sulfate pressurized inhalation suspension with HFO-1234ze(E) propellant**

| **Components** | **Function** | **Amount (per canister)** | **Weight%** |
|---|---|---|---|
| Budesonide, micronized | API | 7.60 mg | 0.073 |
| Albuterol Sulfate, micronized | API | 17.0 mg | 0.164 |
| Porous Particles of DSPC | Suspending particles | 34.60 mg | 0.334 |
| HFO-1234ze(E) | Propellant | 10.31 g | 99.429 |

**Table 3B. Budesonide and albuterol sulfate pressurized inhalation suspension with HFO-1234ze(E) propellant, 80/90 µg per actuation**

| **Components** | **Function** | **Amount (per canister)** | **Weight%** |
|---|---|---|---|
| Budesonide, micronized | API | 15.1 mg | 0.146 |
| Albuterol Sulfate, micronized | API | 17.0 mg | 0.164 |
| Porous Particles of DSPC | Suspending particles | 34.60 mg | 0.333 |
| HFO-1234ze(E) | Propellant | 10.31 g | 99.357 |

In one embodiment, a composition described herein comprising a combination of two or more active agents may contain budesonide and formoterol as active agents. In an embodiment of a composition as described herein, that includes budesonide and formoterol as active agents, the ratio of the total mass of the suspending particles to the total mass of the active agent particles may be selected from between about 1 and about 25, between about 1 and about 20, between about 1.5 and about 10, between about 2.5 and about 15, between about 2.5 and about 10, between about 2.5 and about 8, between about 10 and about 30, between about 15 and about 25, between about 10 and about 200, between about 50 and about 125, and between about 5 and about 50. In all embodiments, the ratio of the active agents to the suspending particles is based on the free base form of the active agents. In an embodiment, the composition is administered by oral inhalation. In a certain embodiment, the composition as described herein that includes budesonide and formoterol, as active agents may be contained in a reservoir in a metered dose inhalation (MDI) device. In some embodiments, the composition may include budenoside, including any pharmaceutically acceptable salts, esters, isomers or solvates thereof, at a concentration that achieves a targeted delivered dose selected from between about 70 µg to about 170 µg, between about 75 µg to about 165 µg, and between about 80 µg to about 160 µg, of budenoside per inhalation. In some embodiments, the composition may include formoterol, including any pharmaceutically acceptable salts, esters, isomers or solvates thereof, at a concentration that achieves a targeted delivered dose selected from between about 1µg to about 5 µg and between about 2 µg to about 4 µg of formoterol per inhalation. In some embodiments, the composition may be administered as two inhalations per dose in two doses per day. In some embodiments, a composition described herein comprising about 0.238% to about 0.358% by weight (w/w) budesonide, about 0.007% to about 0.011% by weight (w/w) formoterol fumarate, about 0.410% to about 0.615% by weight (w/w) DSPC porous particles, and HFO-1234ze(E). In some embodiments, a composition described herein comprising about 0.268% to about 0.329% by weight (w/w) budesonide, about 0.008% to about 0.010% by weight (w/w) formoterol fumarate, about 0.461% to about 0.564% by weight (w/w) DSPC porous particles, and HFO-1234ze(E). In some embodiments, a composition described herein comprising about 0.283% to about 0.314% by weight (w/w) budesonide, about 0.008% to about 0.010% by weight (w/w) formoterol fumarate, about 0.487% to about 0.538% by weight (w/w) DSPC porous particles, and HFO-1234ze(E). In some embodiments, a composition described herein comprising about 0.119% to about 0.180% by weight (w/w) budesonide, about 0.007% to about 0.011% by weight (w/w) formoterol fumarate, about 0.410% to about 0.616% by weight (w/w) DSPC porous particles, and HFO-1234ze(E). In some embodiments, a composition described herein comprising about 0.134% to about 0.165% by weight (w/w) budesonide, about 0.008% to about 0.010% by weight (w/w) formoterol fumarate, about 0.462% to about 0.565% by weight (w/w) DSPC porous particles, and HFO-1234ze(E). In some embodiments, a composition described herein comprising about 0.142% to about 0.157% by weight (w/w) budesonide, about 0.008% to about 0.010% by weight (w/w) formoterol fumarate, about 0.487% to about 0.539% by weight (w/w) DSPC porous particles, and HFO-1234ze(E). Tables 4A and 4B show two exemplary embodiments of a composition containing budesonide and formoterol as active agents. In one embodiment, the exemplary composition of Table 4A can provide a delivered dose of about 160 µg budesonide and about 4.8 µg formoterol fumarate per actuation of the metered dose inhaler. In one embodiment, the exemplary composition of Table 4B can provide a delivered dose of about 80 µg budesonide and about 4.8 µg formoterol fumarate per actuation of the metered dose inhaler.

**Table 4A. Budesonide and formoterol fumarate pressurized inhalation suspension with HFO-1234ze(E) propellant**

| **Components** | **Function** | **Quantity (per canister)** | **% Weight** |
|---|---|---|---|
| Budesonide, micronized | API | 31.05 | 0.2987 |
| Formoterol Fumarate, micronized | API | 0.93 mg | 0.0089 |
| Porous Particles of DSPC | Suspending particles | 53.30 mg | 0.5127 |
| HFO-1234ze(E) | Propellant | 10.31 g | 99.1797 |

**Table 4B. Budesonide and formoterol fumarate pressurized inhalation suspension with HFO-1234ze(E) propellant**

| **Components** | **Function** | **Quantity (per canister)** | **% Weight** |
|---|---|---|---|
| Budesonide, micronized | API | 15.525 mg | 0.1496 |
| Formoterol Fumarate, micronized | API | 0.93 mg | 0.0090 |
| Porous Particles of DSPC | Suspending particles | 53.30 mg | 0.5135 |
| HFO-1234ze(E) | Propellant | 10.31 g | 99.3279 |

Compositions formulated according to the present teachings can inhibit degradation of active agent included therein. For example, in specific embodiments, the compositions described herein inhibit one or more of flocculation, aggregation and the solution mediated transformation of active agent material included in the compositions. The pharmaceutical compositions described herein are suited for respiratory delivery via an MDI in a manner that achieves desirable delivered dose uniformity ("DDU") of each active agent included in a combination of two or more active agents, even with combinations including potent and highly potent actives. As is illustrated in detail in the Examples included herein, even when delivering very low doses of two or more active agents, compositions described herein can achieve a DDU of ± 30%, or better, for each active agent throughout emptying of an MDI canister. In one such embodiment, compositions described herein achieve a DDU of ± 25%, or better, for each active agent throughout emptying of an MDI canister. In another such embodiment, compositions described herein achieve a DDU for the active agent of ± 20%, or better, for each active agent throughout emptying of an MDI canister. In further embodiments, compositions described herein achieve a DDU for the active agent of ±15%, or better, for each active agent throughout emptying of an MDI canister. In still further embodiments, compositions described herein achieve a DDU for the active agent of ±10%, or better, for each active agent throughout emptying of an MDI canister.

Pharmaceutical compositions described herein also serve to substantially preserve FPF and FPD performance throughout emptying of an MDI canister, even after being subjected to accelerated degradation conditions. For instance, compositions according to the present description maintain as much as 80%, 85%, 90%, 95%, or more, of the original FPF and FPD performance throughout emptying of an MDI canister, even after being subjected to accelerated degradation conditions. Compositions described herein provide the added benefit of achieving such performance while being formulated using non-CFC and non-HFA propellants and eliminating or substantially avoiding combination effects often experienced with compositions incorporating multiple active agents. In specific embodiments, the compositions described herein achieve one or all of a targeted DDU, FPF and FPD performance while being formulated with suspension medium including only one or more HFO propellant and without the need to modify the characteristics of the HFO propellant, such as by the addition of, for example, one or more cosolvent, antisolvent, solubilizing agent, adjuvant or other propellant modifying material.

### Methods

Compositions formulated according to the present teachings can inhibit degradation of the active agent included therein. For example, in specific embodiments, the compositions described herein inhibit one or more of flocculation, aggregation and Ostwald ripening of the active agent(s) included in the compositions. The stability provided by the compositions described herein allows the compositions to be dispensed in a manner that achieves desirable delivered dose uniformity throughout emptying of an MDI canister ("DDU"), even where the active agent to be delivered is highly potent and the delivered dose of the active agent is selected from, for example, less than one of 100 µg , 80 µg, 40 µg, 20 µg, 10 µg, 9 µg, 8 µg, 7 µg, 6 µg, 5 µg, 4 µg, 3 µg, 2 µg, 1 µg, 0.5 µg, and 0.1 µg per actuation of the MDI. As is described in detail in the Examples included herein, even at low doses of highly potent active agents, compositions described herein can achieve a DDU of ± 30%, or better, for each of the active agents included in the composition. In an alternative embodiment, compositions described herein achieve a DDU of ± 25%, or better, for each of the active agents included in the composition. In yet further embodiments, compositions described herein achieve a DDU of ± 20%, or better, ±15%, or better, or ±10%, or better, for each of the active agents included in the composition.

Moreover, compositions according to the present description serve to substantially preserve FPF and FPD performance throughout emptying of an MDI canister, even after being subjected to accelerated degradation conditions. For instance, compositions according to the present description maintain as much as 80%, 85%, 90%, 95%, or more, of the original FPF and FPD performance, even when they incorporate multiple active agents. Compositions described herein provide the added benefit of achieving such performance while being formulated using non-CFC and non-HFA propellants. In specific embodiments, the compositions described herein achieve desired one or all of a targeted DDU, FPF and FPD performance while being formulated with suspension medium including only one or more HFO propellant and without the need to modify the characteristics of the HFO propellant, such as by the addition of, for example, one or more cosolvent, antisolvent, solubilizing agent, adjuvant or other propellant modifying material.

The stability and physical characteristics of the compositions described herein support several methods. For example, in one embodiment, a method of formulating a pharmaceutical composition for respiratory delivery of an active agent is provided herein. The method involves the steps of providing a suspension medium comprising an HFO propellant, one or more species of active agent particles and one or more species of suspending particles, as described herein, and combining such constituents to form a composition wherein active agent particles associate with the suspending particles such that a co-suspension as described herein is formed. In one such embodiment, the association of the active agent particles and the suspending particles is such that they do not separate due to their different buoyancies in a propellant. As will be appreciated, a method of formulating a pharmaceutical composition as described herein can include providing two or more species of active agent particles in combination with one or more species of suspending particles. Alternatively, the method may include providing two or more suspending particles in combination with one or more species of active agent particles.

In further embodiments the compositions described herein support, for example, methods for forming stabilized formulations of active agents for pulmonary delivery, methods for preserving the FPF and/or FPD throughout emptying of an MDI canister, methods for pulmonary delivery of potent or highly potent active agents, and methods of achieving a DDU selected from ± 30%, or better, ± 25%, or better, ± 20%, or better, ± 15%, or better, and ± 10%, or better, for potent and highly potent drugs administered through pulmonary delivery.

In methods involving pulmonary delivery of active agents using compositions described herein, the compositions may be delivered by an MDI. Therefore, in particular embodiments of such methods, an MDI loaded with a composition described herein is obtained, and the desired active agent is administered to a patient through pulmonary delivery through actuation of the MDI. For example, in one embodiment, after shaking the MDI device, the mouthpiece is inserted into a patient's mouth between the lips and teeth. The patient typically exhales deeply to empty the lungs and then takes a slow deep breath while actuating the cartridge of the MDI. When actuated, the specified volume of formulation travels to the expansion chamber, out the actuator nozzle and into a high-velocity spray that is drawn into the lungs of a patient. In some embodiments the dose of active agent delivered throughout emptying of an MDI canister is not more than 20% greater than the mean delivered dose and is not less than 20% less than the mean delivered dose. In some embodiments, the dose of active agent delivered throughout emptying of an MDI canister is not more than 15% greater or less than the mean delivered dose. In some embodiments, the dose of active agent delivered throughout emptying of an MDI canister is not more than 10% greater or less than the mean delivered dose.

In specific embodiments of methods for providing a stabilized formulation of active agent for pulmonary delivery, the present disclosure provides methods for inhibiting solution mediated transformation of an active agent in a pharmaceutical formulation for pulmonary delivery. In one embodiment, a suspension medium as described herein, such as a suspension medium formed by an HFO propellant, is obtained. Suspending particles are also obtained or prepared as described herein. One or more species of active agent particles as described herein are also obtained, and the suspension medium, suspending particles and active agent particles are combined to form a co-suspension wherein the active agent particles associate with suspending particles within the continuous phase formed by the suspension medium. When compared to the active agent contained in the same suspension medium in the absence of suspending particles, co-suspensions according to the present description have been found to exhibit a higher tolerance to solution mediated transformation and irreversible crystal aggregation, and thus can lead to improved stability and dosing uniformity, allowing the formulation of active agents that are somewhat physically unstable in the suspension medium alone.

In specific embodiments of methods for preserving the FPF and/or FPD provided by a pharmaceutical formulation for pulmonary delivery of a respirable co-suspension as described herein is provided which is capable of maintaining the FPD and/or the FPF to within ± 20%, ± 15%, ± 10%, or even ± 5% the initial FPD and/or FPF, respectively, throughout emptying of an MDI canister. Such performance can be achieved even after the co-suspension is subjected to accelerated degradation conditions. In one embodiment, a suspension medium as described herein, such as a suspension medium formed by an HFO propellant, is obtained. Suspending particles are also obtained or prepared as described herein. One or more species of active agent particles as described herein are also obtained, and the suspension medium, suspending particles and active agent particles are combined to form a co-suspension wherein the active agent particles associate with suspending particles within the suspension medium. Even after exposure of such composition to one or more temperature cycling events, the co-suspension maintains an FPD or FPF within ± 20%, ± 15%, ± 10%, or even ± 5% of the respective values measured prior to exposure of the composition to the one or more temperature cycling events.

Methods for treating patients suffering from an inflammatory or obstructive pulmonary disease or condition are disclosed herein. In specific examples, such methods include pulmonary delivery of a therapeutically effective amount of a pharmaceutical composition described herein, and in certain such examples, pulmonary administration of the pharmaceutical composition is accomplished by delivering the composition using an MDI. In certain examples, the compositions, methods and systems described herein can be used to treat patients suffering from a disease or disorder selected from asthma, chronic obstructive pulmonary disease (COPD), exacerbation of airways hyper reactivity consequent to other drug therapy, allergic rhinitis, sinusitis, pulmonary vasoconstriction, inflammation, allergies, impeded respiration, respiratory distress syndrome, pulmonary hypertension, pulmonary vasoconstriction, and any other respiratory disease, condition, trait, genotype or phenotype that can respond to the administration of, for example, a LAMA, LABA, SABA, ICS, non-corticosteroid anti-inflammatory agent, or other active agent as described herein, whether alone or in combination with other therapies. In certain embodiments, the compositions described herein can be used to treat pulmonary inflammation and obstruction associated with cystic fibrosis. In specific examples of methods for treating patients suffering from an inflammatory or obstructive pulmonary disease or condition, the pulmonary disease of condition is selected from those specifically described herein, and the method includes pulmonary delivery of a composition according to the present description to the patient via an MDI, wherein the pulmonary delivery of such composition includes administering one or more active agents at a dose or dose range as described in association with the compositions disclosed herein.

### Metered Dose Inhaler Systems

As described in relation to the methods provided herein, the compositions disclosed herein may be used in an MDI system. MDIs are configured to deliver a specific amount of a medicament in aerosol form. In one embodiment, an MDI system includes a pressurized, liquid phase formulation-filled canister disposed in an actuator formed with a mouthpiece. The MDI system may include the formulations described herein, which include a suspension medium comprising an HFO propellant, at least one species of active agent particles and at least one species of suspending particles. The canister used in the MDI be any of any suitable configuration, and in one exemplary embodiment, the canister may have a volume ranging from about 5 ml to about 25 ml, such as, for example a canister having a 19 ml volume. After shaking the device, the mouthpiece is inserted into a patient's mouth between the lips and teeth. The patient typically exhales deeply to empty the lungs and then takes a slow deep breath while actuating the cartridge.

Inside an exemplary cartridge is a metering valve including a metering chamber capable of holding a defined volume of the formulation (e.g., 63 µl or any other suitable volume available in commercially available metering valves), which is released into an expansion chamber at the distal end of the valve stem when actuated. The actuator retains the canister and may also include a port with an actuator nozzle for receiving the valve stem of the metering valve. When actuated, the specified volume of formulation travels to the expansion chamber, out the actuator nozzle and into a high-velocity spray that is drawn into the lungs of a patient.

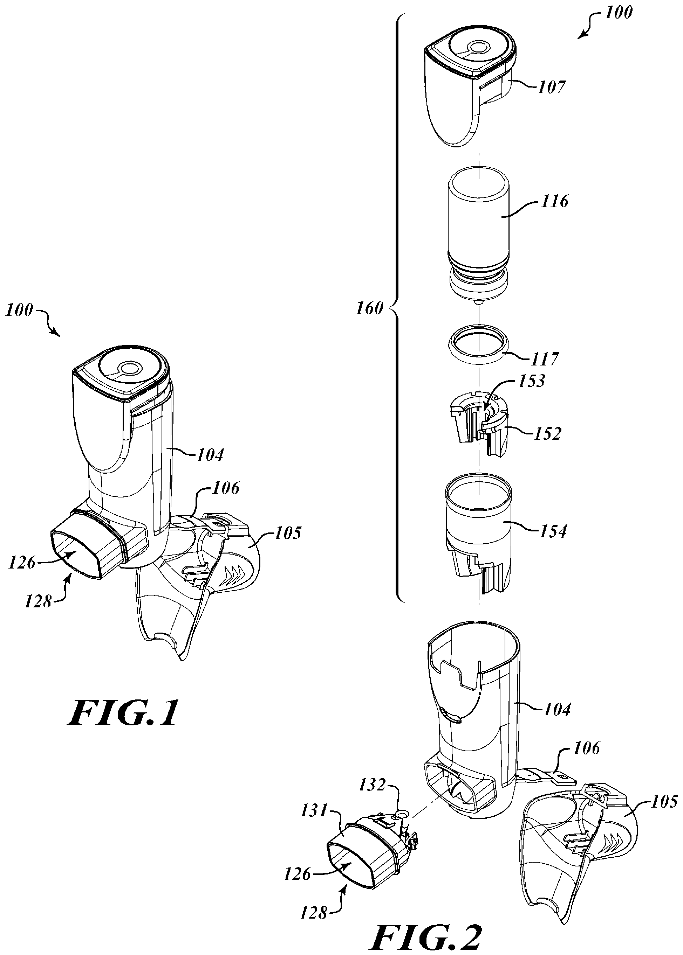

Examples of suitable MDIs are shown and described in International Application Publication No. WO 2019/074799. A suitable MDI may include, for example, the aerosol delivery unit 100 for selectively delivering a dose of aerosolized matter shown in **FIGs 1-3B****,** which includes structures and associated functionality for exposing a discharge passageway of the inhaler to a desiccant material at least during storage of the inhaler.

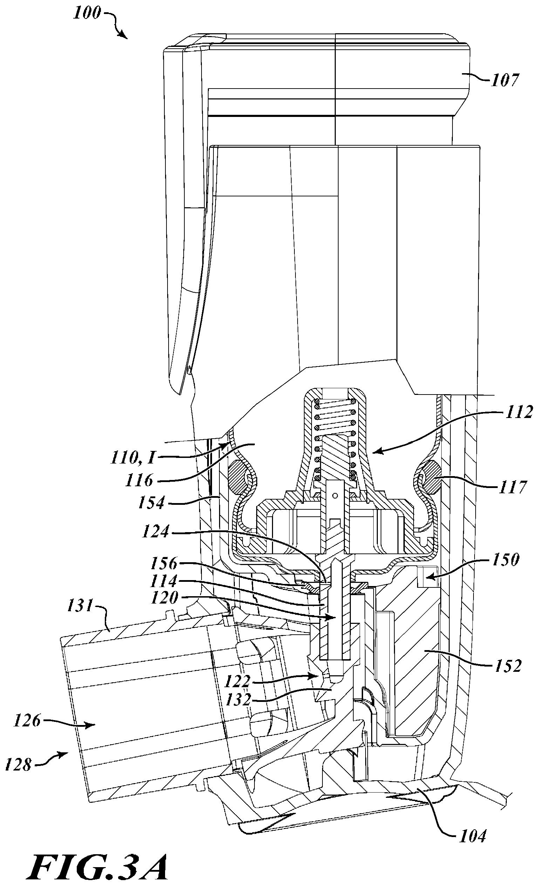

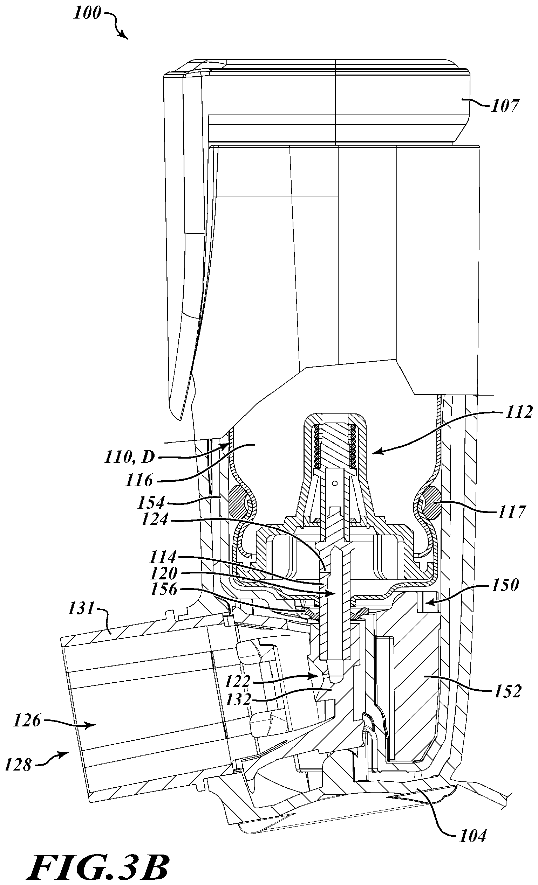

With reference to **FIGs 1** through **3B****,** the aerosol delivery unit 100 includes a base housing 104 and a canister 110 received in the base housing 104, the canister 110 being displaceable from an initial position I, as shown in **FIG. 3A****,** to a discharge position D, as shown in **FIG. 3B****,** for selectively discharging a dose of aerosolized matter for inhalation by a user. The canister 110 comprises a canister body 116, which contains the matter to be discharged, and an outlet valve 112, which includes a movable valve stem 114 that extends from the canister body 116. The valve stem 114 defines a portion of a discharge passageway 120 extending from the canister body 116 to a discharge orifice 122 provided within the aerosol delivery unit 100, which in turn leads to an inhalation passageway 126 through which the aerosolized matter passes before being discharged through a mouthpiece aperture 128 for inhalation by the user during an inhalation event. The discharge passageway 120 and the inhalation passageway 126 may be collectively referred to as a drug delivery tract. As will be appreciated by those of ordinary skill in the relevant art, when the valve stem 114 is displaced relative to the canister body 116, as shown in **FIG. 3B****,** a metered dose of the matter contained with the canister body 116 will be discharged through the discharge orifice 122 for inhalation by a user via the inhalation passageway 126.

With reference to **FIG. 1****,** the aerosol delivery unit 100 may further include a dose counter assembly 107 secured to an upper under of the canister 110 to provide dose counting functionality and to provide a user interface for depressing the canister 110. The aerosol delivery unit 100 may also include a cap 105 to cover the mouthpiece aperture 128 of the aerosol delivery unit 100 when storing the unit 100. The cap 105 may be completely separable from the base housing 104, or may be coupled to the base housing 104 by a tether 106, which enables the cover 105 to be removed from the mouthpiece aperture 128 while still remaining coupled to the base housing 104.

With reference to **FIGs 3A** and **3B****,** the aerosol delivery unit 100 further includes a desiccant chamber 150 containing a desiccant material 152 that is in fluid communication with the discharge passageway 120 at least when the aerosol delivery unit 100 is in a storage configuration and not actively discharging aerosolized matter. For example, in accordance with the example embodiment shown in **FIGs 3A** and **3B****,** the desiccant chamber 150 is provided at an end of the canister 110 between a lower end of the canister body 116 and a separate desiccant housing 154 and stem seal 156 that are coupled to the end of the canister 110. The desiccant material 152 may be provided in a semi-annular form (as shown in **FIG. 2**) and may include a central passage 153 through which the valve stem 114 of the canister 110 extends. The stem seal 156 may be an annular seal formed integrally with the desiccant housing 154, such as, for example, via a multi-shot injection molding process, or may otherwise be provided as a separate seal component coupled to the desiccant housing 154. In some instances, the stem seal 156 may be provided as a bellows type seal that is secured between the valve stem 114 and the desiccant housing 154 to provide a desiccant chamber 150 having a volume that varies as the stem seal 156 is deformed as the canister 110 is displaced during an inhalation event. In other instances, such as the example embodiment shown in **FIGs 3A** and **3B****,** the desiccant chamber 150 may have a fixed volume.

As can be appreciated from **FIG. 3A****,** the desiccant material 152 within the desiccant chamber 150 is in fluid communication with the discharge passageway 120 through an aperture 124 in the side of the valve stem 114 that is otherwise used to pass the matter contained in the canister body 116 toward the discharge orifice 122 when the valve stem 114 is displaced during an inhalation event. In this manner, the discharge passageway 120 remains exposed to the desiccant material 152 when the canister 110 is in the initial position I, such as when storing the unit 100. In some instances, the desiccant material may be sufficient to keep the discharge passageway dry (e.g., < 25%RH) between uses for substantially the entire product life of the canister of material to be discharged.

Advantageously, the desiccant housing 154 may be coupled to the end or collar of the canister 110 to form a cartridge 160 (**FIG. 2**) that is readily removable from the base housing 104. In this manner, the desiccant housing 154 and canister 110 may be easily removed from the base housing 104 to replace the canister 110 when depleted and/or to replace the desiccant material 152 as desired. The desiccant housing 154 may be coupled to the end or collar of the canister 110 via a resilient band, clips, detents or other fastening devices or techniques, including friction fit or interference fit arrangements. Although the desiccant chamber 150 is shown in the example embodiment of **FIGs 3A** and **3B** as being coupled to a lower end or collar of the canister 110, it is appreciated that in other embodiments a desiccant chamber may be provided in a separate desiccant housing that is coupled to the base housing 104 separate from the canister 110, the desiccant chamber may be formed integrally in the base housing itself, or the desiccant chamber may be provided in a separate component that is attached to the base housing 104. In addition, the desiccant material may be provided in a variety of different forms, such as gel form, powder form, granular form or molded form, and may consist of or comprise different materials, such as silica, activated charcoal, calcium sulfate or calcium chloride.

According to the example embodiment of **FIGs 1** through **3B****,** the desiccant housing 154 may be coupled to the end or collar of the canister 110 to form a cartridge 160 that is installable in the base housing 104 to engage a stem seat/nozzle block 132 provided therein. Further details of the components of the cartridge 160 and the stem seat/nozzle block 132 can be seen in the exploded view of **FIG. 2****.** As shown in **FIG. 2****,** the desiccant housing 154 may form a cup-like structure with a generally cylindrical sidewall that is sized and shaped to receive a lower end of the canister 110. The desiccant material 152 may be provided in a molded form. The desiccant material 152 may be configured to be positioned in a lower end of the desiccant housing 154. The desiccant housing 154 may include one or more locating or coupling features to assist in joining or otherwise positioning the desiccant material 152 within the desiccant housing 154. The desiccant material 152 may be shaped so as to not obstruct a valve stem aperture of the stem seal 156 provided in the desiccant housing 154 for receiving the valve stem 114 of the canister 110. For example, the desiccant material 152 may have a semi-annular shape with a central passage 153 or other clearance for the valve stem 114. In some instances, such as in the example embodiment shown in **FIGs 1** through **3B****,** the desiccant material 152 may be shaped to partially encircle the valve stem 114 and may extend beyond a terminal end of the valve stem 114. The desiccant housing 154 and the desiccant material 152 may also be correspondingly shaped, and may each extend beyond a terminal end of the valve stem 114. In this manner, the desiccant material 152 may substantially fill the desiccant chamber 150 and provide a relatively large volume of desiccant material suitable to continuously remove moisture at least from the passage of the valve stem 114 throughout the usable life of the material (e.g., drug formulation) contained in the canister 110.

With reference to **FIGs 3A** and **3B****,** a canister seal 117 may be positioned around the canister body 116, such as around a lower neck portion thereof, to provide a resilient member between the canister body 116 and the desiccant housing 154 which may be compressed when the canister 110 and the desiccant housing 154 are coupled together. The canister seal 117 may provide a seal location to assist in isolating the desiccant chamber 150 when the aerosol delivery unit 100 is fully assembled and in preventing the ingress of moisture into said desiccant chamber 150 other than through the discharge passageway 120. In a similar manner, the stem seal 156 may provide a seal location to assist in isolating the desiccant chamber 150 when the aerosol delivery unit 100 is fully assembled and in preventing the ingress of moisture into said desiccant chamber 150. In this manner, the desiccant chamber 150 is effectively isolated from the external environment apart from the discharge passageway 120, which may be exposed to the external environment through the inhalation passageway 126 when the mouthpiece cap 105 is removed from the base housing 104.

As can be appreciated from a review of **FIGs 3A** and **3B****,** when the valve stem 114 is in an expanded position, the portion of the discharge passageway 120 defined by the valve stem 114 is in fluid communication with the desiccant chamber 152 via the aperture 124 in the side of the valve stem 114. Conversely, when the valve stem 114 of the canister 110 is fully depressed, the desiccant chamber 152 is temporarily isolated from the discharge passageway 120 defined by the valve stem 114.

Again, with the canister 100 loaded in the desiccant housing 154, the valve stem 114 protrudes from a lower end thereof to be subsequently received in the stem seat/nozzle block 132 provided in the base housing 104. According to the example embodiment of **FIG. 2****,** the stem seat/nozzle block 132 may be provided in a mouthpiece unit 131 that is coupleable to the base housing 104 and includes the inhalation passageway 126 and the mouthpiece aperture 128 for delivering aerosolized matter to the user. As illustrated, when the cartridge 160 is installed, the desiccant material 152 may extend from a location above the discharge orifice 122 of the stem seat/nozzle block 132 to a location below the discharge orifice 122, and may substantially fill the desiccant chamber 150 within the desiccant housing 154 to provide a relatively large volume of desiccant material suitable to continuously remove moisture at least from the passage of the valve stem 114 throughout the usable life of the material (e.g., drug formulation) contained in the canister 110. In this manner, embodiments may be particularly well suited to eliminate, reduce or minimize the presence of moisture in the discharge passageway 120 and to eliminate, reduce or minimize any fouling associated therewith even when not completely isolating the discharge passageway 120 from the external environment after discharging the material during the inhalation event.

According to some embodiments, an MDI is provided, such as the aerosol delivery unit 100 shown in **FIGs 1-3B****,** wherein one or more internal components of the outlet valve 112 is at least partially composed of bromobutyl material (e.g., bromobutyl rubber).

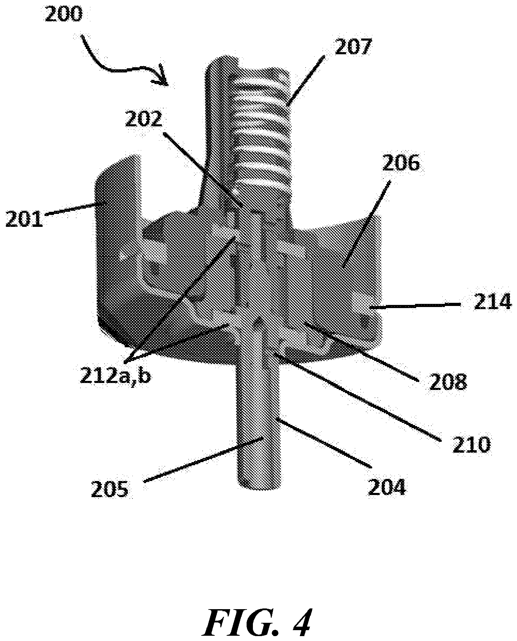

For example, **FIG. 4** illustrates an outlet valve 200 of a canister 201 of an MDI containing a formulation to be discharged, wherein the outlet valve 200 is provided with one or more internal components that comprise or consist of a bromobutyl material (e.g., bromobutyl rubber). For instance, the outlet valve 200 includes an inner core 202 and a valve stem 204 that are movably displaceable relative to a valve body 206 and to a metering chamber 208 to dispense a metered amount of a formulation through a discharge passageway 205 of the outlet valve 200 during operation of the MDI device. The inner core 202 and the valve stem 204 are biased toward an extended position by a spring element 207 and are selectively depressible to dispense a metered dose of formulation.

To assist in establishing a consistent metered dose of formulation to be discharged, the outlet valve 200 further comprises a plurality of gaskets to seal and isolate an internal cavity of the metering chamber 208 relative to the valve body 206 and the canister 201 and to seal and isolate an internal formulation cavity of the canister 201 from an external environment. More particularly, upper and lower seat gaskets 212a,b are provided that slidably engage the inner core 202 and the valve stem 204 to seal and isolate the internal cavity of the metering chamber 208 relative to the valve body 206 and the canister 201. As shown in **FIG. 4****,** the upper seat gasket 212a is provided between the metering chamber 208 and the valve body 206 and encircles and seals against a portion of the displaceable inner core 202. The lower seat gasket 212b is provided between the metering chamber 208 and the canister 201 and encircles and seals against a portion of the displaceable valve stem 204, which projects from the canister 201. Advantageously, one or more of the seat gaskets 212a,b may comprise or consist of a bromobutyl material (e.g., bromobutyl rubber). In addition, as further shown in **FIG. 4****,** a neck gasket 214 is provided between the valve body 206 and the canister 201 to further assist in sealing and isolating the internal formulation cavity from the external environment. Advantageously, the neck gasket 214 may comprise or consist of a bromobutyl material (e.g., bromobutyl rubber).

It has been shown that by forming one or more of the internal gaskets of the outlet valve 200, namely, one or more of the seat gaskets 212a,b and/or the neck gasket 214, to comprise or consist of a bromobutyl material (e.g., bromobutyl rubber), the outlet valve 200 is particularly effective in discharging and maintaining a consistent metered dose of the formulation throughout operation over time and in avoiding fouling or clogging of a discharge orifice of the MDI device. In addition, the outlet valve 200 is particularly effective in avoiding formulation weight loss over time compared to other suitable gasket materials. Accordingly, such a configured MDI is particularly well suited to deliver formulation to a user.

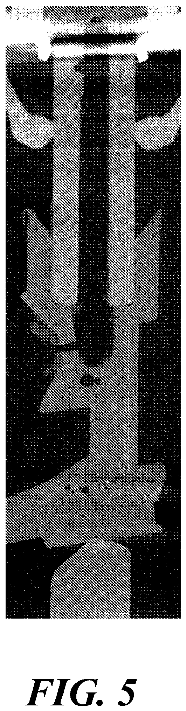

As an example, **FIG. 5** shows a CT scan of a discharge passageway of an MDI having a formulation canister with an outlet valve that includes internal seat and neck gaskets composed of a bromobutyl material (e.g., bromobutyl rubber), wherein the MDI was used to repeatedly discharge a formulation under controlled environmental conditions (25°C / 60% RH). Notably, **FIG. 5** shows a discharge orifice of the MDI substantially free of deposited or accumulated matter despite repeated use of the MDI to dispense formulations described herein.

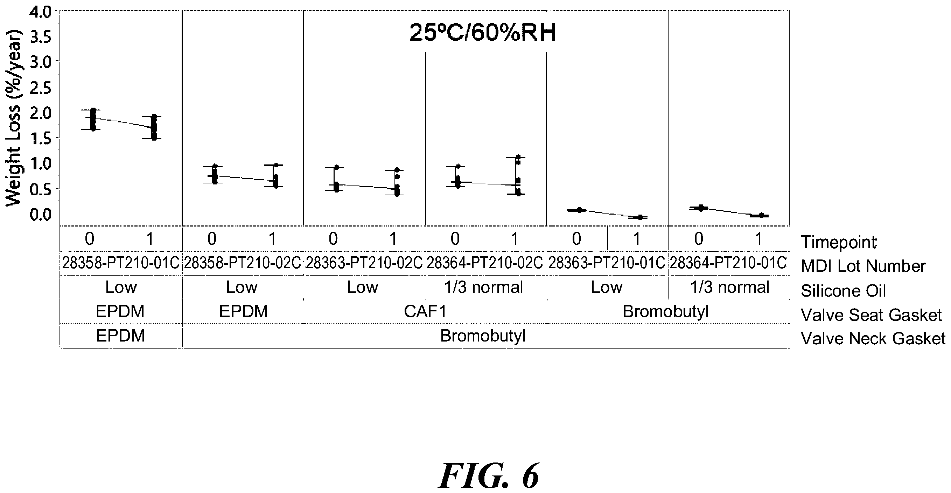

**FIG. 6** provides a comparison of formulation weight loss over time for different valve seat gasket and valve neck gasket materials. As can be appreciated from **FIG. 6****,** configurations in which the valve neck gasket composed a bromobutyl material (e.g., bromobutyl rubber) consistently showed significant reductions in weight loss over time as compared to a control configuration (leftmost column in the chart). Further, when the valve seat gaskets also composed a bromobutyl material, e.g., bromobutyl rubber, (rightmost columns in the chart), the weight loss over time approached 0%. As such, providing internal gaskets composed of a bromobutyl material (e.g., bromobutyl rubber) showed unexpected performance.

The following abbreviations are used throughout the present disclosure including the Drawing and Examples:
- AB: Albuterol
- AS: Albuterol Sulfate
- BD: Budesonide
- FF: Formoterol Fumarate
- GP: Glycopyrrolate
- RF: Roflumilast
- BDA: budesonide/albuterol (combo)
- BGF: budesonide/glycopyrrolate/formoterol (combo)
- GFF: glycopyrolate/formoterol fumarate (combo)
- BFF: budesonide/formoterol fumarate (combo)
- BGFR: budesonide/glycopyrrolate/formoterol fumarate/roflumilast (combo)
- BDA-1234ze: budesonide/albuterol (combo) in HFO-1234ze(E) formulation
- BDA-134a: budesonide/albuterol (combo) in HFA-134a formulation
- BFF-1234ze: budesonide/formoterol fumarate (combo) in HFO-1234ze(E) formulation
- BFF-134a: budesonide/formoterol fumarate (combo) in HFA-134a formulation
- CFC-11: Trichlorofluoromethane
- CFC-113: 1,1,2-Trichloro-1,2,2-trifluoroethane
- CFC-114: 1,2-Dichlorotetrafluoroethane
- HCFC-124: 1-Chloro-1,2,2,2-tetrafluoroethane
- HFA-227ea: 1,1,1,2,3,3,3-Heptafluoropropane
- HFC-125: Pentafluoroethane, also known as 1,1,1,2,2-Pentafluoroethane
- HFC-152a: 1,1-Difluoroethane
- HFC-245cb: 1,1,1,2,2-Pentafluoropropane
- HFO-1225ye(Z): cis-1,2,3,3,3-Pentafluoropropene
- HFO-1225ye(E): *trans-1,2,3,3,3-* Pentafluoropropene
- HFO-1234yf: 2,3,3,3-Tetrafluoropropene
- HFO-1234ze(Z): cis-1,3,3,3-Tetrafluoroprop-1-ene
- PP: Porous Particles of Phospholipids

Any active agents and reagents used in the following examples are either commercially available or, with the benefit of the teachings provided herein, can be prepared according to standard literature procedures by those skilled in the art.

### EXAMPLES

### Example 1

Suspending particles were manufactured by spray drying an emulsion of PFOB (perfluorooctyl bromide) and water stabilized by DSPC (1,2-Distearoyl-sn-Glycero-3-Phosphocholine). Detailed preparation procedures can be found in WO 2010/138862, WO 2010/138868, and WO 2010/138884. The particle size distribution of the suspending particles was determined by laser diffraction. 50% by volume of the suspending particles were smaller than 2.9 µm, the Geometric Standard Deviation of the distribution was 1.8.

Active agent particles formed of glycopyrrolate (Pyrrolidinium, 3-((cyclopentylhydroxyphenylacetyl)oxy)-1,1-dimethyl-, bromide) were formed by micronizing glycopyrrolate using a jet mill. The particle size distribution of the micronized glycopyrrolate (GP) was determined by laser diffraction. 50% by volume of the micronized particles exhibited an optical diameter smaller than 2.1 µm, 90% by volume were smaller than 5 µm.

Formoterol fumarate, (±)-2-hydroxy-5-[(1RS)-1-hydroxy-2-[[(1RS)-2-(4-methoxyphenyl)-1-methylethyl]-amino]ethyl]formanilide fumarate, also known as (±)-2'-hydroxy-5-[(RS)-1-hydroxy-2-[[RS)-p-methoxy-α-methylphenethyl]-amine]ethyl]formanilide fumarate, dihydrate was received micronized by the manufacturer (Inke) and used as active agent particles. The particle size distribution of the micronized formoterol fumarate (FF) was determined by laser diffraction. 50% by volume of the micronized particles exhibited an optical diameter smaller than 1.6 µm, and 90% by volume exhibited an optical diameter smaller than 3.9 µm.

Active agent particles formed of budesonide, 16,17-(butylidenebis(oxy))-11,21-dihydroxy-(11-β,16-α)-pregna-1,4-diene-3,20-dione, were formed by micronizing budesonide using a jet mill. The particle size distribution of the budesonide (BD) was determined by laser diffraction. 50% by volume of the micronized particles exhibited an optical diameter smaller than 1.9 µm, 90% by volume exhibited an optical diameter smaller than 4.3 µm.

Active agent particles formed of albuterol were formed by micronizing albuterol sulfate using a jet mill. The particle size distribution of the albuterol sulfate (AS) was determined by laser diffraction. 50% by volume of the micronized particles exhibited an optical diameter smaller than 1.5 µm, 90% by volume exhibited an optical diameter smaller than 3.3 µm.

Active agent particles formed of roflumilast were formed by micronizing roflumilast using a jet mill. The particle size distribution of the roflumilast (RF) was determined by laser diffraction. 50% by volume of the micronized particles exhibited an optical diameter smaller than 1.0 µm, 90% by volume exhibited an optical diameter smaller than 2.4 µm.

Metered dose inhalers were prepared by first dispensing the appropriate quantities of suspending particles and active agent particles an addition vessel (AV) and adding an appropriate quantity of HFO-1234ze(E) (1,3,3,3-Tetrafluoropropene) propellant. The mixture is agitated to facilitate powder wetting and then transferred to a pressure vessel where the suspension is mixed. Valves comprised of 50 µL metering chambers (BK357, Bespak, King's Lynn, UK) are crimped onto fluorinated ethylene polymer (FEP) coated aluminum cans (Presspart, Blackbum, UK) and the suspension is then pressure filled through the valve. The canisters were fitted with polypropylene actuators with a 0.32 mm orifice (# 10024269, Bespak, King's Lynn, UK).

### Example 2

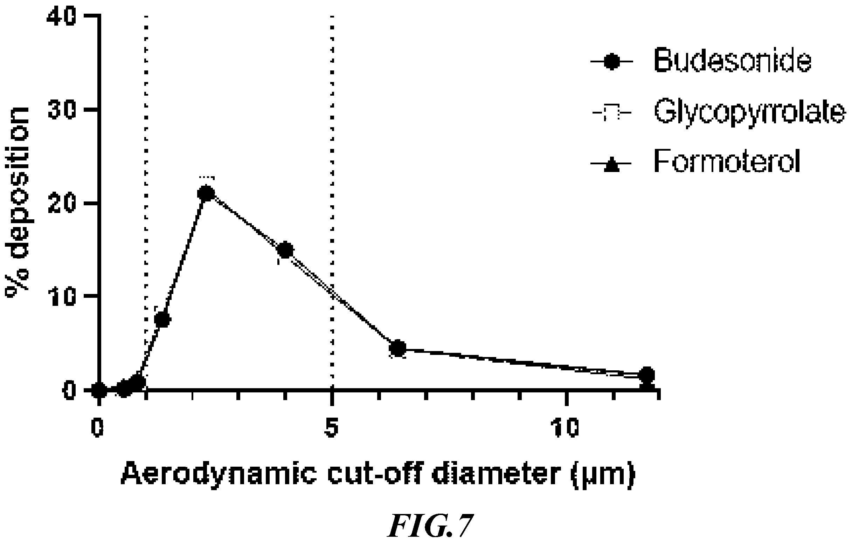

Metered dose inhalers containing a triple co-suspension composition comprising glycopyrrolate, budesonide, and formoterol active agent particles were prepared, with each type of active agent particle being provided as a micronized, crystalline API material. The active agent particles were suspended in HFO-1234ze(E) propellant, either with or without phospholipid particles. In the formulation containing phospholipid particles, the three types of active agent particles showed uniform deposition distributions as shown in **FIG. 7****.** The three types of active agent particles showed individual deposition distributions in the formulation without phospholipid particles.

### Example 3

Metered dose inhalers containing a triple co-suspension composition comprising glycopyrrolate, budesonide, and formoterol active agent particles were prepared, with each type of active agent particle being provided as a micronized, crystalline API material. The active agent particles were suspended in HFA-134a propellant or in HFO-1234ze(E) propellant, without phospholipid particles. The deposition distribution of budesonide in each formulation was tested at 0% and at 50% relative humidity. The HFA-134 propellant formulation showed a greater impact of relative humidity upon the deposition distribution than did the HFO-1234ze(E) propellant.

### Example 4

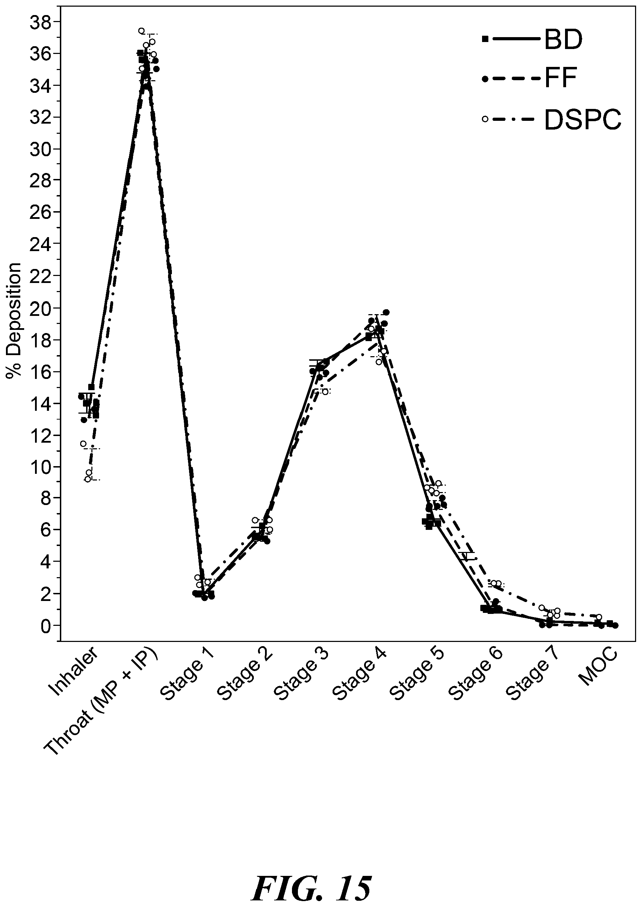

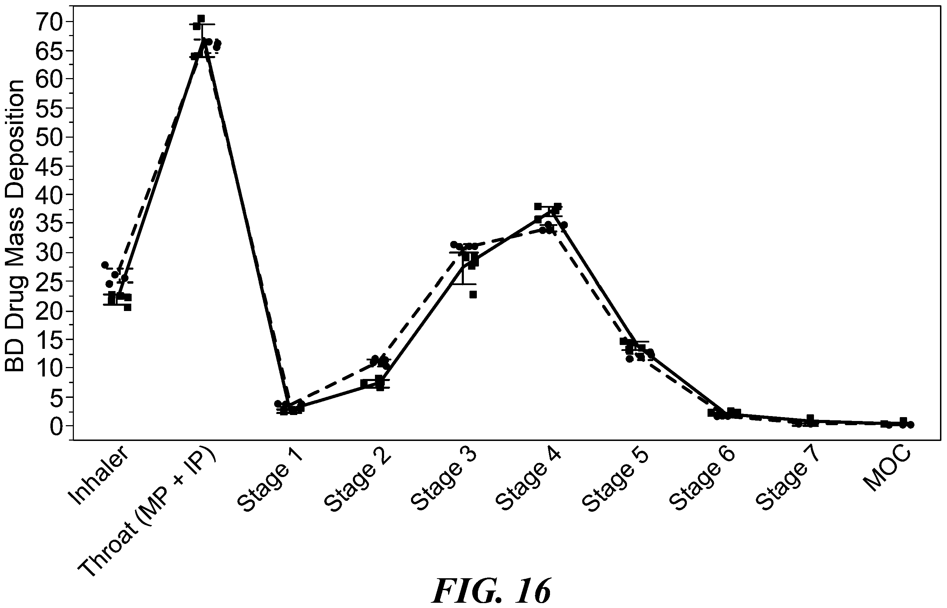

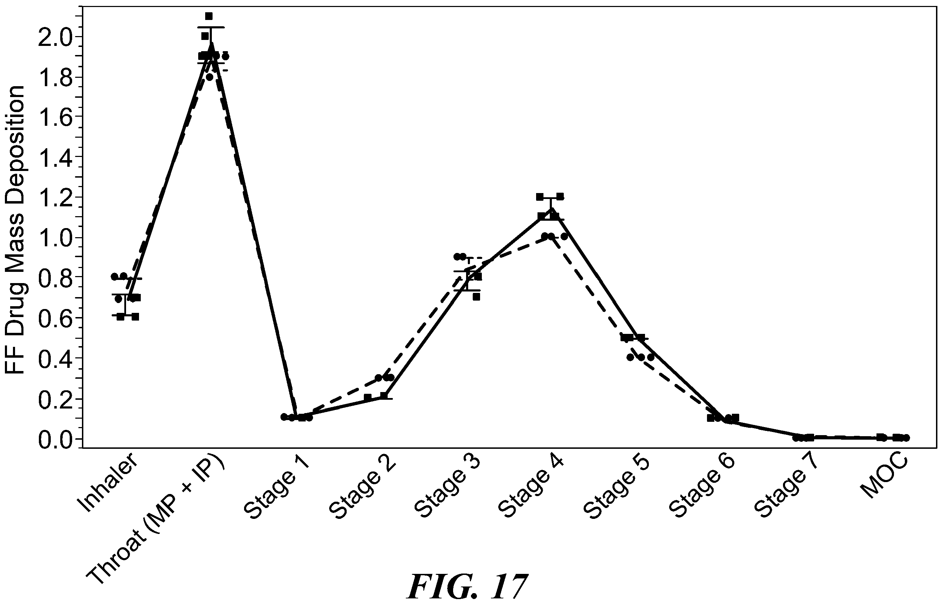

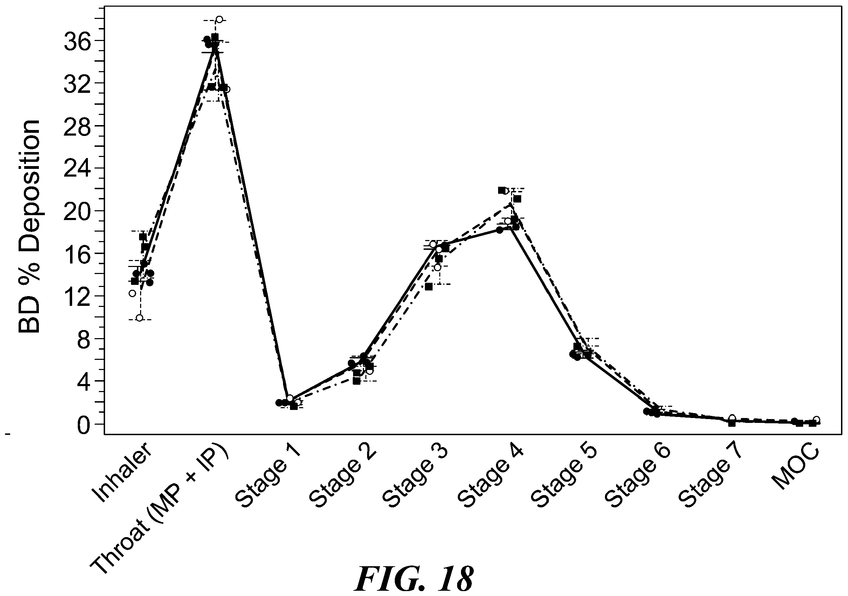

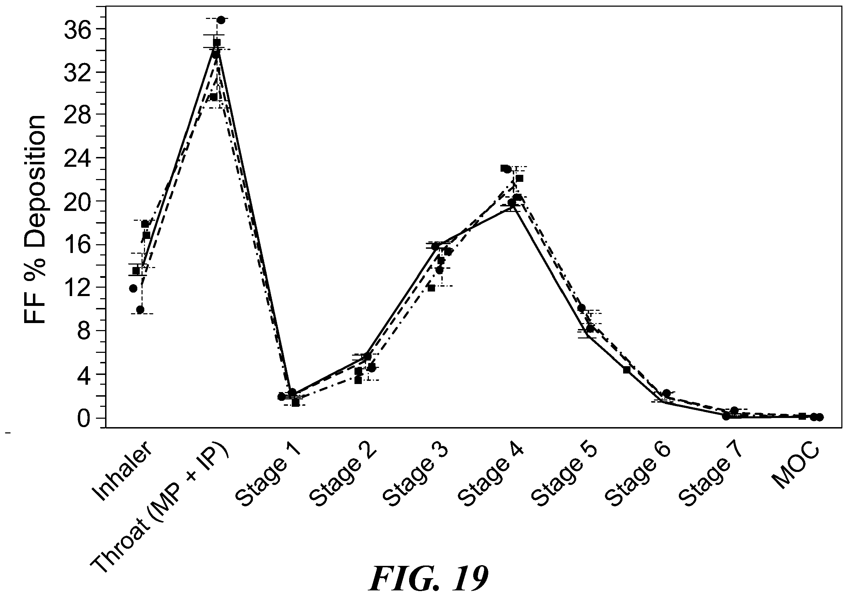

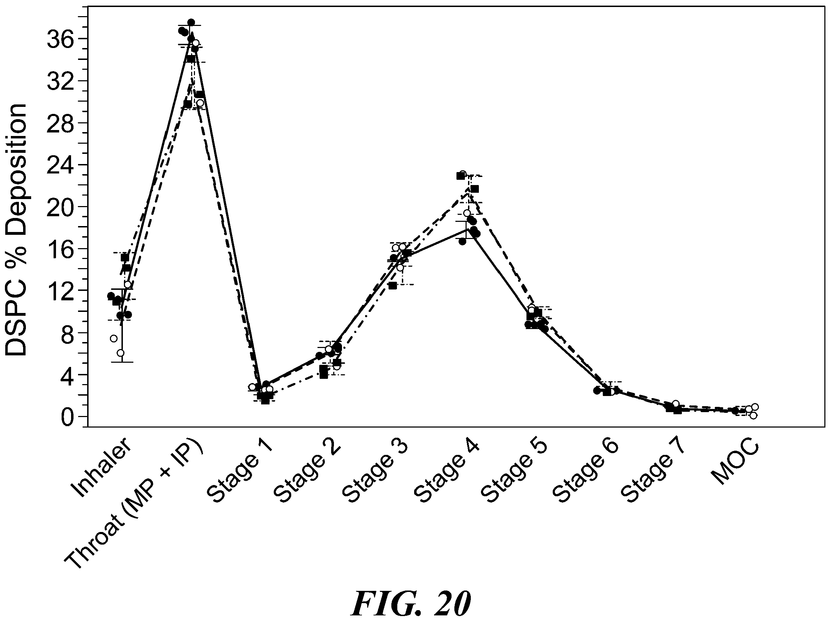

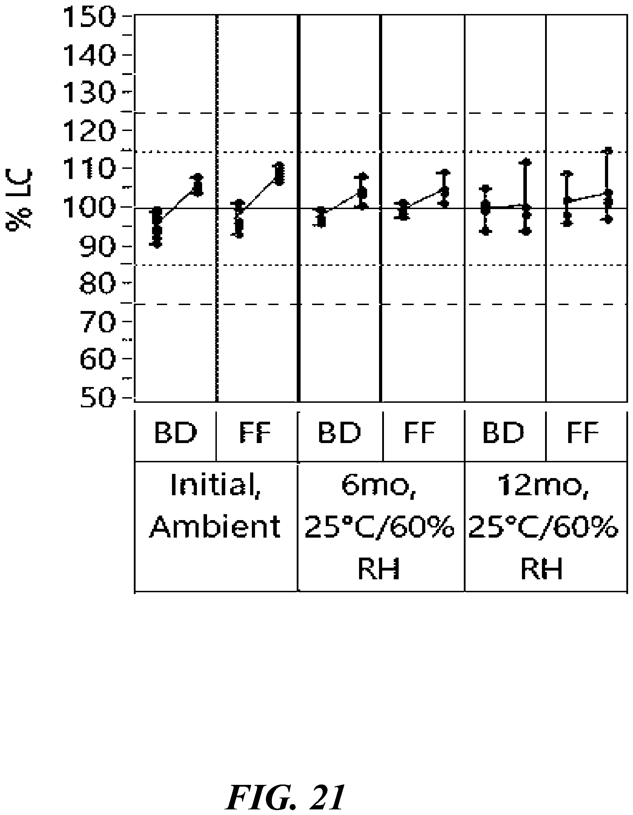

Metered dose inhalers containing a dual co-suspension composition comprising budesonide and formoterol active agent particles were prepared, with each type of active agent particle being provided as a micronized, crystalline API material. The active agent particles were suspended in HFO-1234ze(E) propellant, either with or without phospholipid particles. As shown in **FIG. 15****,** the two types of active agent particles and the suspending particles showed a uniform deposition distribution. Table 6 provides the FPF (Fine Particle Fraction), FPD (Fine Particle Dose), MMAD (Mass Median Aerodynamic Diameter), and Throat Deposition as characterized by NGI (Next Generation Impactor). As shown in **FIGs 16** and **17****,** budesonide and formoterol fumarate, respectively, produced a similar aPSD (aerodynamic particle size distributions) by NGI in HFO-1234ze(E) as in HFA-134a. Table 7 provides the FPF (Fine Particle Fraction), FPD (Fine Particle Dose), MMAD (Mass Median Aerodynamic Diameter), and Throat Deposition as characterized by NGI (Next Generation Impactor). As shown in **FIGs 18****,** **19****,** and **20****,** the aPSD of budesonide, formoterol fumarate, and the suspending particle depicted as DSPC, respectively, was stable for twelve months when stored valve down and protected at 25°C/60% RH. Table 8 provides the FPF (Fine Particle Fraction), FPD (Fine Particle Dose), MMAD (Mass Median Aerodynamic Diameter), and Throat Deposition as characterized by NGI (Next Generation Impactor). As shown in **FIG. 21****,** budesonide and formoterol fumarate demonstrated consistent delivered dose as represented as %LC (percent of label claim) and were stable for twelve months when stored valve down and protected at 25°C/60% RH.

**Table 6. BD, FF, and DSPC Fine Particle Fraction (FPF), Fine Particle Dose (FPD), Mass Median Aerodynamic Diameter (MMAD), and Throat Deposition of BFF-1234ze**

| **Active** | **FPF, <6.4 µm (%)** | **FPD, <6.4 µm (µg/act)** | **MMAD (µm)** | **Throat Deposition (%)** |
|---|---|---|---|---|
| BD | 50 | 79.3 | 3.87 | 35.4 |
| FF | 51 | 2.4 | 3.73 | 34.8 |
| DSPC | 50 | 131.7 | 3.64 | 36.3 |

**Table 7. BD and FF Fine Particle Fraction (FPF), Fine Particle Dose (FPD), Mass Median Aerodynamic Diameter (MMAD), and Throat Deposition of BFF-1234ze and BFF-134a formulations as characterized by NGI**

| **Active** | **Formulation** | **FPF, <6.4 µm (%)** | **FPD, <6.4 µm (µg/act)** | **MMAD (µm)** | **Throat Deposition (%)** |
|---|---|---|---|---|---|
| BD | BFF-134a | 52 | 81.8 | 3.54 | 66.7 |
| | BFF-1234ze | 50 | 79.3 | 3.87 | 65.6 |
| FF | BFF-134a | 53 | 2.5 | 3.38 | 2.0 |
| | BFF-1234ze | 51 | 2.4 | 3.73 | 1.9 |

**Table 8. BD, FF, and DSPC Fine Particle Fraction (FPF), Fine Particle Dose (FPD), Mass Median Aerodynamic Diameter (MMAD), and Throat Deposition Stability Data for BFF-1234ze**

| **Active** | **Storage** | **FPF, <6.4 µm (%)** | **FPD, <6.4 µm (µg/act)** | **MMAD (µm)** | **Throat Deposition (%)** |
|---|---|---|---|---|---|
| BD | Initial | 50 | 79.3 | 3.87 | 65.6 |
| | 6 month 25°C/60%RH | 52 | 70.5 | 3.72 | 55.1 |
| | 12 month 25°C /60%RH | 53 | 86.8 | 3.58 | 64.4 |
| FF | Initial | 51 | 2.4 | 3.73 | 1.9 |
| | 6 month 25°C/60%RH | 54 | 2.1 | 3.53 | 1.5 |
| | 12 month 25°C /60%RH | 56 | 2.6 | 3.36 | 1.8 |
| DSPC | Initial | 50 | 131.7 | 3.64 | 107.3 |
| | 6 month 25°C/60%RH | 55 | 122.1 | 3.47 | 79.0 |
| | 12 month 25°C /60%RH | 57 | 150.9 | 3.33 | 64.3 |

In the formulation without phospholipid particles, the two types of active agent particles showed individual deposition distributions, while in the formulation containing phospholipid particles, the two types of active agent particles showed uniform deposition distributions.

### Example 5

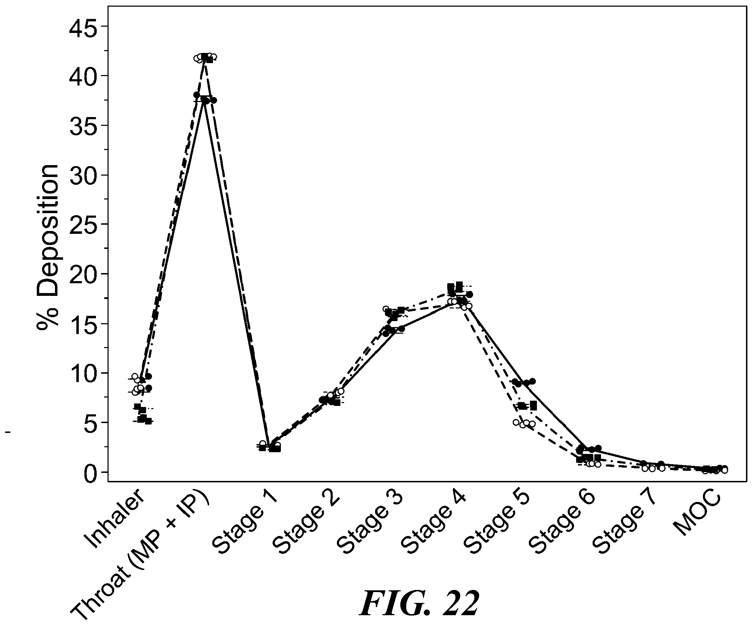

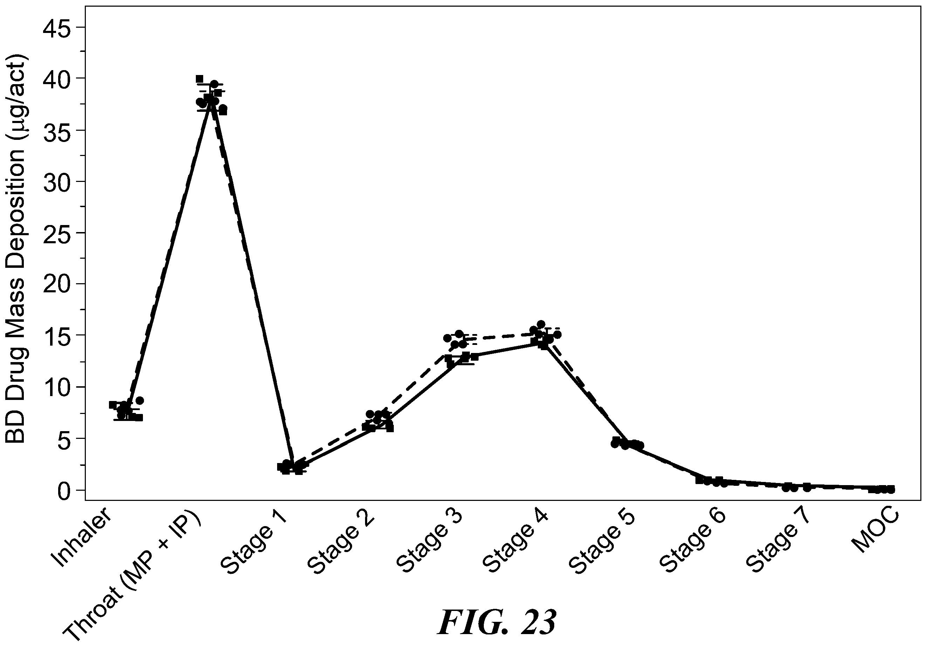

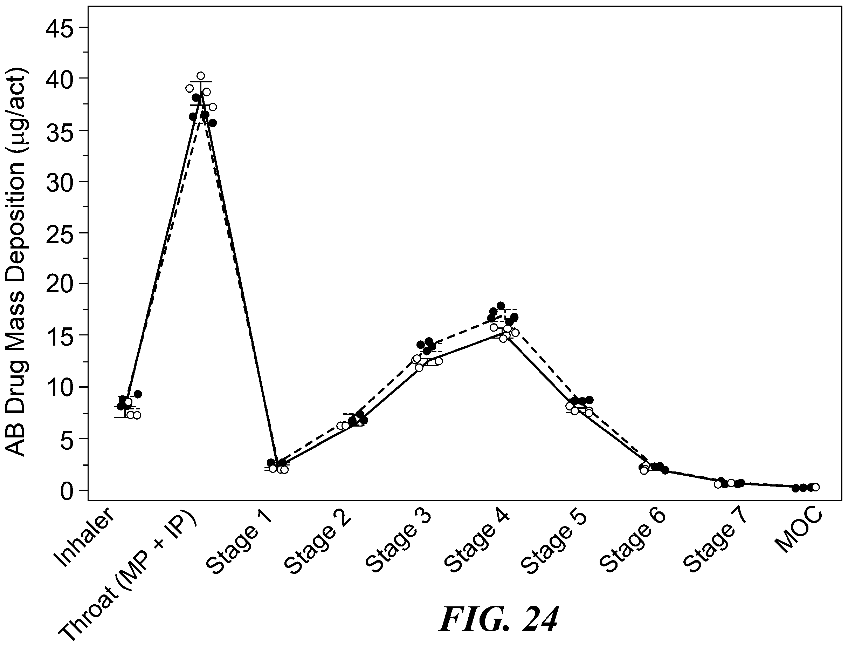

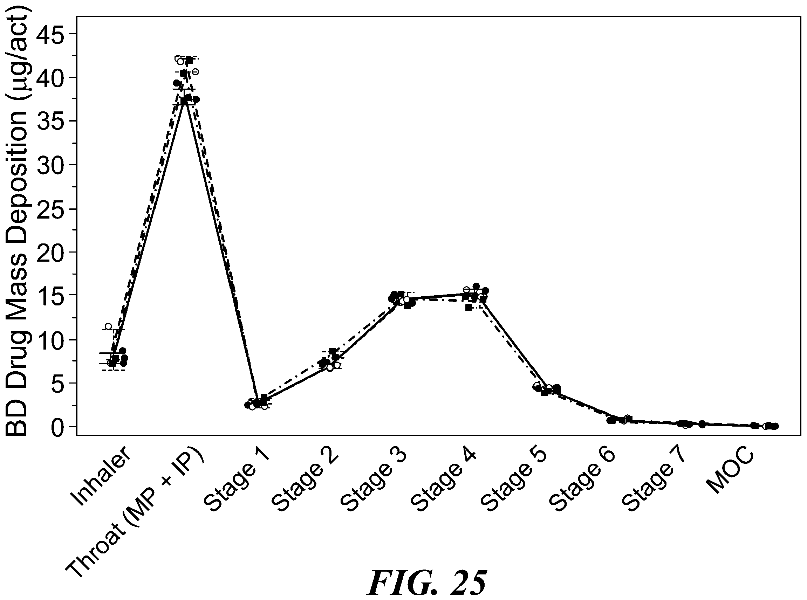

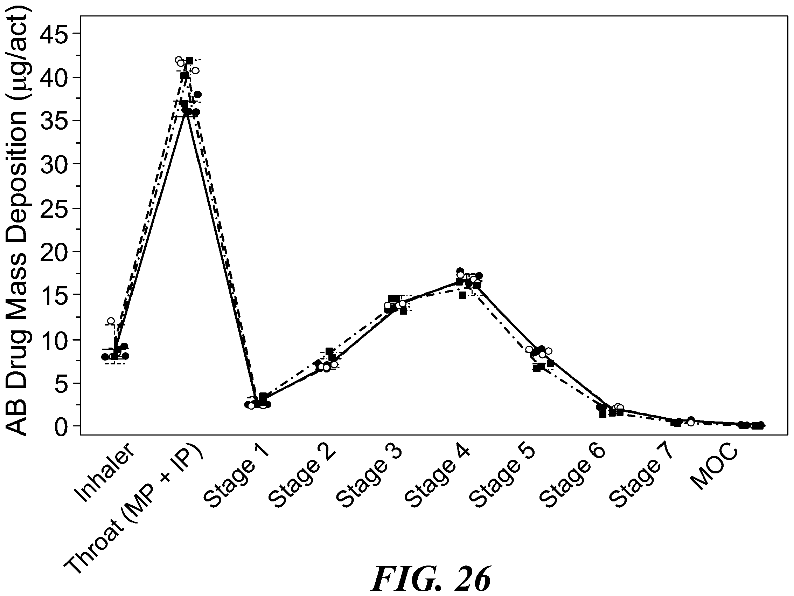

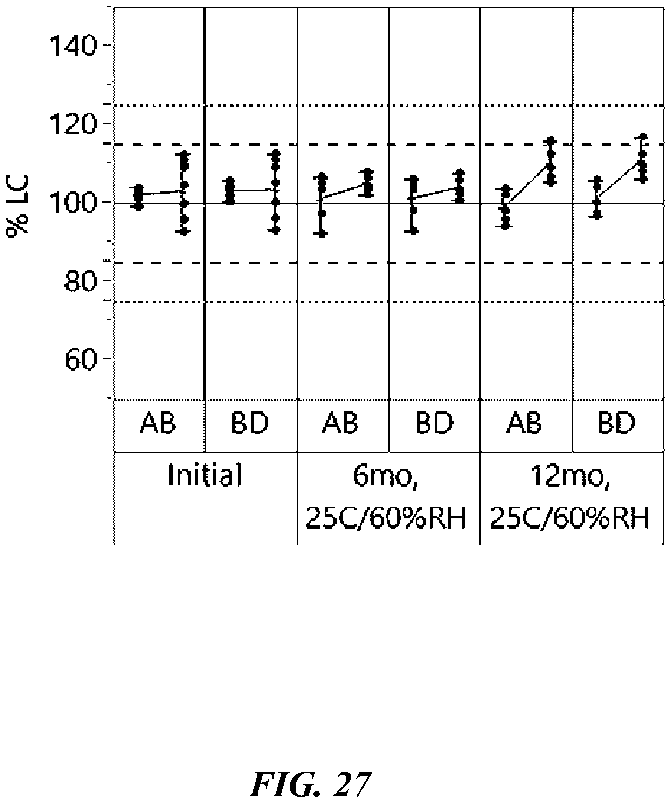

Metered dose inhalers containing a dual co-suspension composition comprising budesonide and albuterol active agent particles were prepared, with each type of active agent particle being provided as a micronized, crystalline API material. The active agent particles were suspended in HFO-1234ze(E) propellant, either with or without phospholipid particles. As shown in **FIG. 22****,** the two types of active agent particles and the suspending particles showed a uniform deposition distribution. Table 9 provides the FPF (Fine Particle Fraction), FPD (Fine Particle Dose), MMAD (Mass Median Aerodynamic Diameter), and Throat Deposition as characterized by NGI (Next Generation Impactor). As shown in **FIGs 23** and **24****,** budesonide and albuterol, respectively, produced a similar aPSD (aerodynamic particle size distributions) by NGI in HFO-1234ze(E) as in HFA-134a. Table 10 provides the FPF (Fine Particle Fraction), FPD (Fine Particle Dose), MMAD (Mass Median Aerodynamic Diameter), and Throat Deposition as characterized by NGI (Next Generation Impactor). As shown in **FIGs 25****,** and **26****,** the aPSD of budesonide and albuterol, respectively, was stable for twelve months when stored valve down and protected at 25°C/60% RH. Table 11 provides the FPF (Fine Particle Fraction), FPD (Fine Particle Dose), MMAD (Mass Median Aerodynamic Diameter), and Throat Deposition as characterized by NGI (Next Generation Impactor). As shown in **FIG. 27****,** budesonide and albuterol demonstrated consistent delivered dose as represented as %LC (percent of label claim) and were stable for twelve months when stored valve down and protected at 25°C/60% RH.

**Table 9. BD, AB, and DSPC Fine Particle Fraction (FPF), Fine Particle Dose (FPD), Mass Median Aerodynamic Diameter (MMAD), and Throat Deposition of BDA-1234ze**

| **Active** | **FPF, <6.4 µm (%)** | **FPD, <6.4 µm (µg/act)** | **MMAD (µm)** | **Throat Deposition (%)** |
|---|---|---|---|---|
| AB | 48 | 42.5 | 3.67 | 36.5 |
| BD | 43 | 35.3 | 4.18 | 37.8 |
| DSPC | 46 | 80.0 | 3.89 | 77.4 |

**Table 10. BD and AB Fine Particle Fraction (FPF), Fine Particle Dose (FPD), Mass Median Aerodynamic Diameter (MMAD), and Throat Deposition of BDA-1234ze and BDA-134a formulations as characterized by NGI**

| **Active** | **Formulation** | **FPF, <6.4 µm (%)** | **FPD, <6.4 µm (µg/act)** | **MMAD (µm)** | **Throat Deposition (%)** |
|---|---|---|---|---|---|
| BD | BDA-134a | 42 | 33.0 | 4.00 | 38.2 |
| | BDA-1234ze | 43 | 35.3 | 4.18 | 37.8 |
| AB | BDA-134a | 45 | 38.4 | 3.64 | 38.6 |
| | BDA-1234ze | 48 | 42.5 | 3.67 | 36.5 |

**Table 11. BD and AB Fine Particle Fraction (FPF), Fine Particle Dose (FPD), Mass Median Aerodynamic Diameter (MMAD), and Throat Deposition Stability Data for BDA-1234ze**

| **Active** | **Storage** | **FPF, <6.4 µm (%)** | **FPD, <6.4 µm (µg/act)** | **MMAD (µm)** | **Throat Deposition (%)** |
|---|---|---|---|---|---|
| BD | Initial | 43 | 35.3 | 4.18 | 37.8 |
| | 6 month 25°C/60%RH | 41 | 35.1 | 4.17 | 41.5 |
| | 12 month 25°C /60%RH | 40 | 33.8 | 4.39 | 39.9 |
| AB | Initial | 48 | 42.5 | 3.67 | 36.5 |
| | 6 month 25°C/60%RH | 45 | 42.3 | 3.70 | 41.5 |
| | 12 month 25°C /60%RH | 43 | 39.1 | 4.01 | 39.8 |

In the formulation without phospholipid particles, the two types of active agent particles showed individual deposition distributions, while in the formulation containing phospholipid particles, the two types of active agent particles showed uniform deposition distributions.

### Example 6

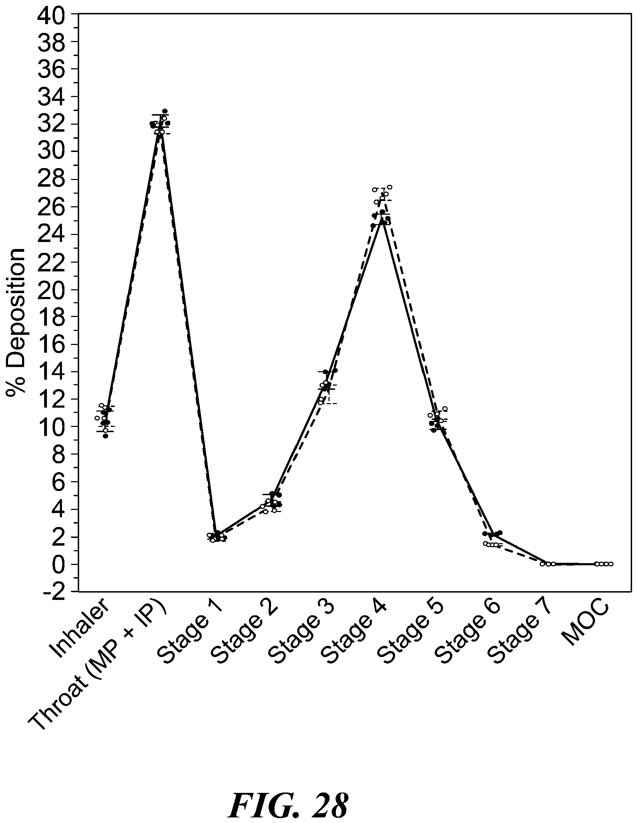

Metered dose inhalers containing a dual co-suspension composition comprising glycopyrrolate and formoterol active agent particles were prepared, with each type of active agent particle being provided as a micronized, crystalline API material. The active agent particles were suspended in HFO-1234ze(E) propellant, either with or without phospholipid particles. As shown in **FIG. 28****,** the two types of active agent particles and the suspending particles showed a uniform deposition distribution in the formulation containing phospholipid particles.

### Example 7

Metered dose inhalers containing a triple co-suspension composition comprising glycopyrrolate, budesonide, and formoterol active agent particles were prepared, with each type of active agent particle being provided as a micronized, crystalline API material. The active agent particles were suspended in either HFA-134a or HFO-1234ze(E) propellant, and formulated either with or without phospholipid particles.

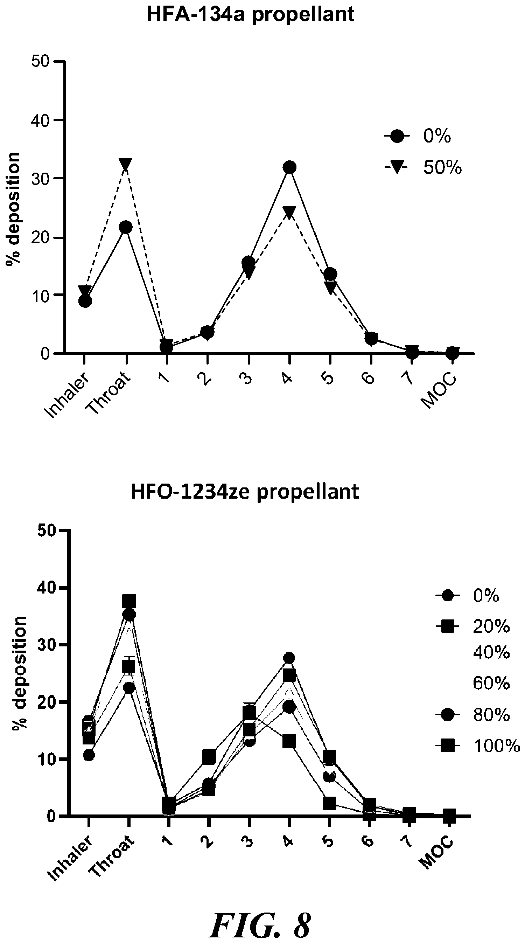

The deposition distribution of the formoterol active agent particles was tested at several different environmental humidity (RH) levels, ranging from 0% to 100%. The formoterol active agent particles in the formulations without phospholipid particles showed increased throat and stage 3 deposition in HFO-1234ze(E) as compared to HFA-134a. This difference was not observed, however, in the formulations that included phospholipid particles, which showed similar formoterol deposition distributions in HFO-1234ze(E) (**FIG. 8**, bottom panel) and in HFA-134a (**FIG. 8**, top panel).

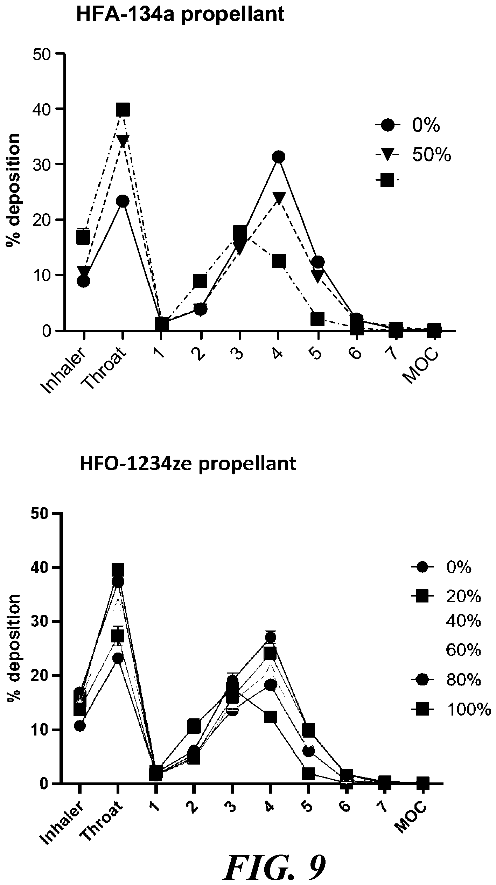

The deposition distribution of the budesonide active agent particles was tested at several different environmental humidity (RH) levels, ranging from 0% to 100%. The budesonide deposition distribution in the HFA-134a formulations without phospholipid particles is more sensitive to RH levels than in the HFO-1234ze(E) formulations without phospholipid particles. In the presence of phospholipid particles, both the HFA-134a and HFO-1234ze(E) formulations are more sensitive to RH as compared to the formulations without phospholipid particles, and both formulations showed similar changes to deposition distribution based on RH levels (**FIG. 9**).

The deposition distribution of the glycopyrrolate active agent particles was tested at several different environmental humidity (RH) levels, ranging from 0% to 100%. In the presence of phospholipid particles, both the HFA-134a and HFO-1234ze(E) formulations are more sensitive to RH as compared to the formulations without phospholipid particles, and both formulations showed similar changes to deposition distribution based on RH levels.

### Example 8

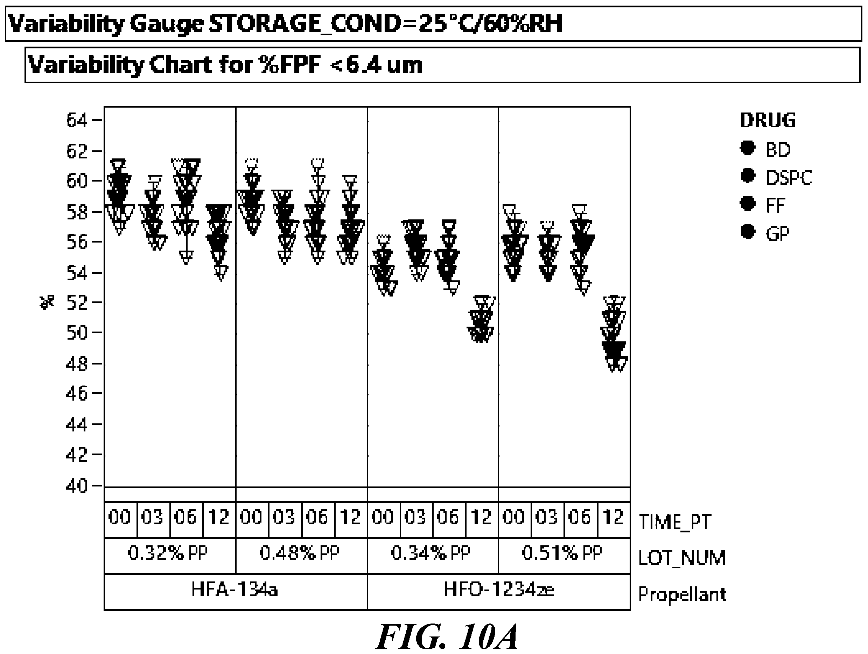

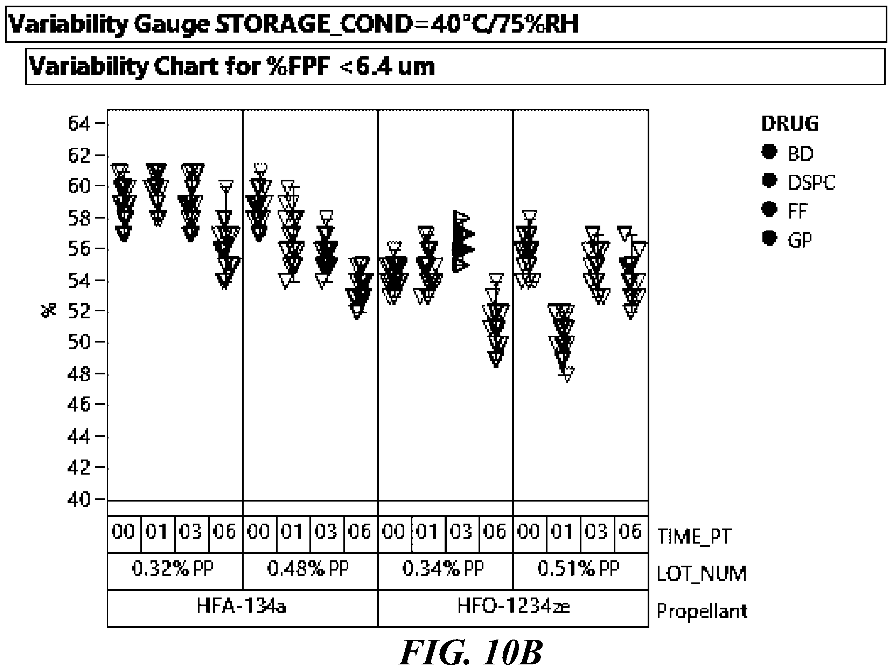

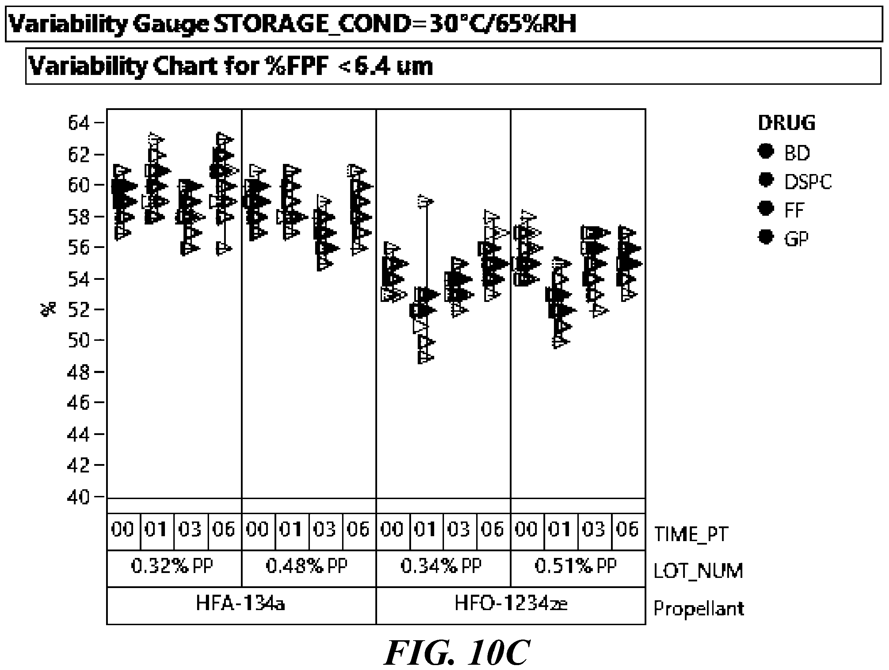

The fine particle fraction (FPF) present in the delivered dose upon actuation of an MDI containing budesonide, formoterol, or glycopyrrolate active agent particles and phospholipid particles was measured following storage of the MDI under various temperature and relative humidity conditions for varied periods of time. (**FIGs 10A****,** **10B****,** **10C****)**

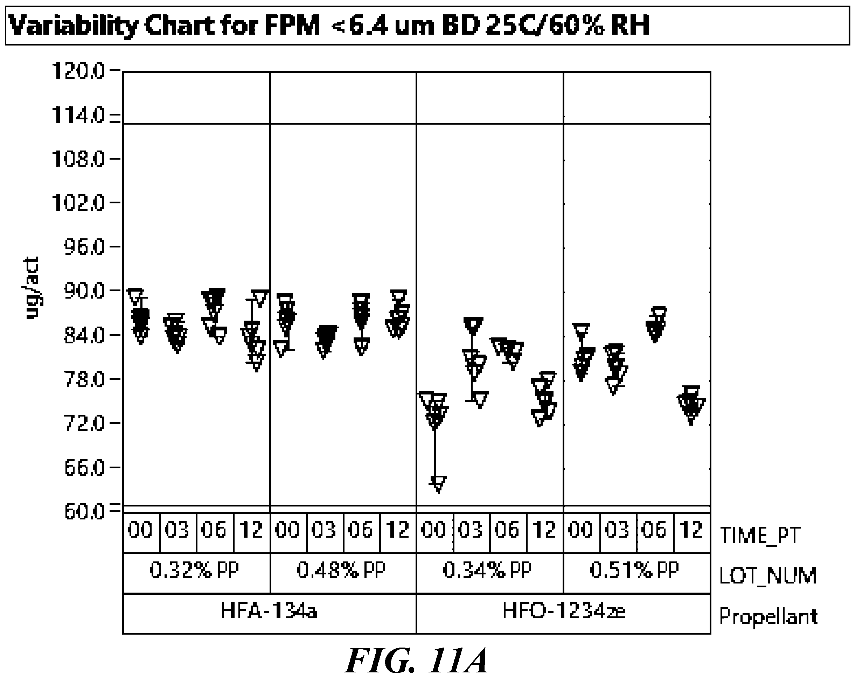

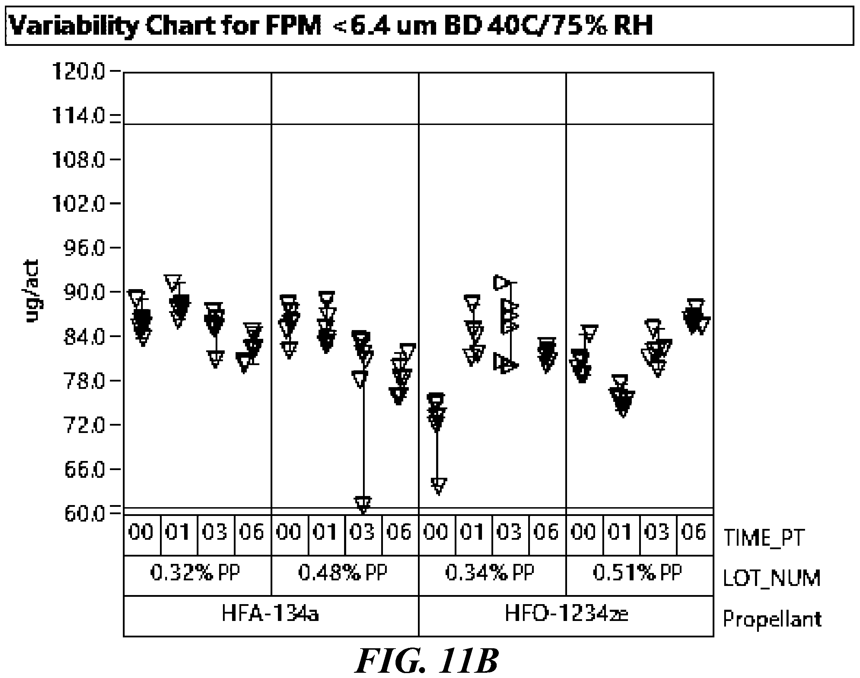

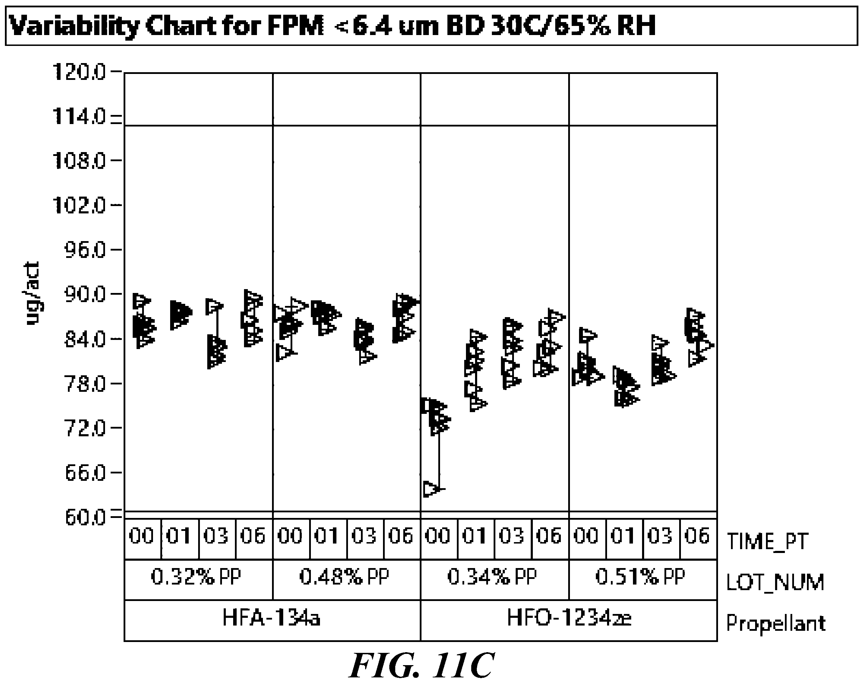

The fine particle mass (FPM) present in the delivered dose upon actuation of an MDI containing budesonide and phospholipid particles was measured following storage of the MDI under various temperature and relative humidity conditions for varied periods of time. (**FIGs 11A****,** **11B****,** **11C**)

### Example 9

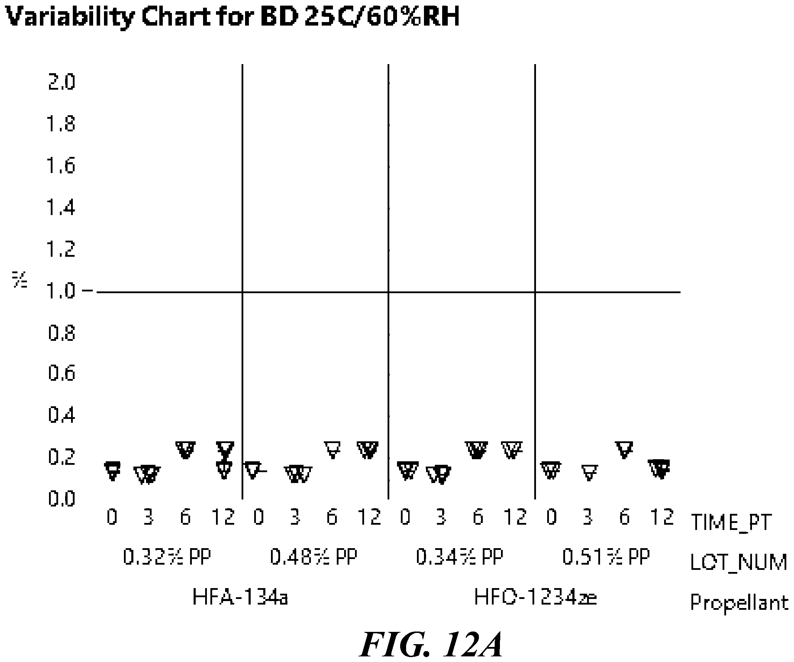

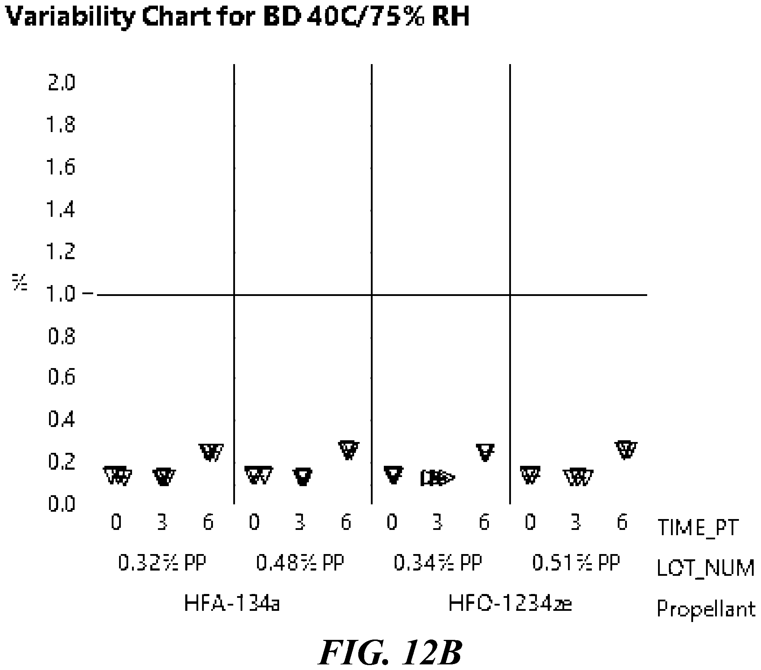

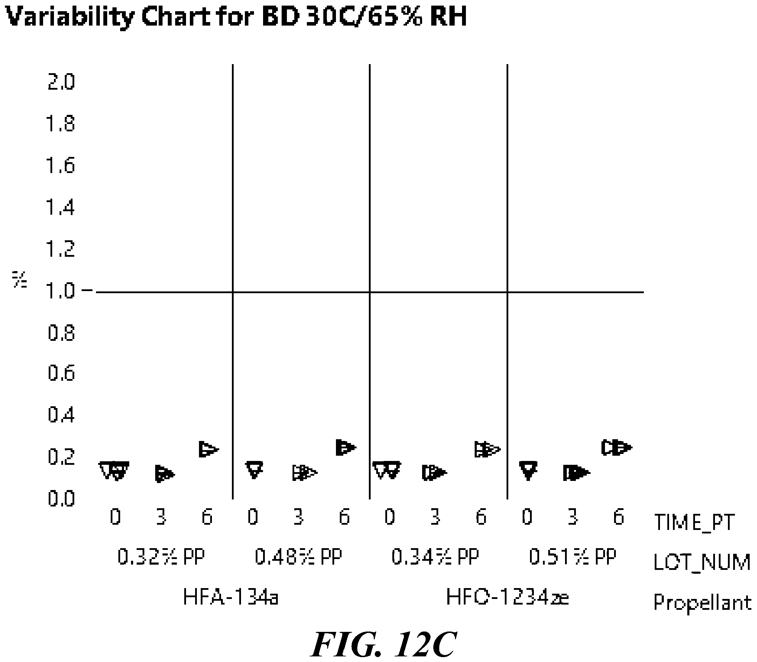

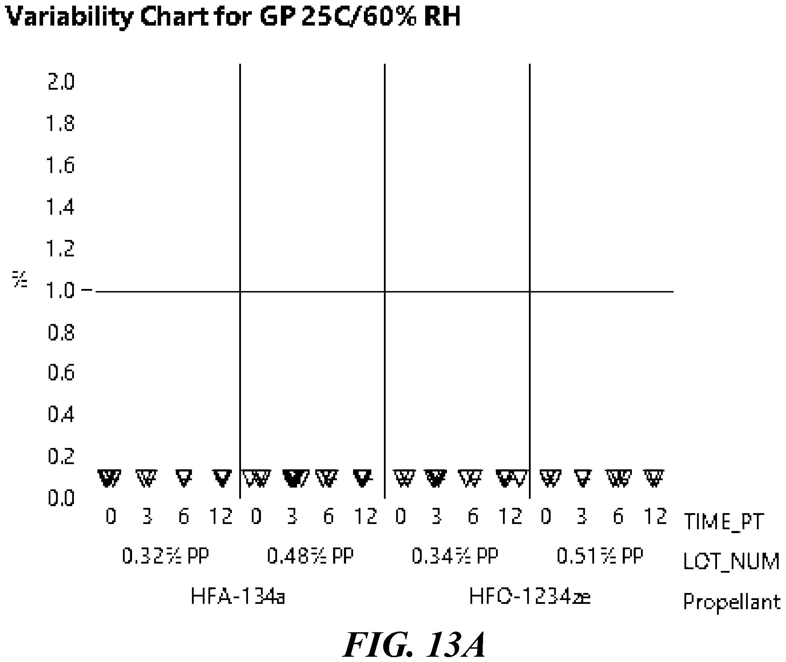

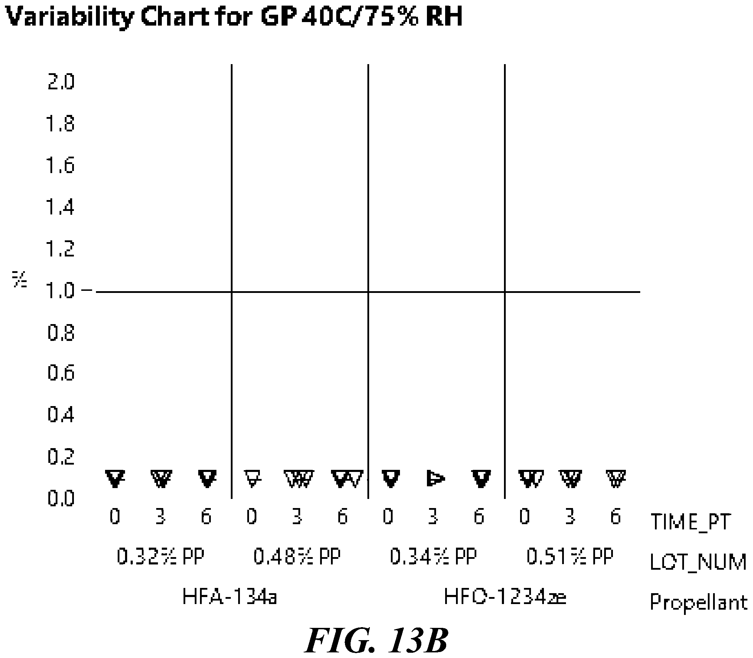

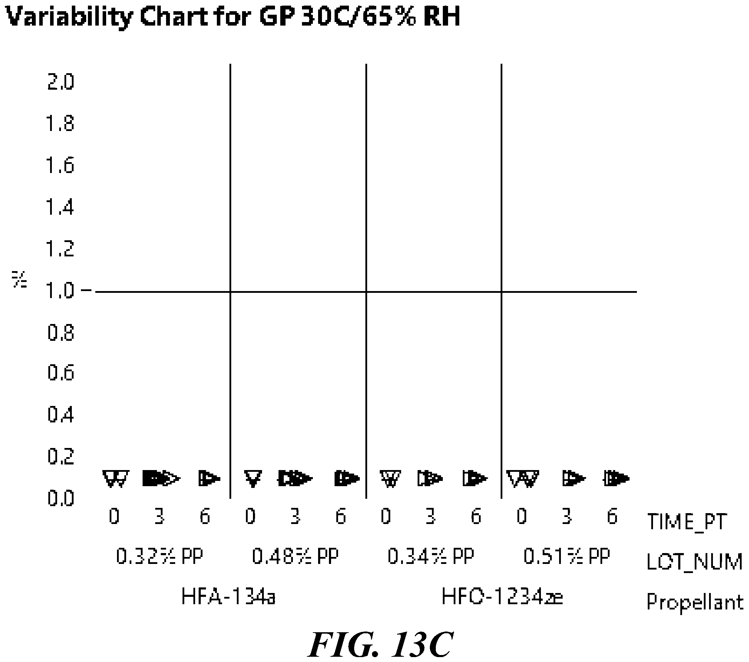

Degradation of budesonide (**FIGs 12A****,** **12B****,** **12C**) and glycopyrrolate (**FIGs 13A****,** **13B****,** **13C**) active agent particles in an MDI canister containing active agent particles and phospholipid particles was measured following storage of the MDI under various temperature and relative humidity conditions for varied periods of time.

### Example 10

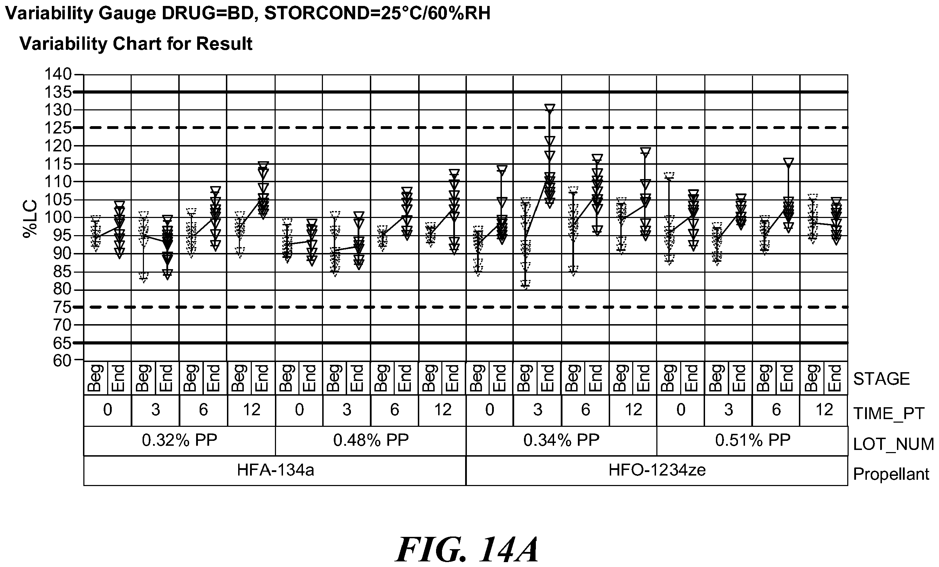

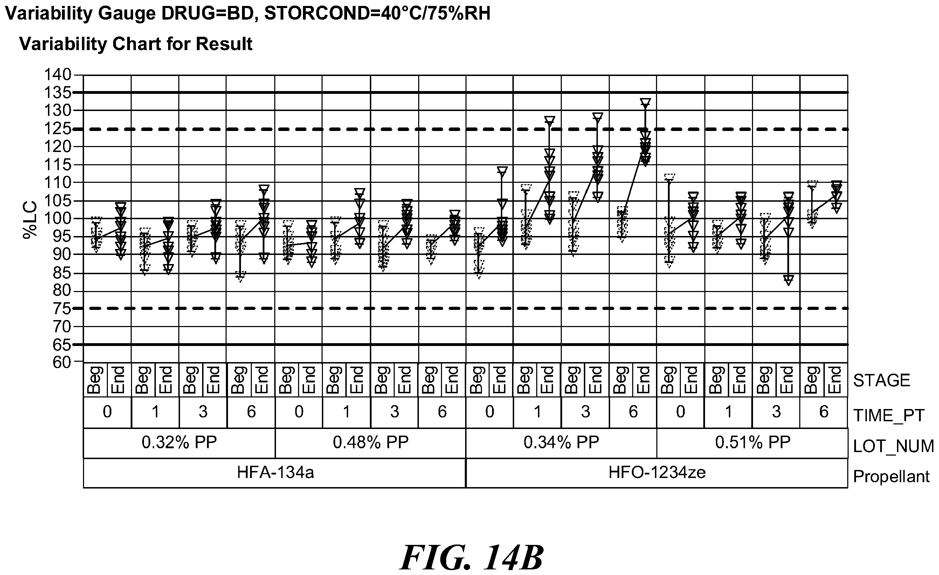

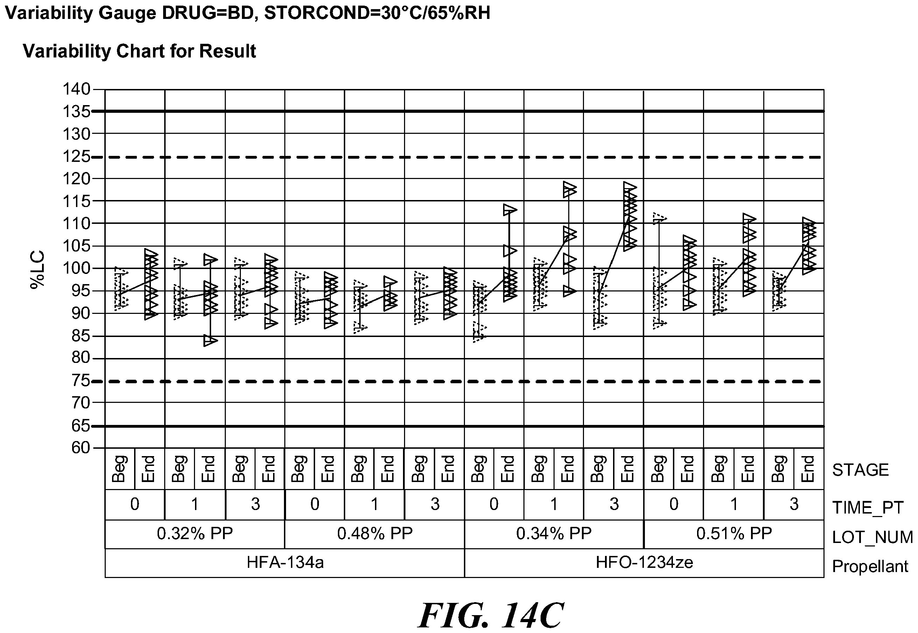

Delivered dose uniformity upon actuation of an MDI containing budesonide active agent particles and phospholipid particles was measured following storage of the MDI under various temperature and relative humidity conditions for varied periods of time. (**FIGs 14A****,** **14B****,** **14C**)

### Example 11

Metered dose inhalers containing a quadruple co-suspension composition comprising glycopyrrolate, budesonide, formoterol, and roflumilast active agent particles were prepared, with each type of active agent particle being provided as a micronized, crystalline API material. The active agent particles were suspended in HFO-1234ze(E) propellant, and formulated with phospholipid particles. The deposition distribution of each type of active agent particles was tested for a freshly prepared MDI, after three months of storage at 25°C and 75% relative humidity, and after three months of storage at 40°C and 75% relative humidity. The quadruple formulation demonstrates consistent aerosol distribution for each of the four types of active agent particles, and this distribution is consistent after three months of storage at the tested temperatures and relative humidity levels.

### Example 12

A randomized, single blind, 3-period, 3-treatment, single-dose, crossover study was conducted to assess the relative bioavailability of BGF MDI HFO-1234ze(E) and BGF MDI HFC-152a compared with BGF MDI HFA-134a in healthy subjects.

The investigational medical products include (1) Test Product of budesonide/glycopyrronium/formoterol (BGF) metered dose inhaler (MDI) formulated with HFO-1234ze(E) propellant and (2) Reference Product of budesonide/glycopyrronium/formoterol (BGF) metered dose inhaler (MDI) formulated with HFA-134a propellant. The indication studied is chronic obstructive pulmonary disease (COPD) and the development phase is Phase 1.

### STUDY OBJECTIVES:

### Primary objective:

To evaluate the relative bioavailabilities between the test formulations and the reference formulation for fixed dose combinations (FDCs) of budesonide, glycopyrronium, and formoterol when administered as budesonide, glycopyrronium, and formoterol (BGF) metered dose inhaler (MDI) with 3 different propellants.

### Secondary objectives:

To determine the pharmacokinetic (PK) parameters of BGF when administered as 3 different propellant formulations. To assess the safety and tolerability of a combination of BGF when administered as single doses in 3 different propellant formulations in healthy subjects.

### STUDY DESIGN:

This study was a randomized, single blind, 3-period, 3-treatment, single-dose, single-center, crossover study. The study included the assessment of PK properties of BGF MDI formulated with 3 different propellants: hydrofluoroolefin (HFO-1234ze(E)) - Treatment A (test), hydrofluorocarbon (HFC-152a) - Treatment B (test), and hydrofluoroalkane (HFA-134a) - Treatment C (reference).

The study comprised of:
- Screening period: up to 28 days prior to first dosing.
- Three treatment periods of maximum 3 days each: subjects were resident from the morning of the day before the first dosing with BGF MDI (Day -1) in Treatment Period 1, throughout all treatment and washout periods up to discharge on Day 2 of Treatment Period 3.
- Follow-up: within 3 to 7 days after the last administration of BGF MDI. There was a washout period of 3 to 7 days between each dose. Each subject received 3 single-dose treatments of BGF MDI (1 dose HFO-1234ze(E) [Treatment A]; 1 dose HFC-152a [Treatment B] and 1 dose HFA-134a [Treatment C]), following an overnight fast of at least 8 hours.

### MAIN INCLUSION CRITERIA:

Healthy, non-smoking male subjects aged 18 to 60 years with suitable veins for cannulation or repeated venepuncture. Subjects had to have a body mass index (BMI) between 18 and 30 kg/m2, inclusive and weigh at least 50 kg and no more than 100 kg, inclusive. Subjects had to have a forced expiratory volume in one second (FEV1) ≥ 80% of the predicted value regarding age, height, and ethnicity at the screening visit.

### INVESTIGATIONAL MEDICINAL PRODUCTS:

Treatment A (test): BGF MDI HFO-1234ze(E) with strength/concentrations of 160/7.2/4.8 µg per actuation.
Treatment B (test): BGF MDI HFC-152a with strength/concentrations of 160/7.2/4.8 µg per actuation.
Treatment C (reference): BGF MDI HFA-134a with strength/concentrations of 160/7.2/4.8 µg per actuation.

### DURATION OF STUDY:

Each subject was to be involved in the study for up to 53 days.

### TREATMENT COMPLIANCE:

Dosing took place at the Parexel Early Phase Clinical Unit in Los Angeles. The administration of all investigational medicinal products (IMPs) was recorded in Parexel's electronic source data capturing and information management system (CLINBASE^{™}). Compliance was assured by direct supervision and witnessing of IMP administration.

### CRITERIA FOR EVALUATION:

### Pharmacokinetic Parameters:

- Primary PK parameters: Cmax, AUCinf, and AUClast for test and reference treatment.
- Secondary PK parameters: tmax, t½λz, MRT, λz, CLIP, Vz/F, TRCmax, TRAUCinf, and TRAUClast.

### Safety Variables:

- Adverse events (AEs)/Serious adverse events (SAEs).
- Vital signs (systolic and diastolic blood pressure, pulse rate, body temperature, oxygen saturation, and respiratory rate).
- Twelve-lead safety and digital electrocardiograms (ECGs) as well as cardiac telemetry
- Physical examination.
- Laboratory assessments (hematology, clinical chemistry and urinalysis)
- Spirometry.
- Taste assessment.

### STATISTICAL METHODS:

### Determination of Sample Size:

This was a pilot PK study to determine the relative bioavailabilities between 2 test formulations of BGF MDI compared with the conventional formulation. Therefore, no sample size calculation was performed.

It was expected that 48 healthy subjects (number of subjects were increased from 24 to 48 as per protocol amendment 2 to account for replacement subjects due to a dosing deviation involving the first 23 subjects) were to be randomized to a 6 sequence Williams design for 3 periods and 3 treatments: ABC, BCA, CAB, ACB, BAC and CBA, in order to ensure at least 20 evaluable subjects at the end of the last treatment period.

Subjects were considered evaluable if they had an evaluable PK profile, i.e., (1) received active treatment, (2) did not significantly violate protocol inclusion or exclusion criteria, or deviate significantly from the protocol, and (3) did not have unavailable or incomplete data which may have influenced the PK analysis, Presentation and Analysis of Pharmacokinetic Data:
All PK concentrations, parameter summaries and statistical analyses were presented for the PK Analysis Set, unless otherwise specified. The PK concentration and parameter listings were presented for the Safety Analysis Set and included all reportable individual PK results. Individual PK concentration and parameter data for any subjects not included in the PK Analysis Set or excluded from the descriptive summary tables, figures and/or inferential statistical analyses were included in the listings and flagged with an appropriate footnote.

The test treatments, Treatments A and B (BGF MDI HFO and BGF MDI HFC, respectively), were separately compared to the reference treatment, Treatment C (BGF MDI HF A), for each analyte. The statistical analyses were performed using a linear mixed effects analysis of variance model, using the natural logarithm of Cmax, AUCinf, and AUClast as the response variables, with sequence, and period, treatment as fixed effects and subject nested within sequence as random effect. Transformed back from the logarithmic scale, geometric means together with the intra-subject coefficient of variation confidence intervals (CIs) (2-sided 95%) for Cmax, AUCinf, and AUClast were estimated and presented. In addition, ratios of geometric means together with CIs (2-sided 90%) were estimated and presented.

Additionally, the median difference in untransformed tmax between the test treatments and the reference treatment for each analyte and the corresponding 90% CIs for the median differences, for each analyte were calculated using the non parametric Hodges Lehmann method.

### Presentation and Analysis of Safety and Eligibility Data:

Safety data (scheduled and unscheduled) were presented in the data listings. Continuous variables were summarized using descriptive statistics (n, mean, standard deviation [SD], minimum, median, maximum) by treatment. Categorical variables were summarized in frequency tables (frequency and proportion) by treatment, if applicable. The analysis of the safety variables was based on the Safety Analysis Set.

Adverse events were summarized by Preferred Term (PT) and System Organ Class (SOC) using Medical Dictionary for Regulatory Activities (MedDRA) vocabulary. Furthermore, listings of SAEs and AEs that led to withdrawal were made and the number of subjects who had any AEs, SAEs, AEs that led to withdrawal, and AEs with severe intensity were summarized. Adverse events that occurred before dosing were reported separately.

Tabulations and listings of data for vital signs, clinical laboratory tests, digital ECGs, and 12-lead safety ECGs (listings only), telemetry (listings only), and spirometry were presented. Results from the taste assessment were presented separately in listings only. Any new or aggravated clinically relevant abnormal medical physical examination finding compared to the baseline assessment was reported as an AE. Data were summarized for the observed values at each scheduled assessment, together with the corresponding changes from the baseline when baseline was defined. Clinical laboratory data were reported in the units provided by the clinical laboratory for the Safety Review Committee (SRC) meeting, and in Système International (SI) units in the Clinical Study Report (CSR).

Out of range values for safety laboratory assessments were flagged in individual listings as well as summarized descriptively using agreed standard reference ranges and/or extended reference ranges (e.g., AstraZeneca, program, or laboratory ranges).

### PROTOCOL DEVIATIONS:

In total, important protocol deviations were reported for 26 (55.3%) subjects during the study:
- For Treatment A (HFO propellant): 23 (48.9%) subjects were reported with other important protocol deviations (subject did not self dose with the inhaler as outlined in the protocol. Nurse administered the dose).
- For Treatment B (HFC propellant): 23 (48.9%) subjects were reported with other important protocol deviations (subject did not self dose with the inhaler as outlined in the protocol. Nurse administered the dose) and 2 (4.3%) subjects did not receive the full dose expected due to issues during inhalation.
- For Treatment C (HFA propellant): 23 (48.9%) subjects were reported with other important protocol deviations (subject did not self dose with the inhaler as outlined in the protocol. Nurse administered the dose) and 1 (2.1%) subject did not receive the full dose expected due to issues during inhalation.

The number of subjects were increased from 24 to 48 as per protocol amendment 2 to account for replacement subjects due to a dosing deviation involving the first 23 subjects.

There were 23 subjects excluded from the PK Analysis Set due to protocol deviations reported. No important protocol deviations related to COVID-19 were reported during the study.

### PHARMACOKINETIC RESULTS:

- Systemic exposure to budesonide from BGF MDI HFO was comparable to BGF MDI HFA, with GMRs and 90% CIs of 111.7% (91.01%, 137.1%), 104.7% (91.95%, 119.2%) and 107.2% (94.53%, 121.9%) for Cmax, AUCinf and AUClast, respectively
- Systemic exposure to glycopyrronium from BGF MDI HFO was comparable to BGF MDI HFA, with GMRs and 90% CIs of 108.3% (85.50%, 137.3%) and 106.1% (86.18%, 130.6%) for Cmax and AUClast, respectively.
- Systemic exposure to formoterol from BGF MDI HFO was comparable to BGF MDI HFA, with GMRs and 90% CIs of 109.1% (97.02%, 122.7%), 96.00% (70.33%, 131.0%) and 98.13% (86.44%, 111.4%) for Cmax, AUCinf and AUClast, respectively.

### SAFETY RESULTS:

- There were no deaths, SAEs, or AEs that led to the discontinuation of the IMP reported during this study.
- No new safety signals were observed, no clinically relevant trends were observed for vital signs, physical examination, laboratory results, spirometry and taste assessment, and no abnormal clinically significant 12-lead safety and digital ECG, as well as cardiac telemetry findings were reported.
- The combination of budesonide, glycopyrronium, and formoterol when administered as single doses in 3 different propellant formulations demonstrated an acceptable safety profile and was well tolerated in the studied population.

In view of this clinical study, systemic exposure to budesonide, glycopyrronium, and formoterol was similar for BGF MDI HFO-1234ze(E) compared with the reference product, BGF MDI HFA-134a. There was no indication of meaningful differences between the products in this taste assessment. The combination of budesonide, glycopyrronium, and formoterol when administered as single doses in HFO-1234ze(E) and HFA-134a formulations demonstrated an acceptable safety profile and was well tolerated in the studied population.

### Example 13

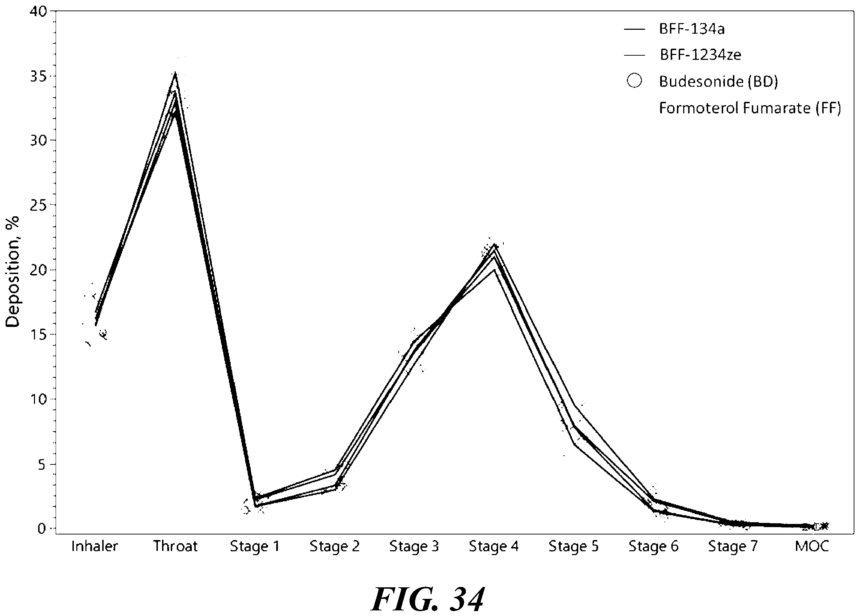

**FIG. 34** depicts the aerosol particle size distribution (aPSD) measured by a Next Generation Impactor (NGI), expressed as a percent of the total recovered mass, for budesonide (BD) and formoterol fumarate (FF) active agent particles actuated from an MDI containing a dual fixed dose combination co-suspension of budesonide and formoterol fumarate active agent particles suspended in HFA-134a (formulation referred to as BFF-134a) or HFO-1234ze(E) (formulation referred to as BFF-1234ze) propellant with phospholipid suspending particles. The profiles demonstrate a similar aerosol distribution between HFA-134a and HFO-1234ze(E) for both active agent particles.

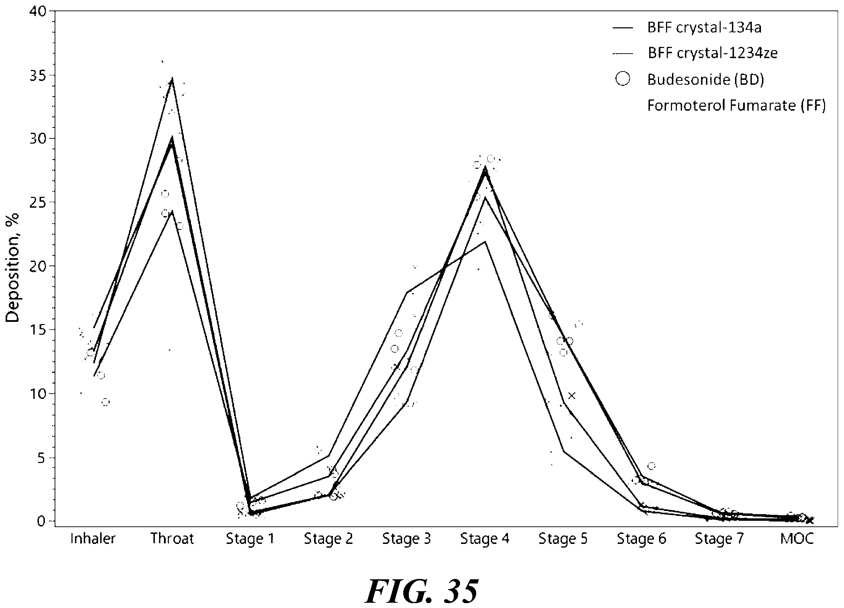

**FIG. 35** depicts the aPSD measured by NGI, expressed as a percent of the total recovered mass, for budesonide (BD) and formoterol fumarate (FF) active agent particles actuated from an MDI containing a triple fixed dose combination co-suspension of budesonide and formoterol fumarate active agent particles suspended in HFA-134a (formulation referred to as BFF crystal-134a) or HFO-1234ze(E) (formulation referred to as BFF crystal-1234ze) propellant without phospholipid suspending particles. The profiles demonstrate unique aerosol distributions between HFA-134a and HFO-1234ze(E) for both active agent particles.

Table 12 provides a summary of the fine particle fraction, <6.4 µm (FPF), fine particle dose, <6.4 µm (FPD), mass median aerodynamic diameter (MMAD), and throat deposition of budesonide and formoterol fumarate calculated from the NGI datasets of BFF-134a, BFF-1234ze, BFF crystal-134a, and BFF crystal-1234ze.

**Table 12. BD and FF Fine Particle Fraction (FPF), Fine Particle Dose (FPD), Mass Median Aerodynamic Diameter (MMAD), and Throat Deposition of BFF-134a, BFF-1234ze, BFF crystal-134a, and BFF crystal-1234ze**

| **Formulation** | **Active** | **FPF, <6.4 µm (%)** | **FPD, <6.4 µm (µg/act)** | **MMAD (µm)** | **Throat Deposition (%)** |
|---|---|---|---|---|---|
| BFF-134a | BD | 54 | 83.3 | 3.45 | 33.6 |
| | FF | 56 | 2.7 | 3.24 | 32.1 |
| BFF-1234ze | BD | 50 | 82.6 | 3.68 | 35.2 |
| | FF | 53 | 2.5 | 3.47 | 32.9 |
| BFF crystal-134a | BD | 67 | 163.4 | 3.01 | 24.3 |
| | FF | 53 | 3.8 | 3.85 | 34.7 |
| BFF crystal-1234ze | BD | 62 | 93.1 | 2.80 | 29.6 |
| | FF | 58 | 2.6 | 3.18 | 30.1 |

### Example 14

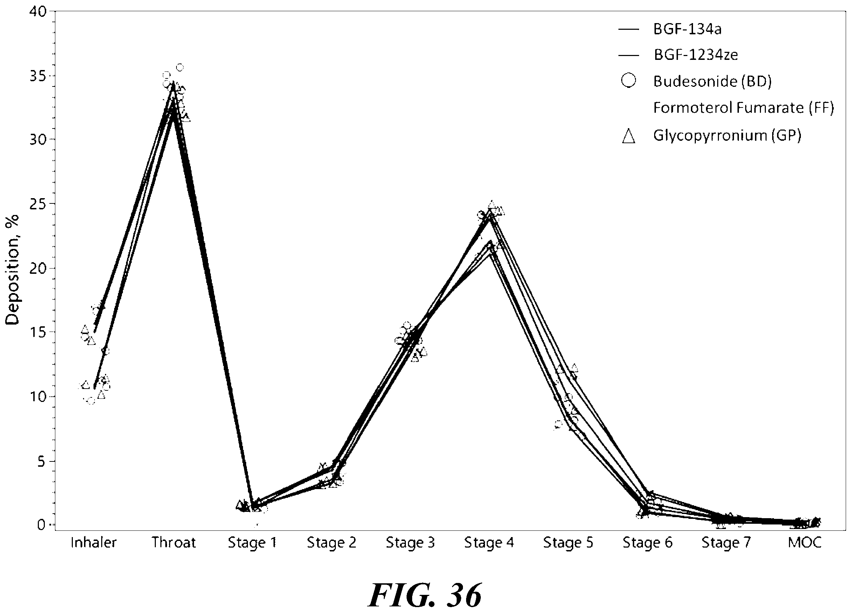

**FIG. 36** depicts the aerosol particle size distribution (aPSD) measured by a Next Generation Impactor (NGI), expressed as a percent of the total recovered mass, for budesonide (BD), glycopyrronium (GP), and formoterol fumarate (FF) active agent particles actuated from an MDI containing a triple fixed dose combination co-suspension of budesonide, glycopyrronium, and formoterol fumarate active agent particles suspended in HFA-134a (formulation referred to as BGF-134a) or HFO-1234ze(E) (formulation referred to as BGF-1234ze) propellant with phospholipid suspending particles. The profiles demonstrate a similar aerosol distribution between HFA-134a and HFO-1234ze(E) for all active agent particles.

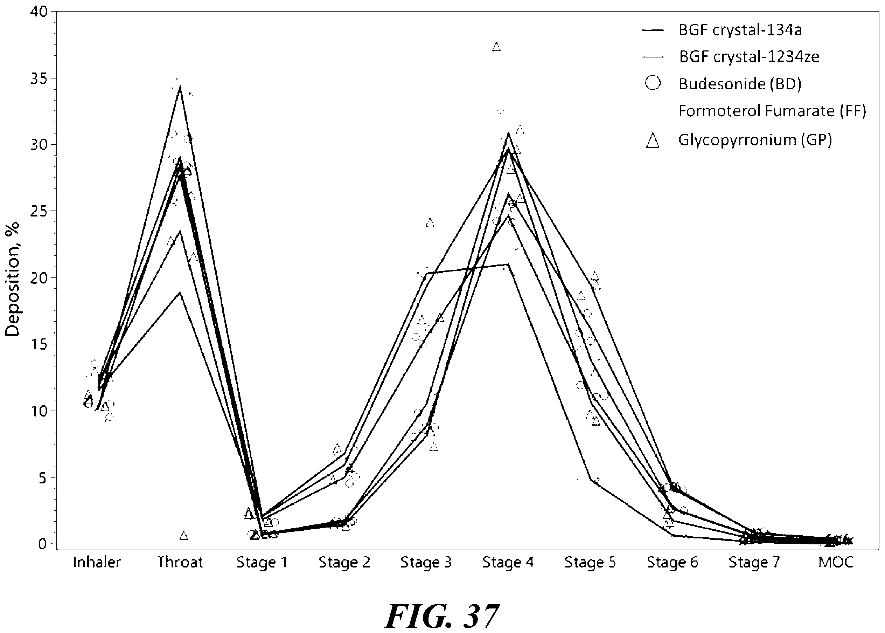

**FIG. 37** depicts the aPSD measured by NGI, expressed as a percent of the total recovered mass, for budesonide (BD), glycopyrronium (GP), and formoterol fumarate (FF) active agent particles actuated from an MDI containing a triple fixed dose combination co-suspension of budesonide, glycopyrronium, and formoterol fumarate active agent particles suspended in HFA-134a (formulation referred to as BGF crystal-134a) or HFO-1234ze(E) (formulation referred to as BGF crystal-1234ze) propellant without phospholipid suspending particles. The profiles demonstrate unique aerosol distributions between HFA-134a and HFO-1234ze(E) for all active agent particles.

Table 13 provides a summary of the fine particle fraction, <6.4 µm (FPF), fine particle dose, <6.4 µm (FPD), mass median aerodynamic diameter (MMAD), and throat deposition of budesonide, glycopyrronium, and formoterol fumarate calculated from the NGI datasets of BGF-134a, BGF-1234ze, BGF crystal-134a, and BGF crystal-1234ze.

**Table 13. BD, GP, and FF fine particle fraction, <6.4 µm (FPF), fine particle dose, <6.4 µm (FPD), mass median aerodynamic diameter (MMAD), and throat deposition of BGF-134a, BGF-1234ze, BGF crystal-134a, and BGF crystal-1234ze**

| **Formulation** | **Active** | **FPF, <6.4 µm (%)** | **FPD, <6.4 µm (µg/act)** | **MMAD (µm)** | **Throat Deposition (%)** |
|---|---|---|---|---|---|
| BGF-134a | BD | 56 | 93.9 | 3.83 | 3.33 |
| | GP | 59 | 4.5 | 3.68 | 3.15 |
| | FF | 59 | 3.0 | 3.69 | 3.18 |
| BGF-1234ze | BD | 52 | 82.4 | 3.87 | 3.61 |
| | GP | 54 | 3.8 | 3.70 | 3.50 |
| | FF | 54 | 2.6 | 3.73 | 3.48 |
| BGF crystal-134a | BD | 64 | 107.2 | 3.32 | 2.69 |
| | GP | 71 | 5.2 | 3.50 | 2.61 |
| | FF | 66 | 3.3 | 4.10 | 2.90 |
| BGF crystal-1234ze | BD | 61 | 179.2 | 3.32 | 3.32 |
| | GP | 70 | 8.2 | 3.50 | 3.51 |
| | FF | 52 | 4.7 | 4.10 | 4.10 |

## Claims

1. A pharmaceutical composition deliverable from a metered dose inhaler, the pharmaceutical composition comprising:
a propellant of pharmaceutical grade (1E)-1,3,3,3-Tetrafluoro-1-propene (HFO-1234ze(E)) having a purity of at least 99.90%;
a plurality of one or more species of active agent particles; and
a plurality of phospholipid particles comprising perforated microstructures;
wherein the one or more active agents are selected from a long-acting muscarinic antagonist (LAMA), a long-acting β2-agonists (LABA), a short-acting beta-agonists (SABA), an inhaled corticosteroid (ICS), and a non-corticosteroid anti-inflammatory agent, wherein the pharmaceutical composition comprises:
(i) a plurality of budesonide particles, and
a plurality of albuterol particles;
(ii) a plurality of glycopyrrolate particles, and
a plurality of formoterol particles; or
(iii) a plurality of budesonide particles, and
a plurality of formoterol particles.

2. The pharmaceutical composition according to claim 1, wherein (i) the SABA is present at a concentration in the range of 0.04 mg/mL to 2.25 mg/mL, (ii) the LABA is present at a concentration in the range of 0.01 mg/mL to 1 mg/mL, (iii) the ICS is present at a concentration in the range of 0.1 mg/mL to 20 mg/mL and/or (iv) the LAMA is present at a concentration in the range of 0.04 mg/mL to 2.25 mg/mL.

3. The pharmaceutical composition according to claim 1 or 2, wherein the perforated microstructures comprise 1,2-Distearoyl-sn-glycero-3-phosphocholine (DSPC) and calcium chloride.

4. The pharmaceutical composition according to any one of claims 1 to 3, wherein the phospholipid particles are present at a concentration in the range of 0.1 mg/mL to 10 mg/mL.

5. The pharmaceutical composition according to any one of the preceding claims, wherein the albuterol particles are in the propellant at a concentration sufficient to provide a delivered dose of glycopyrrolate per actuation of the metered dose inhaler selected from between 5 µg and 50 µg per actuation, between 2 µg and 25 µg per actuation, and between 6 µg and 15 µg per actuation.

6. The pharmaceutical composition according to any one of the preceding claims, wherein the albuterol particles comprise albuterol sulfate, for example micronized and crystalline albuterol sulfate.

7. The pharmaceutical composition according to any one of the preceding claims, wherein the formoterol particles are included in the composition at a concentration sufficient to provide a delivered dose of formoterol selected from between 1 µg and 30 µg, between 0.5 µg and 10 µg, between 2 µg and 5 µg, between 3 µg and 0 µg, between 5 µg and 10 µg, and between 3 µg and 30 µg per actuation of the metered dose inhaler.

8. The pharmaceutical composition according to any one of the preceding claims, wherein the formoterol particles comprise formoterol fumarate, for example micronized and crystalline formoterol fumarate.

9. The pharmaceutical composition according to any one of the preceding claims, wherein the budesonide particles are included in the composition at a concentration sufficient to provide a delivered dose of budesonide selected from between 50 µg and 400 µg, between 20 µg and 600 µg, between 30 µg and 100 µg, between 50 µg and 200 µg, and between 150 µg and 350 µg per actuation of the metered dose inhaler.

10. The pharmaceutical composition according to any one of the preceding claims, wherein the budesonide particles comprise micronized budesonide.

11. The pharmaceutical composition according to any one of the preceding claims, wherein the phospholipid particles are included in the composition at a concentration sufficient to provide a delivered dose of the phospholipid particles selected from between 50 µg and 400 µg.

12. A metered dose inhaler comprising a canister with an outlet valve including an actuator for dispensing a metered amount of a pharmaceutical composition according to any one of claims 1 through 11, wherein the canister contains the pharmaceutical composition.

13. The metered dose inhaler according to claim 12, wherein the outlet valve comprises a neck gasket and at least one seat gasket; and the neck gasket or the at least one seat gasket is composed of bromobutyl material.

14. The metered dose inhaler according to claim 12 or 13, exhibiting less than 10%, 9%, 8%, 7%, 6%, or 5% reduced shot weight per actuation throughout emptying of the canister and/or exhibiting less than 1.0%, 0.5%, 0.4%, 0.3%, 0.2%, or 0.1% weight loss at 25°C/60% RH per year.

15. The metered dose inhaler according to any one of claims 12 to 14, which exhibits a delivered dose uniformity (DDU) for the pharmaceutical formulation selected from a DDU of ± 20%, or better, a DDU of ± 15%, or better, and a DDU of ± 10%, or better, throughout emptying of the canister.

16. A pharmaceutical composition according to any one of claims 1 to 11 for use in the treatment of a pulmonary disease or disorder.

17. The pharmaceutical composition for use according to claim 16, wherein the treatment comprises a method of treating a pulmonary disease or disorder in a patient, the method comprising administering the pharmaceutical composition to the patient by actuating a metered dose inhaler; wherein the metered dose inhaler contains the pharmaceutical composition.

18. The pharmaceutical composition for use according to claim 16 or 17, wherein the pulmonary disease or disorder is asthma or COPD.

19. The pharmaceutical composition for use according to any one of claims 16 to 18, wherein the metered dose inhaler is described according to any one of the claims 12 to 15.

## Patentansprüche

1. Pharmazeutische Zusammensetzung, zuführbar aus einem Inhalator mit abgemessener Dosis, wobei die pharmazeutische Zusammensetzung Folgendes umfasst:
ein Treibmittel von pharmazeutischer Qualität (1E)-1,3,3,3-Tetrafluor-1-propen (HFO-1234ze(E)) mit einer Reinheit von zumindest 99,90 %;
eine Vielzahl einer oder mehrerer Spezies von Wirkstoffpartikeln; und
eine Vielzahl von Phospholipidpartikeln, umfassend perforierte Mikrostrukturen;
wobei der eine oder die mehreren Wirkstoffe aus einem langwirkenden Musca-rinantagonisten (LAMA), langwirkenden β2-Agonisten (LABA), kurzwirkenden Beta-Agonisten (SABA), einem inhalierten Kortikosteroid (ICS) und einem Nicht-Kortikosteroid-Entzündungshemmer ausgewählt sind, wobei die pharmazeutische Zusammensetzung Folgendes umfasst:
(i) eine Vielzahl von Budesonidpartikeln und
eine Vielzahl von Albuterolpartikeln;
(ii) eine Vielzahl von Glykopyrrolatpartikeln und
eine Vielzahl von Formoterolpartikeln; oder
(iii) eine Vielzahl von Budesonidpartikeln und
eine Vielzahl von Formoterolpartikeln.

2. Pharmazeutische Zusammensetzung nach Anspruch 1, wobei (i) der SABA in einer Konzentration im Bereich von 0,04 mg/ml bis 2,25 mg/ml vorhanden ist, (ii) der LABA in einer Konzentration im Bereich von 0,01 mg/ml bis 1 mg/ml vorhanden ist, (iii) das ICS in einer Konzentration im Bereich von 0,1 mg/ml bis 20 mg/ml vorhanden ist und/oder (iv) der LAMA in einer Konzentration im Bereich von 0,04 mg/ml bis 2,25 mg/ml vorhanden ist.

3. Pharmazeutische Zusammensetzung nach Anspruch 1 oder 2, wobei die perforierten Mikrostrukturen 1,2-Distearoyl-sn-glycero-3-phosphocholin (DSPC) und Calciumchlorid umfassen.

4. Pharmazeutische Zusammensetzung nach einem der Ansprüche 1 bis 3, wobei die Phospholipidpartikel in einer Konzentration im Bereich von 0,1 mg/ml bis 10 mg/ml vorhanden sind.

5. Pharmazeutische Zusammensetzung nach einem der vorangegangenen Ansprüche, wobei die Albuterolpartikel in dem Treibmittel in einer Konzentration vorliegen, die ausreichend ist, um eine zugeführte Dosis von Glycopyrrolat pro Betätigung des Inhalators mit abgemessener Dosis bereitzustellen, die aus zwischen 5 µg und 50 µg pro Betätigung, zwischen 2 µg und 25 µg pro Betätigung und zwischen 6 µg und 15 µg pro Betätigung ausgewählt ist.

6. Pharmazeutische Zusammensetzung nach einem der vorangegangenen Ansprüche, wobei die Albuterolpartikel Albuterolsulfat, beispielsweise mikronisiertes und kristallines Albuterolsulfat, umfassen.

7. Pharmazeutische Zusammensetzung nach einem der vorangegangenen Ansprüche, wobei die Formoterolpartikel in einer Konzentration in der Zusammensetzung enthalten sind, die ausreichend ist, um eine zugeführte Dosis von Formoterol bereitzustellen, die aus zwischen 1 µg und 30 µg, zwischen 0,5 µg und 10 µg, zwischen 2 µg und 5 µg, zwischen 3 µg und 0 µg, zwischen 5 µg und 10 µg und zwischen 3 µg und 30 µg pro Betätigung des Inhalators mit abgemessener Dosis ausgewählt ist.

8. Pharmazeutische Zusammensetzung nach einem der vorangegangenen Ansprüche, wobei die Formoterolpartikel Formoterolfumarat, beispielsweise mikronisiertes und kristallines Formoterolfumarat, umfassen.

9. Pharmazeutische Zusammensetzung nach einem der vorangegangenen Ansprüche, wobei die Budesonidpartikel in einer Konzentration in der Zusammensetzung enthalten sind, die ausreichend ist, um eine zugeführte Dosis von Budesonid bereitzustellen, die aus zwischen 50 µg und 400 µg, zwischen 20 µg und 600 µg, zwischen 30 µg und 100 µg, zwischen 50 µg und 200 µg und zwischen 150 µg und 350 µg pro Betätigung des Inhalators mit abgemessener Dosis ausgewählt ist.

10. Pharmazeutische Zusammensetzung nach einem der vorangegangenen Ansprüche, wobei die Budesonidpartikel mikronisiertes Budesonid umfassen.

11. Pharmazeutische Zusammensetzung nach einem der vorangegangenen Ansprüche, wobei die Phospholipidpartikel in einer Konzentration in der Zusammensetzung enthalten sind, die ausreichend ist, um eine zugeführte Dosis der Phospholipidpartikel bereitzustellen, die aus zwischen 50 µg und 400 µg ausgewählt ist.

12. Inhalator mit abgemessener Dosis, umfassend eine Kartusche mit einem Auslassventil, einschließlich eines Betätigungselements zum Abgeben einer abgemessenen Menge einer pharmazeutischen Zusammensetzung nach einem der Ansprüche 1 bis 11, wobei die Kartusche die pharmazeutische Zusammensetzung enthält.

13. Inhalator mit abgemessener Dosis nach Anspruch 12, wobei das Auslassventil eine Halsdichtung und zumindest eine Sockeldichtung umfasst; und wobei die Halsdichtung oder die zumindest eine Sockeldichtung aus Brombutyl-Material besteht.

14. Inhalator mit abgemessener Dosis nach Anspruch 12 oder 13, der ein um weniger als 10 %, 9 %, 8 %, 7 %, 6 % oder 5 % reduziertes Stoßgewicht pro Betätigung während des Leerens der Kartusche aufweist und/oder weniger als 1,0 %, 0,5 %, 0,4 %, 0,3 %, 0,2 % oder 0,1 % Gewichtsverlust bei 25 °C/60 % rF pro Jahr aufweist.

15. Inhalator mit abgemessener Dosis nach einem der Ansprüche 12 bis 14, der eine Einheitlichkeit der zugeführten Dosis (DDU) für die pharmazeutische Formulierung aufweist, die aus einer DDU von ± 20 % oder besser, einer DDU von ± 15 % oder besser und einer DDU von ± 10 % oder besser während des Leerens der Kartusche aufweist.

16. Pharmazeutische Zusammensetzung nach einem der Ansprüche 1 bis 11 zur Verwendung bei der Behandlung einer pulmonalen Erkrankung oder Störung.

17. Pharmazeutische Zusammensetzung zur Verwendung nach Anspruch 16, wobei die Behandlung ein Verfahren zum Behandeln einer pulmonalen Erkrankung oder Störung in einem Patienten umfasst, wobei das Verfahren das Verabreichen der pharmazeutischen Zusammensetzung an den Patienten durch Betätigen eines Inhalators mit abgemessener Dosis umfasst; wobei der Inhalator mit abgemessener Dosis die pharmazeutische Zusammensetzung enthält.

18. Pharmazeutische Zusammensetzung zur Verwendung nach Anspruch 16 oder 17, wobei die pulmonale Erkrankung oder Störung Asthma oder COPD ist.

19. Pharmazeutische Zusammensetzung zur Verwendung nach einem der Ansprüche 16 bis 18, wobei der Inhalator mit abgemessener Dosis gemäß einem der Ansprüche 12 bis 15 beschrieben ist.

## Revendications

1. Composition pharmaceutique pouvant être administrée à partir d'un inhalateur à dose mesurée, la composition pharmaceutique comprenant :
un propulseur de (1E)-1,3,3,3-Tétrafluoro-1-propène (HFO-1234ze(E)) de qualité pharmaceutique présentant une pureté d'au moins 99,90 % ;
une pluralité d'une ou plusieurs espèces de particules d'agent actif ; et
une pluralité de particules phospholipidiques comprenant des microstructures perforées ;
dans lequel les un ou plusieurs agents actifs sont choisis parmi un antagoniste muscarinique à action prolongée (LAMA), un β2-agoniste à action prolongée (LABA), un bêta-agoniste à action courte (SABA), un corticostéroïde inhalé (ICS) et un agent anti-inflammatoire non corticostéroïde, dans laquelle la composition pharmaceutique comprend :
(i) une pluralité de particules de budésonide, et
une pluralité de particules d'albutérol ;
(ii) une pluralité de particules de glycopyrrolate, et une pluralité de particules de formotérol ; ou
(iii) une pluralité de particules de budésonide, et une pluralité de particules de formotérol.

2. Composition pharmaceutique selon la revendication 1, dans laquelle (i) le SABA est présent à une concentration dans la plage de 0,04 mg/mL à 2,25 mg/mL, (ii) le LABA est présent à une concentration dans la plage de 0,01 mg/mL à 1 mg/mL, (iii) le ICS est présent à une concentration dans la plage de 0,1 mg/mL à 20 mg/mL et/ou (iv) le LAMA est présent à une concentration dans la plage de 0,04 mg/mL à 2,25 mg/mL.

3. Composition pharmaceutique selon la revendication 1 ou 2, dans laquelle les microstructures perforées comprennent de la 1,2-Distéaroyl-sn-glycéro-3-phosphocholine (DSPC) et du chlorure de calcium.

4. Composition pharmaceutique selon l'une quelconque des revendications 1 à 3, dans laquelle les particules de phospholipides sont présentes à une concentration comprise entre 0,1 mg/mL et 10 mg/mL.

5. Composition pharmaceutique selon l'une quelconque des revendications précédentes, dans laquelle les particules d'albutérol sont dans le propulseur à une concentration suffisante pour fournir une dose administrée de glycopyrrolate par actionnement de l'inhalateur à dose mesurée choisie entre 5 µg et 50 µg par actionnement, entre 2 µg et 25 µg par actionnement, et entre 6 µg et 15 µg par actionnement.

6. Composition pharmaceutique selon l'une quelconque des revendications précédentes, dans laquelle les particules d'albutérol comprennent du sulfate d'albutérol, par exemple du sulfate d'albutérol micronisé et cristallin.

7. Composition pharmaceutique selon l'une quelconque des revendications précédentes, dans laquelle les particules de formotérol sont incluses dans la composition à une concentration suffisante pour fournir une dose administrée de formotérol choisie entre 1 µg et 30 µg, entre 0,5 µg et 10 µg, entre 2 µg et 5 µg, entre 3 µg et 0 µg , entre 5 µg et 10 µg, et entre 3 µg et 30 µg par actionnement de l'inhalateur à dose mesurée.

8. Composition pharmaceutique selon l'une quelconque des revendications précédentes, dans laquelle les particules de formotérol comprennent du fumarate de formotérol, par exemple du fumarate de formotérol micronisé et cristallin.

9. Composition pharmaceutique selon l'une quelconque des revendications précédentes, dans laquelle les particules de budésonide sont incluses dans la composition à une concentration suffisante pour fournir une dose administrée de budésonide choisie entre 50 µg et 400 µg, entre 20 µg et 600 µg , entre 30 µg et 100 µg, entre 50 µg et 200 µg , et entre 150 µg et 350 µg par actionnement de l'inhalateur à dose mesurée.

10. Composition pharmaceutique selon l'une quelconque des revendications précédentes, dans laquelle les particules de budésonide comprennent du budésonide micronisé.

11. Composition pharmaceutique selon l'une quelconque des revendications précédentes, dans laquelle les particules de phospholipide sont incluses dans la composition à une concentration suffisante pour fournir une dose administrée des particules de phospholipide choisie entre 50 µg et 400 µg.

12. Inhalateur à dose mesurée comprenant une cartouche avec une soupape de sortie comprenant un actionneur pour distribuer une quantité mesurée d'une composition pharmaceutique selon l'une quelconque des revendications 1 à 11, dans lequel la cartouche contient la composition pharmaceutique.

13. Inhalateur à dose mesurée selon la revendication 12, dans lequel la soupape de sortie comprend un joint de col et au moins un joint de siège ; et le joint de col ou le au moins un joint de siège est composé d'un matériau de bromobutyle.

14. Inhalateur à dose mesurée selon la revendication 12 ou 13, présentant un poids injectable réduit de 10 %, 9 %, 8 %, 7 %, 6 % ou 5 % par actionnement tout au long du vidage de la cartouche et/ou présentant moins de 1,0 %, 0,5 %, 0,4 %, 0,3 %, 0,2 % ou 0,1 % de perte de poids à 25°C/60 % HR par an.

15. Inhalateur à dose mesurée selon l'une quelconque des revendications 12 à 14, qui présente une uniformité de dose administrée (DDU) pour la formulation pharmaceutique choisie parmi une DDU de ± 20 %, ou mieux, une DDU de ± 15 %, ou mieux, et une DDU de ± 10 %, ou mieux, tout au long du vidage de la cartouche.

16. Composition pharmaceutique selon l'une quelconque des revendications 1 à 11 à utiliser dans le traitement d'une maladie ou d'un trouble pulmonaire.

17. Composition pharmaceutique à utiliser selon la revendication 16, dans laquelle le traitement comprend un procédé de traitement d'une maladie ou d'un trouble pulmonaire chez un patient, le procédé comprenant l'administration de la composition pharmaceutique au patient en actionnant un inhalateur à dose mesurée ; dans laquelle l'inhalateur à dose mesurée contient la composition pharmaceutique.

18. Composition pharmaceutique à utiliser selon la revendication 16 ou 17, dans laquelle la maladie ou le trouble pulmonaire est l'asthme ou la BPCO.

19. Composition pharmaceutique à utiliser selon l'une quelconque des revendications 16 à 18, dans laquelle l'inhalateur à dose mesurée est décrit selon l'une quelconque des revendications 12 à 15.
